# EUROPEAN PATENT APPLICATION

(11) **EP 4 729 520 A1**
(43) Date of publication of application: **22.04.2026**
(21) Application number: 24206945.8
(22) Date of filing: 16.10.2024
(51) Int. Cl.: C07D 487/14, C07D 487/20, C07D 519/00

(54) **CONFORMATIONALLY FIXED DIPROLINE MIMETICS AND THEIR USE**

(71) Applicant: Prosion GmbH, 50674 Köln (DE)
(72) Inventor: Albat, Dominik Ralph, 50676 Köln (DE); Chiha, Slim, 50674 Köln (DE); Strippel, Julian Philipp, 50939 Köln (DE); La Pietra, Luigi, 50676 Köln (DE); Müller, Matthias, 10559 Berlin (DE); Schmalz, Hans-Günther, 50321 Brühl (DE)
(74) Representative: Hertin und Partner Rechts- und Patentanwälte PartG mbB

(57) **Abstract**

The invention relates to chemical compounds useful as structural mimetics of peptides comprising proline-rich helix structures, preferably polyproline-type II (PPII) helix structures. The invention further relates to the medical use of the chemical compounds in the treatment of a medical condition associated with a modified intracellular signal transduction process mediated by a proline-rich helix structure and a protein having a binding domain binding to said proline-rich helix structure such as an infectious disease, immune disease, neurological disease, cardiometabolic disease and a cancerous disease. The invention further relates to pharmaceutical compositions comprising a compound of the present invention or a pharmaceutically acceptable salt thereof and a pharmaceutically acceptable carrier. The invention further relates to an in vitro method for modifying an intracellular signal transduction process mediated by a proline-rich helix structure, comprising contacting a compound of the invention with a biological cell.

## Description

### DESCRIPTION

The invention is in the field of biochemistry, chemical compounds and pharmaceutical uses thereof. The invention relates to chemical compounds useful as structural mimetics of peptides comprising proline-rich helix structures, preferably polyproline-type II (PPII) helix structures.

The invention further relates to chemical compounds useful as inhibitors of proline-rich motif mediated protein-protein interactions. The invention further relates to the medical use of the chemical compounds in the treatment of a medical condition associated with a modified intracellular signal transduction process mediated by a proline-rich helix structure, such as but not limited to cancer, infections, immune disorders, neurological disease and/or cardiometabolic disease.

The invention further relates to pharmaceutical compositions comprising a compound of the present invention or a pharmaceutically acceptable salt thereof and a pharmaceutically acceptable carrier.

The invention further relates to an in vitro method for modifying an intracellular signal transduction process mediated by a proline-rich helix structure, comprising contacting a compound of the invention with a biological cell.

### BACKGROUND OF THE INVENTION

Interactions between proteins play a central role in most physiological and biochemical processes in living organisms. Proteins not only perform biocatalytic tasks (for example as enzymes) but are also involved in various biological processes including physiological but also pathological processes of various medical conditions. Selective inhibition of the physical interaction between peptide ligands and the corresponding protein receptors can have a significant influence on various biological mechanisms in the cell. In light thereof there is a need to develop molecules that inhibit specific protein-protein interactions in order to achieve biological and therapeutic effects.

A current challenge in the development of new active pharmaceutical agents is to specifically target and modify protein-protein interactions by small molecule inhibitors (Ross et al., 2010). Thereby, a particular challenge is to develop molecules that bind with high affinity and selectivity to target proteins (or protein domains) that are otherwise difficult to address.

An example of such small molecule inhibitors addressing protein-protein interactions are structural mimetics of diproline units. Compared to other amino acids, proline has a special role due to its chemical structure. Among the essential (proteinogenic) amino acids, proline is unique in that it is the only natural amino acid that contains a secondary amino function, which is also incorporated into a ring (Morgan et al., 2013). Furthermore, proline is a lipophilic amino acid, and

proline-rich motifs are involved in many relevant protein-protein interactions that represent relevant but as yet pharmacologically unexplored drug targets (Kay et al., 2000). Structural biology studies have shown that the binding of proline-rich sequences to proteins that have domains specialised for their recognition requires the formation of defined secondary structures, in many cases a so-called polyproline type 2 helix (PPII helix) (Ball et al., 2005). By providing polyproline mimetics mimicking these secondary structures, some of these interactions can be selectively inhibited. One example are ligands for Ena/VASP EVH1 and other protein domains that are specialised in the recognition of proline-rich sequences in a PPII helix conformation (Opitz et al., 2015).

In the course of developing small molecule ligands for Ena/VASP EVH1 and other protein domains that are specialised in the recognition of proline-rich sequences (Kay et al., 2005), it was shown that by combining proline-derived dipeptide modules (so-called ProMs), peptideanalogous and conformationally pre-organised compounds can be produced that bind to the target protein with high affinity and selectivity (Opitz et al., 2015).

Optimisation of the drug structure of these small molecule ligands led to the identification of improved therapeutic agents that bind to Ena/VASP EVH1 with binding constants in the nanomolar concentration range and that impair the invasion and extravasation of breast cancer cells (Barone et al., 2020). Further suitable diproline mimetics and molecular building blocks for such mimetics, e.g., as ligands for Ena/VASP EVH1 are disclosed in WO 2008/040332, WO 2013/030111, WO2016/092069 and WO2019/162524. Reliable synthesis methods have been developed for these diproline mimentics and molecular building blocks (Dohmen et al., 2020; Albat et al., 2021; Albat et al, 2022; Albat et al. 2023).

Although the compounds disclosed in the prior art inhibit protein-protein interactions, e.g., proline-rich motif-based protein-protein interactions by selective binding to a receptor such as PPII helix-based protein-protein interactions, and are suitable as therapeutic compounds, there is still a need to provide novel and improved compounds useful as polyproline mimetics (mimetics for proline-rich peptides) or as building blocks of such mimetics which have improved binding properties, stability, pharmacokinetic and pharmacodynamic properties and corresponding biological effects.

### SUMMARY OF THE INVENTION

In light of the prior art, the technical problem underlying the present invention is to provide improved and/or alternative means which can be used as mimetics for proline-rich peptides, in particular as pharmaceutical agents for the treatment of various diseases associated with a modified protein-protein interaction.

Another problem of the present invention is the provision of improved and/or alternative means which can be used as inhibitors of proline-rich motif-based protein-protein interactions such as PPII helix-based protein-protein interactions, in particular as pharmaceutical agents for the treatment of various diseases associated with a modified protein-protein interaction.

Another problem underlying the present invention is the provision of improved and/or alternative compounds which can be used as mimetics for proline-rich peptides or as molecular building blocks of such mimetics.

Another problem underlying the present invention is the provision of improved and/or alternative compounds which can be used as mimetics for proline-rich peptides or as molecular building blocks of such mimetics, which show a high metabolic stability in biological environment such as in the presence of enzymes and upon storage.

Another problem underlying the present invention is the provision of improved and/or alternative compounds which can be used as mimetics for proline-rich peptides or as molecular building blocks of such mimetics, which show beneficial pharmacokinetic properties such as high uptake into target cells.

Another problem underlying the present invention is the provision of improved and/or alternative compounds which can be used as therapeutic agents for the treatment of cancer, infections, immune disorders, neurological disease and/or cardiometabolic disease.

These problems are solved by the features of the independent claims. Preferred embodiments of the present invention are provided in the dependent claims.

In one aspect, the invention relates to a compound according to **Formula 1** 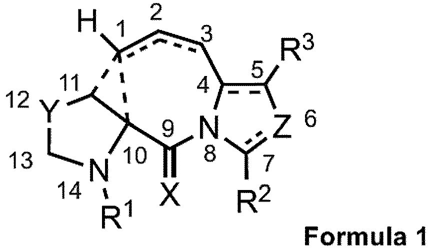 wherein between carbon **C-1** and **C-10,** or **C-1-** and **C-11** a covalent bond is present,
carbon **C-1** and **C-2,** or **C-2** and **C-3** are covalently linked by a single bond or a double bond,
up to three hydrogen atoms are replaced by F,
carbon **C-4** and **C-5,** and **Z** and **C-7** are covalently linked by a single bond or a double bond,
**X** is O, S or absent (two H covalently bound to **C-9**),
**Y** is C₁ to C₂ alkylidene, O, S, OCH₂ or SCH₂,
**Z** is C₁ to C₂ alkylidene, CH, -CH₂-CH₂-, O, S, NH, OCH₂, SCH₂ or N**R**, wherein **R** is C₁ to C₈ branched or linear alkyl (preferably C₁ to C₅ branched or linear alkyl), acyl, alkoxy carbonyl, amino carbonyl or sulfonyl, when **C-4** and **C-5,** and **Z** and **C-7** are covalently linked by a single bond, or
**Z** is N, CH or C**R**, wherein **R** is C₁ to C₈ branched or linear alkyl (preferably C₁ to C₅ branched or linear alkyl), acyl, hydroxy alkyl, alkoxy carbonyl, amino carbonyl, sulfonyl or halogen, when **C-4** and **C-5,** and **Z** and **C-7** are covalently linked by a double bond,
**R¹** is H, alkoxy carbonyl, C₁ to C₅ alkyl aminocarbonyl (preferably C₁ to C₃ alkyl aminocarbonyl), sulfonyl, C₁ to C₈ branched or linear alkyl (preferably C₁ to C₅ branched or linear alkyl), aryl, heteroaryl (preferably comprising N, S and/or O), or acyl -(C=O)-**R**, wherein **R** is C₁ to C₅ branched or linear alkyl (preferably C₁ to C₃ branched or linear alkyl), aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide,
**R²** is H, C₁ to C₈ branched or linear alkyl (preferably C₁ to C₅ branched or linear alkyl), alkoxy carbonyl, N-substituted or N-unsubstituted amino carbonyl, aryl aminocarbonyl, heteroaryl aminocarbonyl, (preferably comprising N, S and/or O), sulfonyl or acyl -(C=O)-**R**, herein **R** is C₁ to C₅ branched or linear alkyl (preferably C₁ to C₃ branched or linear alkyl), aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide, and
**R³** is H or C₁ to C₈ branched or linear alkyl (preferably C₁ to C₅ branched or linear alkyl).

The compounds of the invention are useful as polyproline mimetics (mimetics for proline-rich peptides) and as molecular building blocks for such mimetics, e.g., in combination with other polyproline mimetics or as part of a peptide. The compounds of the present invention and compounds and peptides comprising these effectively bind to proteins which are involved in intracellular protein-protein interactions mediated by proline-rich motif containing peptides such as to proteins comprising an Src homology 3 (SH3) domain, WW domain, Ena/VASP homology 1 domain (EVH1), GYF domain, UEV domain and/or profilin domain. This is demonstrated in the examples below. The compounds described herein exhibit surprisingly beneficial properties, such as the low IC50 values required for effective binding to the EnaH-EVH1 protein comprising the polypeptide binding Ena/VASP homology 1 domain (EVH1) (see table 2).

These proteins play critical roles in the regulation of cellular processes, including actin dynamics, protein turnover, and signal transduction. EnaH-EVH1 is a member of the Ena/VASP family of proteins that regulates actin dynamics and cell migration. It is crucial in processes such as embryonic development and immune responses. Overexpression or dysregulation of EnaH-EVH1 is for example linked to cancer metastasis, particularly in breast cancer, where it enhances tumor cell migration and invasion. It further plays a role in neurological disorders (such as by influencing neurite outgrowth and synapse formation) and cardiovascular diseases (such as by affecting cell adhesion and migration). The Src homology 3 (SH3) domain is found in proteins involved in intracellular signaling, particularly in the Src family kinases, such as Fyn.

The Fyn SH3 domain mediates protein-protein interactions that are essential for cell growth, differentiation, and survival. Dysregulation of Fyn and its SH3 domain is associated with cancer (by promoting uncontrolled cell growth and metastasis), neurodegenerative diseases (by influencing neuronal signaling), and immune disorders (by affecting immune cell function and inflammation). WW domain-comprising proteins are known play a role in signaling, transcriptional regulation, and protein degradation and are linked to several diseases such as cancer (by dysregulated signaling pathways) and cardiometabolic disorders (by impacting vascular inflammation and signaling).

Further, GYF domain-comprising proteins mediate protein-protein interactions involved in signal transduction and transcriptional regulation. Thereby, dysregulation of these proteins is associated with cancerous diseases (by aberrant signaling) and may influence neurological and cardiometabolic disorders by affecting glucose metabolism and vascular function.

UEV domain-comprising proteins play regulatory roles in the ubiquitin-proteasome system, influencing protein degradation and cellular homeostasis. These proteins are thus associated with cancer (by regulating the degradation of signaling molecules), neurodegenerative diseases (by influencing protein quality control and aggregation), and cardiometabolic disorders (by affecting lipid metabolism, inflammation, and cardiac function).

The compounds of the present invention specifically binding to these proteins and thereby modulating these proteins and their mediated intracellular signaling are thus particularly useful as therapeutic agents, as they precisely target the underlying molecular mechanisms that contribute to the development and progression of various diseases associated with these proteins, offering therapeutic strategies for conditions such as cancer, neurodegenerative disorders, cardiovascular diseases, immune disorders, and metabolic syndromes. The compounds described herein may also be employed to develop additional compounds, based on the scaffolds and structures herein, to develop optimized therapeutic agents.

The present invention is based on novel chemical structures, as peptide mimetic compounds according to the present invention have been described in the prior art. To the best knowledge of the inventors, no disclosure or suggestion is evident in the prior art that such compounds have beneficial pharmacokinetic properties and high effectivity as peptide mimetic compounds in the therapeutic applications according to the present invention. In light of the prior art, it would not have been possible for a person of ordinary skill in the art to predict these functional improvements evident in the structures according to the invention.

In one embodiment the compound according to **Formula 1** is characterized in that up to three hydrogen atoms bound to any of carbon atoms C-1, C-2, C-3, C-4, C-5, C-7, C-9, C-10, C-11 and C-13 are replaced by F.

In one embodiment the compound according to **Formula 1** is characterized in that
**R¹** is H, alkoxy carbonyl, C₁ to C₃ alkyl aminocarbonyl, sulfonyl, C₁ to C₅ branched or linear alkyl, aryl, heteroaryl (preferably comprising N, S and/or O), or acyl -(C=O)-**R**, wherein R is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide,
**R²** is H, C₁ to C₅ branched or linear alkyl, alkoxy carbonyl, N-substituted amino carbonyl, aryl aminocarbonyl, heteroaryl aminocarbonyl, (preferably comprising N, S and/or O), sulfonyl or acyl -(C=O)-**R**, herein **R** is C₁ to C₃ branched or linear alkyl, aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide,
**R³** is H or C₁ to C₅ branched or linear alkyl,
**X** is O, S or absent (two H covalently bound to **C-9**),
**Y** is C₁ to C₂ alkylidene, O, S, OCH₂ or SCH₂, and
**Z** is C₁ to C₂ alkylidene, CH, -CH₂-CH₂-, O, S, NH, OCH₂, SCH₂ or N**R**, wherein **R** is C₁ to C₈ branched or linear alkyl (preferably C₁ to C₅ branched or linear alkyl), acyl, alkoxy carbonyl, amino carbonyl or sulfonyl, when **C-4** and **C-5,** and **Z** and **C-7** are covalently linked by a single bond, or
**Z** is N, CH or C**R**, wherein **R** is C₁ to C₈ branched or linear alkyl (preferably C₁ to C₅ branched or linear alkyl), acyl, hydroxy alkyl, alkoxy carbonyl, amino carbonyl, sulfonyl or halogen, when **C-4** and **C-5,** and **Z** and **C-7** are covalently linked by a double bond.

In one embodiment the compound according to **Formula 1** is characterized in that
**Z** is C₁ to C₂ alkylidene, CH, -CH₂-CH₂-, O, S, NH, OCH₂, SCH₂ or N**R,** wherein **R** is C₁ to C₅ branched or linear alkyl, acyl, alkoxy carbonyl, amino carbonyl or sulfonyl, when **C-4** and **C-5,** and
**Z** and **C-7** are covalently linked by a single bond, or
**Z** is N, CH or C**R**, wherein **R** is C₁ to C₅ branched or linear alkyl, acyl, hydroxy alkyl, alkoxy carbonyl, amino carbonyl, sulfonyl or halogen, when **C-4** and **C-5,** and **Z** and **C-7** are covalently linked by a double bond.

In one embodiment the compound according to **Formula 1** is characterized in that **C-4** and **C-5,** and **Z** and **C-7** are covalently linked by a single bond and **Z** is CH.

In one embodiment the compound according to **Formula 1** is characterized in that **R¹** is alkoxy carbonyl, preferably an alkoxy carbonyl protection group, preferably selected from the group consisting of Boc (tert-butyloxycarbonyl), Fmoc (fluorenylmethoxycarbonyl), benzyl oxycarbonyl (Cbz) and allyloxy carbonyl (Alloc). In one embodiment the protection group of **R¹** is removed after synthesis of the compound (deprotection). In one embodiment after deprotection the compound is used for further synthesis of a compound useful as polyproline mimetic.

In one embodiment the compound according to **Formula 1** is characterized in that **X** is O.

In one embodiment the compound according to **Formula 1** is characterized in that **Y** is C₁ alkylidene.

In one embodiment the compound according to **Formula 1** is characterized in that **R¹** is H, alkoxy carbonyl, C₁ to C₃ alkyl aminocarbonyl, or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide.

In one embodiment the compound according to **Formula 1** is characterized in that **R²** is alkoxy carbonyl, or acyl -(C=O)-**R**, herein **R** is C₁ to C₃ branched or linear alkyl, aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide.

In one embodiment the compound according to **Formula 1** is characterized in that **R³** is H.

In one embodiment the invention relates to a compound according to **Formula 2,** 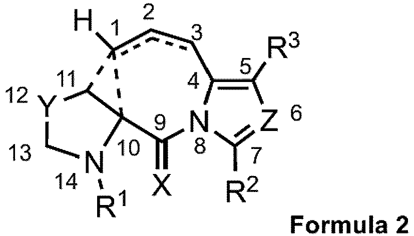 wherein
between carbon **C-1** and **C-10,** or **C-1-** and **C-11** a covalent bond is present,
carbon **C-1** and **C-2,** or **C-2** and **C-3** are covalently linked by a single bond or a double bond,
**Z** is N, CH or C**R,** wherein **R** is C₁ to C₈ branched or linear alkyl (preferably C₁ to C₅ branched or linear alkyl), acyl, hydroxy alkyl, alkoxy carbonyl, amino carbonyl, sulfonyl or halogen,
**X** is O, S or absent (two H covalently bound to **C-9**),
**Y** is C₁ to C₂ alkylidene, O, S, OCH₂ or SCH₂,
**R¹** is H, alkoxy carbonyl, C₁ to C₅ alkyl aminocarbonyl (preferably C₁ to C₃ alkyl aminocarbonyl), sulfonyl, C₁ to C₈ branched or linear alkyl (preferably C₁ to C₅ branched or linear alkyl), aryl, heteroaryl (preferably comprising N, S and/or O), or acyl -(C=O)-**R**, wherein **R** is C₁ to C₅ branched or linear alkyl (preferably C₁ to C₃ branched or linear alkyl), aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide,
**R²** is H, C₁ to C₈ branched or linear alkyl (preferably C₁ to C₅ branched or linear alkyl), alkoxy carbonyl, N-substituted or N-unsubstituted amino carbonyl, aryl aminocarbonyl, heteroaryl aminocarbonyl, (preferably comprising N, S and/or O), sulfonyl or acyl -(C=O)-**R**, herein **R** is C₁ to C₅ branched or linear alkyl (preferably C₁ to C₃ branched or linear alkyl), aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide, and
**R³** is H or C₁ to C₈ branched or linear alkyl (preferably C₁ to C₅ branched or linear alkyl).

In one embodiment the compound according to **Formula 2** is characterized in that
**R¹** is H, alkoxy carbonyl, C₁ to C₃ alkyl aminocarbonyl, sulfonyl, C₁ to C₅ branched or linear alkyl, aryl, heteroaryl (preferably comprising N, S and/or O), or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide,
**R²** is H, C₁ to C₅ branched or linear alkyl, alkoxy carbonyl, N-substituted amino carbonyl, aryl aminocarbonyl, heteroaryl aminocarbonyl, (preferably comprising N, S and/or O), sulfonyl or acyl -(C=O)-**R**, herein **R** is C₁ to C₃ branched or linear alkyl, aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide,
**R³** is H or C₁ to C₅ branched or linear alkyl,
**X** is O, S or absent (two H covalently bound to **C-9**),
**Y** is C₁ to C₂ alkylidene, O, S, OCH₂ or SCH₂, and
**Z** is N, CH or CR, wherein **R** is C₁ to C₅ branched or linear alkyl, acyl, hydroxy alkyl, alkoxy carbonyl, amino carbonyl, sulfonyl or halogen.

In one embodiment the compound according to **Formula 2** is characterized in that **Z** is N, CH or C**R**, wherein **R** is C₁ to C₅ branched or linear alkyl, acyl, hydroxy alkyl, alkoxy carbonyl, amino carbonyl, sulfonyl or halogen. In one embodiment the compound according to **Formula 2** is characterized in that **Z** is CH.

In one embodiment the compound according to **Formula 2** is characterized in that **R¹** is alkoxy carbonyl, preferably an alkoxy carbonyl protection group, preferably selected from the group consisting of Boc (tert-butyloxycarbonyl), Fmoc (fluorenylmethoxycarbonyl), benzyl oxycarbonyl (Cbz) and alloxy carbonyl (Alloc). In one embodiment the protection group of **R¹** is removed after synthesis of the compound (deprotection). In one embodiment after deprotection the compound is used for further synthesis of a compound useful as polyproline mimetic.

In one embodiment the compound according to **Formula 2** is characterized in that **X** is O.

In one embodiment the compound according to **Formula 2** is characterized in that **Y** is C₁ alkylidene.

In one embodiment the compound according to **Formula 2** is characterized in that **R¹** is H, alkoxy carbonyl, C₁ to C₃ alkyl aminocarbonyl, or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide.

In one embodiment the compound according to **Formula 2** is characterized in that **R²** is alkoxy carbonyl, or acyl -(C=O)-**R**, herein **R** is C₁ to C₃ branched or linear alkyl, aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide.

In one embodiment the compound according to **Formula 2** is characterized in that **R³** is H.

In one embodiment the invention relates to a compound according to **Formula 3,** 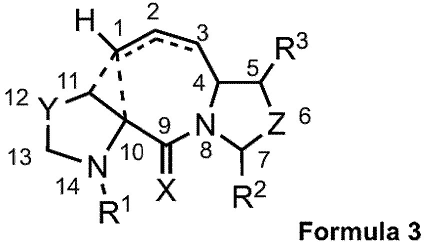 wherein
between carbon **C-1** and **C-10,** or **C-1-** and **C-11** a covalent bond is present,
carbon **C-1** and **C-2,** or **C-2** and **C-3** are covalently linked by a single bond or a double bond,
**Z** is C₁ to C₂ alkylidene, CH, -CH₂-CH₂-, O, OCH₂ or SCH₂,
**X** is O, S or absent (two H covalently bound to **C-9**),
**Y** is C₁ to C₂ alkylidene, O, S, OCH₂ or SCH₂,
**R¹** is H, alkoxy carbonyl, C₁ to C₅ alkyl aminocarbonyl (preferably C₁ to C₃ alkyl aminocarbonyl), sulfonyl, C₁ to C₈ branched or linear alkyl (preferably C₁ to C₅ branched or linear alkyl), aryl, heteroaryl (preferably comprising N, S and/or O), or acyl -(C=O)-**R**, wherein **R** is C₁ to C₅ branched or linear alkyl (preferably C₁ to C₃ branched or linear alkyl), aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide,
**R²** is H, C₁ to C₈ branched or linear alkyl (preferably C₁ to C₅ branched or linear alkyl), alkoxy carbonyl, N-substituted or N-unsubstituted amino carbonyl, aryl aminocarbonyl, heteroaryl aminocarbonyl, (preferably comprising N, S and/or O), sulfonyl or acyl -(C=O)-**R**, herein **R** is C₁ to C₅ branched or linear alkyl (preferably C₁ to C₃ branched or linear alkyl), aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide, and
**R³** is H or C₁ to C₈ branched or linear alkyl (preferably C₁ to C₅ branched or linear alkyl).

In one embodiment the compound according to **Formula 3** is characterized in that
**R¹** is H, alkoxy carbonyl, C₁ to C₃ alkyl aminocarbonyl, sulfonyl, C₁ to C₅ branched or linear alkyl, aryl, heteroaryl (preferably comprising N, S and/or O), or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O) a residual group of an amino acid, an amino acid (preferably an alpha-, beta- or gamma-amino acid), or a peptide,
**R²** is H, C₁ to C₅ branched or linear alkyl, alkoxy carbonyl, N-substituted amino carbonyl, aryl aminocarbonyl, heteroaryl aminocarbonyl, (preferably comprising N, S and/or O), sulfonyl or acyl -(C=O)-**R**, herein **R** is C₁ to C₃ branched or linear alkyl, aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide,
**R³** is H or C₁ to C₅ branched or linear alkyl,
**X** is O, S or absent (two H covalently bound to **C-9**),
**Y** is C₁ to C₂ alkylidene, O, S, OCH₂ or SCH₂, and
**Z** is C₁ to C₂ alkylidene, CH, -CH₂-CH₂-, O, OCH₂ or SCH₂.

In one embodiment the compound according to **Formula 3** is characterized in that **Z** is CH.

In one embodiment the compound according to **Formula 3** is characterized in that **R¹** is alkoxy carbonyl, preferably an alkoxy carbonyl protection group, preferably selected from the group consisting of Boc (tert-butyloxycarbonyl), Fmoc (fluorenylmethoxycarbonyl), benzyl oxycarbonyl (Cbz) and alloxy carbonyl (Alloc). In one embodiment the protection group of **R¹** is removed after synthesis of the compound (deprotection). In one embodiment after deprotection the compound is used for further synthesis of a compound useful as polyproline mimetic.

In one embodiment the compound according to **Formula 3** is characterized in that **X** is O.

In one embodiment the compound according to **Formula 3** is characterized in that **Y** is C₁ alkylidene.

In one embodiment the compound according to **Formula 3** is characterized in that **R¹** is H, alkoxy carbonyl, C₁ to C₃ alkyl aminocarbonyl, or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide.

In one embodiment the compound according to **Formula 3** is characterized in that **R²** is alkoxy carbonyl, or acyl -(C=O)-**R**, herein **R** is C₁ to C₃ branched or linear alkyl, aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide

In one embodiment the compound according to **Formula 3** is characterized in that **R³** is H.

In one embodiment the invention relates to a compound according to **Formula 4a** or**4b,** 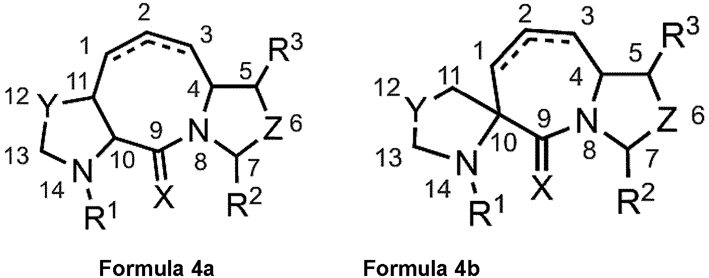 wherein
carbon **C-1** and **C-2,** or **C-2** and **C-3** are covalently linked by a single bond or a double bond,
**Z** is C₁ to C₂ alkylidene, CH, -CH₂-CH₂-, O, OCH₂ or SCH₂, and
**X** is O, S or absent (two H covalently bound to **C-9**),
Y is C₁ to C₂ alkylidene, O, S, OCH₂ or SCH₂,
**R¹** is H, alkoxy carbonyl, C₁ to C₅ alkyl aminocarbonyl (preferably C₁ to C₃ alkyl aminocarbonyl), sulfonyl, C₁ to C₈ branched or linear alkyl (preferably C₁ to C₅ branched or linear alkyl), aryl, heteroaryl (preferably comprising N, S and/or O), or acyl -(C=O)-**R**, wherein **R** is C₁ to C₅ branched or linear alkyl (preferably C₁ to C₃ branched or linear alkyl), aryl, heteroaryl (preferably comprising N, S and/or O), a bicyclic or tricyclic ring structure (preferably comprising N, S and/or O),a residual group of an amino acid, an amino acid (preferably an alpha-, beta- or gamma-amino acid), or a peptide,
**R²** is H, C₁ to C₈ branched or linear alkyl (preferably C₁ to C₅ branched or linear alkyl), alkoxy carbonyl, N-substituted or N-unsubstituted amino carbonyl, aryl aminocarbonyl, heteroaryl aminocarbonyl, (preferably comprising N, S and/or O), sulfonyl or acyl -(C=O)-**R**, herein **R** is C₁ to C₅ branched or linear alkyl (preferably C₁ to C₃ branched or linear alkyl), aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide, and
**R³** is H or C₁ to C₈ branched or linear alkyl (preferably C₁ to C₅ branched or linear alkyl).

In one embodiment the compound according to **Formula 4a** or **4b** is characterized in that
**R¹** is H, alkoxy carbonyl, C₁ to C₃ alkyl aminocarbonyl, sulfonyl, C₁ to C₅ branched or linear alkyl, aryl, heteroaryl (preferably comprising N, S and/or O), or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide,
**R²** is H, C₁ to C₅ branched or linear alkyl, alkoxy carbonyl, N-substituted amino carbonyl, aryl aminocarbonyl, heteroaryl aminocarbonyl, (preferably comprising N, S and/or O), sulfonyl or acyl -(C=O)-**R**, herein **R** is C₁ to C₃ branched or linear alkyl, aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide,
**R³** is H or C₁ to C₅ branched or linear alkyl,
**X** is O, S or absent (two H covalently bound to **C-9**),
**Y** is C₁ to C₂ alkylidene, O, S, OCH₂ or SCH₂, and
**Z** is C₁ to C₂ alkylidene, CH, -CH₂-CH₂-, O, OCH₂ or SCH₂.

In one embodiment the compound according to **Formula 4a or 4b** is characterized in that **Z** is CH.

In one embodiment the compound according to **Formula 4a or 4b** is characterized in that **R¹** is alkoxy carbonyl, preferably an alkoxy carbonyl protection group, preferably selected from the group consisting of Boc (tert-butyloxycarbonyl), Fmoc (fluorenylmethoxycarbonyl), benzyl oxycarbonyl (Cbz) and alloxy carbonyl (Alloc). In one embodiment the protection group of **R¹** is removed after synthesis of the compound (deprotection). In one embodiment after deprotection the compound is used for further synthesis of a compound useful as polyproline mimetic.

In one embodiment the compound according to **Formula 4a or 4b** is characterized in that **X** is O.

In one embodiment the compound according to **Formula 4a or 4b** is characterized in that **Y** is C₁ alkylidene.

In one embodiment the compound according to **Formula 4a or 4b** is characterized in that **R¹** is H, alkoxy carbonyl, C₁ to C₃ alkyl aminocarbonyl, or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide.

In one embodiment the compound according to **Formula 4a or 4b** is characterized in that **R²** is alkoxy carbonyl, or acyl -(C=O)-**R**, herein **R** is C₁ to C₃ branched or linear alkyl, aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide.

In one embodiment the compound according to **Formula 4a or 4b** is characterized in that **R³** is H.

In one embodiment the invention relates to a compound according to **Formula 5a or 5b** 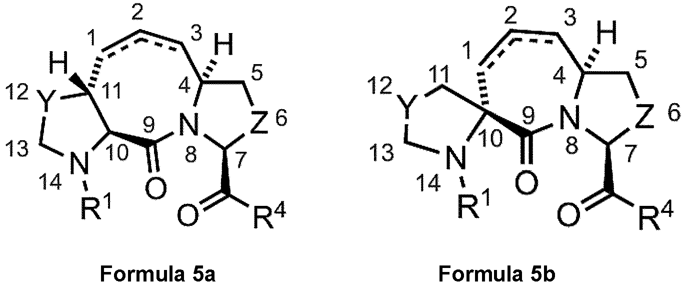 wherein
carbon **C-1** and **C-2,** or **C-2** and **C-3** are covalently linked by a single bond or a double bond,
**R¹** is H, alkoxy carbonyl, C₁ to C₅ alkyl aminocarbonyl (preferably C₁ to C₃ alkyl aminocarbonyl), sulfonyl, C₁ to C₈ branched or linear alkyl (preferably C₁ to C₅ branched or linear alkyl), aryl, heteroaryl (preferably comprising N, S and/or O), or acyl -(C=O)-**R**, wherein **R** is C₁ to C₅ branched or linear alkyl (preferably C₁ to C₃ branched or linear alkyl), aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide, and
**R⁴** is OH, oxy alkyl, alkoxy, amine (preferably NH₂, NH-alkyl or N(alkyl)₂), a residue attached by an N, wherein the residue is an amino acid, a peptide, a bicyclic or tricyclic ring structure (preferably comprising N, S and/or O), a residual group of an amino acid, an amino acid (preferably an alpha-, beta- or gamma-amino acid), a peptide or an amino alcohol.

In one embodiment the compound according to **Formula 5a or 5b** is characterized in that carbon **C-1** and **C-2,** or **C-2** and **C-3** are covalently linked by a single bond or a double bond,
**R¹** is H, alkoxy carbonyl, C₁ to C₃ alkyl aminocarbonyl, or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide, and
**R⁴** is OH, oxy alkyl, NH₂, NH-alkyl or N(alkyl)₂, a residue attached by an N, wherein the residue is an amino acid, a peptide, a bicyclic or tricyclic ring structure (preferably comprising N, S and/or O), a residual group of an amino acid, an amino acid (preferably an alpha-, beta- or gamma-amino acid), a peptide or an amino alcohol.

In one embodiment the compound according to **Formula 5a or 5b** is characterized in that **R¹** is alkoxy carbonyl, preferably an alkoxy carbonyl protection group, preferably selected from the group consisting of Boc (tert-butyloxycarbonyl), Fmoc (fluorenylmethoxycarbonyl), benzyl oxycarbonyl (Cbz) and alloxy carbonyl (Alloc). In one embodiment the protection group of **R¹** is removed after synthesis of the compound (deprotection). In one embodiment after deprotection the compound is used for further synthesis of a compound useful as polyproline mimetic.

In one embodiment the compound according to **Formula 5a or 5b** is characterized in that **R¹** is H, alkoxy carbonyl, C₁ to C₃ alkyl aminocarbonyl, or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide.

In one embodiment the compound according to **Formula 5a or 5b** is characterized in that **R⁴** is alkoxy or a residue attached by an N, wherein the residue is an amino acid, a peptide, a bicyclic or tricyclic ring structure (preferably comprising N, S and/or O), a residual group of an amino acid, an amino acid (preferably an alpha-, beta- or gamma-amino acid), a peptide or an amino alcohol.

In one embodiment the compound is one of 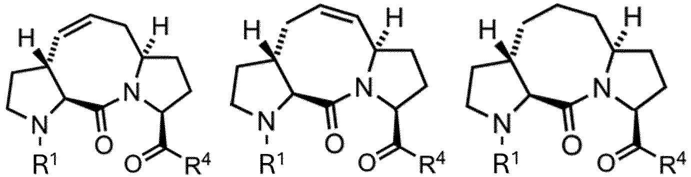 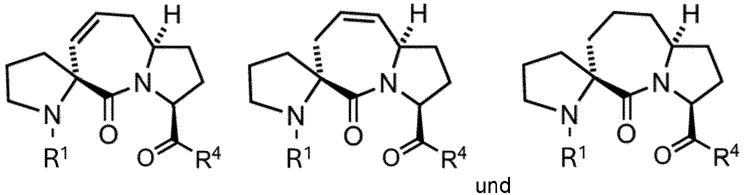 wherein
**R¹** is H, alkoxy carbonyl, C₁ to C₅ alkyl aminocarbonyl (preferably C₁ to C₃ alkyl aminocarbonyl), sulfonyl, C₁ to C₈ branched or linear alkyl (preferably C₁ to C₅ branched or linear alkyl), aryl, heteroaryl (preferably comprising N, S and/or O), or acyl -(C=O)-**R**, wherein **R** is C₁ to C₅ branched or linear alkyl (preferably C₁ to C₃ branched or linear alkyl), aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide, and
**R⁴** is OH, oxy alkyl, amine (preferably NH₂, NH-alkyl or N(alkyl)₂), a residue attached by an N, wherein the residue is an amino acid, a peptide, a bicyclic or tricyclic ring structure (preferably comprising N, S and/or O), a residual group of an amino acid, an amino acid (preferably an alpha- , beta- or gamma-amino acid), a peptide or an amino alcohol.

In one embodiment **R¹** is alkoxy carbonyl, preferably an alkoxy carbonyl protection group, preferably selected from the group consisting of Boc (tert-butyloxycarbonyl), Fmoc (fluorenylmethoxycarbonyl), benzyl oxycarbonyl (Cbz) and alloxy carbonyl (Alloc). In one embodiment the protection group of **R¹** is removed after synthesis of the compound (deprotection). In one embodiment after deprotection the compound is used for further synthesis of a compound useful as polyproline mimetic.

In one embodiment **R¹** is H, alkoxy carbonyl, C₁ to C₃ alkyl aminocarbonyl, or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide.

In one embodiment **R⁴** is alkoxy or a residue attached by an N, wherein the residue is an amino acid, a peptide, a bicyclic or tricyclic ring structure (preferably comprising N, S and/or O), a residual group of an amino acid, an amino acid (preferably an alpha-, beta- or gamma-amino acid), a peptide or an amino alcohol.

In one embodiment **R¹** is (S)-2-chloro-phenylalanine or N-acetylated (S)-2-chloro-phenylalanine, optionally substituted with an amino acid or a peptide, and **R⁴** is alkoxy, or a residue attached via an N atom, wherein the residue is an amino acid, a peptide, a bicyclic or tricyclic ring group (preferably comprising N and optionally S and/or O).

In one embodiment the compound is one of 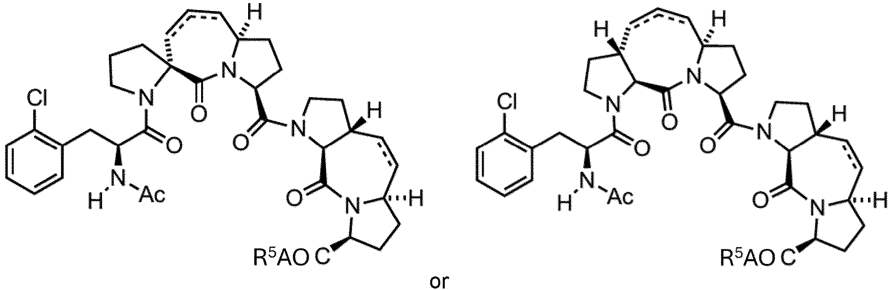 wherein
**R⁵** is H, C₁ to C₈ branched or linear alkyl (preferably C₁ to C₅ branched or linear alkyl), a bicyclic or tricyclic ring structure (preferably comprising N, S and/or O), a residual group of an amino acid, an amino acid (preferably an alpha-, beta- or gamma-amino acid), or a peptide, and
**A** is O or NH.

In one embodiment **R⁵** is H, C₁ to C₅ branched or linear alkyl, a bicyclic or tricyclic ring structure (preferably comprising N, S and/or O), a residual group of an amino acid, an amino acid (preferably an alpha-, beta- or gamma-amino acid), or a peptide, and **A** is NH.

In one embodiment **R⁵** is H, C₁ to C₅ branched or linear alkyl, a bicyclic or tricyclic ring structure (preferably comprising N, S and/or O), a residual group of an amino acid, an amino acid (preferably an alpha-, beta- or gamma-amino acid), or a peptide.

In one embodiment **A** is NH.

The structures of the invention, such as the structures of table 1, are represented in some contexts of the description of the invention as protected compound, such as a Boc- protected, an Fmoc-protected or a trimethyl silyl (TBS)-protected compound. When these structures are used in a polyproline mimetic or in a peptide, it is preferred that the structures correspondingly do not include any protective group(s).

In one aspect the invention relates to a pharmaceutical composition, comprising one or more compounds according to any one of the preceding claims or a pharmaceutically acceptable salt thereof and a pharmaceutically acceptable carrier.

In one aspect the invention relates to a compound according to any one of **Formulas 1, 2, 3, 4a, 4b, 5a** and **5b** or the pharmaceutical composition for use in the treatment of a condition associated with a modified intracellular signal transduction process mediated by a poly-proline helix structure.

In one embodiment the proline-rich helix structure is a polyproline-type II (PPT II) helix structure.

In one embodiment the proline-rich helix structure binds to a protein having a Src homology 3 (SH3) domain, WW domain, Ena/VASP homology 1 domain (EVH1), GYF domain, UEV domain and/or profilin domain.

In one embodiment the disease is selected from the group consisting of an infectious disease, immune disease, neurological disease, cardiometabolic disease and a cancerous disease.

In one embodiment the infectious disease is selected from the group consisting of COVID, HIV/AIDs, influenza, pneumonia, cholera, botulism, tuberculosis, malaria, herpes, measles, menigitis, monkeypox, chicken pox, listeriosis, shingelosis, syphilis, Chlamydia infection, pseudomonas infection, anthras, tetanus and leptospirosis. Preferred embodiments relate to infectious diseases associated with a modified intracellular signal transduction process mediated by a proline-rich helix structure.

In one embodiment the immune disease is selected from the group consisting of arthritis, psoriasis, asthma, inflammatory bowel disease, scleroderma, multiple sclerosis, lupus, type 1 diabetes, Graves'disease, Addison's disease, Sjörgen syndrome, myasthenia Gravis, pernicious anemia, vasculitis, Aczema and severe combined immunodeficiency. Preferred embodiments relate to an immune disease associated with a modified intracellular signal transduction process mediated by a proline-rich helix structure.

In one embodiment the neurological disease is selected from the group consisting of Alzheimer's disease, Parkinson's disease, Huntington's disease and amyotrophic lateral sclerosis (ALS). Preferred embodiments relate to neurological diseases associated with a modified intracellular signal transduction process mediated by a proline-rich helix structure.

In one embodiment the cardiometabolic disease is selected from the group consisting of a myocardial infaction, stroke, artheriosclerosis, a circulatory disease, diabetes, insulin resistance, hypertension, obesity, fatty liver disease, hyperlipidemia, dyslipidemia, type 2 diabetes and nephropathy. Preferred embodiments relate to cardiometabolic diseases associated with a modified intracellular signal transduction process mediated by a proline-rich helix structure.

In one embodiment the cancerous disease is a solid cancer, a sarcoma, a lymphoma or a leukemia, preferably selected from the group consisting of a brain tumor, breast cancer, esophageal cancer, head and neck cancer, lung cancer, colon cancer, gallbladder cancer, ovarian cancer, pancreatic cancer, prostate cancer, rectal cancer, skin cancer, stomach cancer, testicular cancer, osteosarcoma, liposarcoma, leiomyosarcoma, myelodysplastic syndrome (MDS), multiple myeloma (MM), acute myeloid leukemia (AML), chronic myeloid leukemia (CML), diffuse large B-cell lymphoma (DLBCL), mantle cell lymphoma (MCL), T-cell lymphoma (TCL) and peripheral T-cell lymphoma (PTCL). Preferred embodiments relate to cancers associated with a modified intracellular signal transduction process mediated by a proline-rich helix structure.

The invention further relates to a compound or composition as described herein for use in the treatment of tumor diseases, characterized in that the compound inhibits the metastasis of a tumor. The invention further relates to a compound or composition as described herein for use in the treatment of tumor diseases, characterized in that the metastasis of a tumor is inhibited by inhibiting the chemotaxis and/or motility of the tumor cells based on a disruption of the actin filament synthesis.

In an embodiment, the invention relates to a compound as described herein, characterized in that it functions or is used as a ligand for a polyproline binding domain of a peptide. In an embodiment, the invention relates to a compound as described herein, characterized in that it functions or is used as a ligand for a polyproline-type II (PPT II) helix binding domain of a peptide.

In one embodiment the invention relates to compounds as a ligand for an Ena/VASP-EVH1 domain. In in one embodiment, the invention relates to the use of the compounds according to the invention as a ligand for a domain selected from the group consisting of a Src homology 3(SH3) domain, WW domain, Ena/VASP homology 1 domain (EVH1), GYF domain, UEV domain and a profilin domain.

The invention therefore encompasses corresponding in vitro, ex vivo and in vivo methods, employing said properties.

The invention also relates to the use of the new structural mimics of the polyproline II helix as inhibitors of protein-protein interactions, wherein preferably an Src homology 3 (SH3) domain, WW domain, Ena/VASP homology 1 domain (EVH1), GYF domain, UEV are involved. Proteins that contain these domains, such as, e.g., VASP (EVH1 domain) or YAP (WW) play an important role in regulating cell motility (especially VASP) and cell proliferation (especially YAP), among others. For example, VASP is highly overexpressed in highly invasive breast cancer cells.

The invention therefore encompasses corresponding in vitro, ex vivo and in vivo methods, employing said properties. In one embodiment, the invention further relates to an in vitro method for modifying an intracellular signal transduction process mediated by a proline-rich helix structure, comprising contacting a compound of the invention with a biological cell.

In a preferred embodiment of the invention, the compounds are used as polyproline mimetics (mimetics of proline-rich peptides). Advantageously, proline-rich amino acid sequences are found in particular in peptides which are involved in signal transduction processes, in particular intracellular signal transduction processes. For the purposes of the invention, the term mimetics can also be understood to mean analogs. The use of the compounds according to the invention is preferred for the treatment of diseases which are associated with a modification of intracellular signal transduction processes which are mediated by proline-rich helix structures, selected from the group consisting of an infectious disease, immune disease, neurological disease, cardiometabolic disease and a cancerous disease.

In one embodiment the invention relates to a method of treating and/or preventing a medical condition associated with a modified intracellular signal transduction process mediated by a proline-rich helix structure, preferably a polyproline-type II (PPT II) helix structure, wherein the medical condition is selected from the group consisting of an infectious disease, immune disease, neurological disease, cardiometabolic disease and a cancerous disease, said method comprising the administration of one or more of the above compounds or compounds falling under the formulae described above to a subject in need thereof, in particular the compounds of Formulas 1, 2, 3, 4a, 4b, 5a and 5b and preferred embodiments and combinations thereof described above.

In one embodiment the invention relates to a method of treatment of preventing a medical condition associated with a modified intracellular signal transduction process mediated by a proline-rich helix structure, preferably a polyproline-type II (PPT II) helix structure, in a subject in need thereof, wherein the medical condition is selected from the group consisting of an infectious disease, immune disease, neurological disease, cardiometabolic disease and a cancerous disease, comprising
(a) identifying the subject as having a modified intracellular signal transduction process mediated by a proline-rich helix structure
(b) selectively inhibiting the modified intracellular signal transduction process mediated by a proline-rich helix structure, in the subject by administering to the subject a compound according to any one of **Formulas 1, 2, 3, 4a, 4b, 5a** and **5b.**

In one embodiment the compound according to any one of Formulas 1, 2, 3, 4a, 4b, 5a and 5b selectively modulate (preferably inhibit) proline-rich helix structure mediated protein-protein interactions, preferably polyproline-type II (PPT II) helix mediated protein-protein interactions.

In embodiments the compounds according to the present invention exhibit the desired property of modulating (preferably inhibiting) proline-rich helix structure mediated protein-protein interactions. A skilled person is capable of determining whether any given compound exhibits the desired properties of the invention using the guidance provided herein and their common general knowledge.

In one aspect, the invention relates to an in vitro method for modulating (preferably inhibiting) a modified intracellular signal transduction process mediated by a proline-rich helix structure, comprising the administration of a compound according to any one of the preceding claims, or a composition comprising said compound, to a cell in which an intracellular signal transduction process mediated by a proline-rich helix structure is to be modulated.

Table 1 shows a number of exemplary compounds of the invention, suitable for the medical use described herein. Any structure may represent an embodiment of the invention and/or be employed in any of the aspects or embodiments of the invention described herein.

**Table 1: Preferred compounds of the invention**

| **Example compound** | **Abbreviation** | **Name** | **Structure** |
|---|---|---|---|
| 1 | PSDA416 | Boc[ProM-40]OtBu | 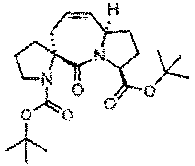 |
| 2 | PSDA419 | H[ProM-40]OMe | 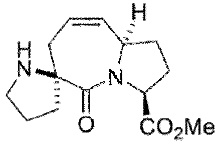 |
| 3 | PSDA422 | Fmoc[2Cl-F][ProM-40]OMe | |
| | | | 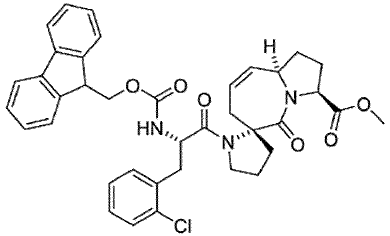 |
| 4 | PSDA424 | Fmoc[2Cl-F][ProM-40]OH | 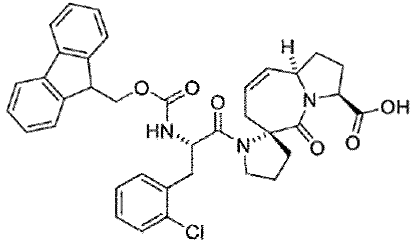 |
| 5 | | Fmoc[2Cl-F][ProM-40][ProM-1]OMe | 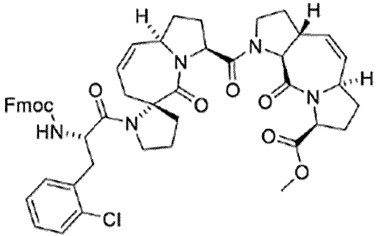 |
| 6 | PRSN-499 | Ac[2Cl-F][ProM-40][ProM-1]OMe | 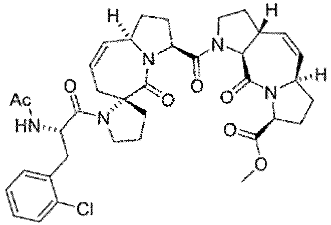 |
| 7 | PSDA561b | Boc[ProM-24]OtBu | 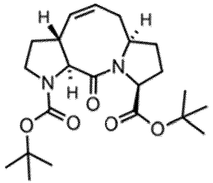 |
| 8 | PSDA565aa | Boc[dH-ProM-24]OtBu | 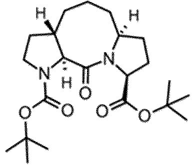 |
| 9 | PSDA388bn | Boc[ProM-40']OtBu | 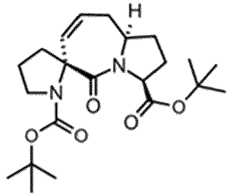 |
| 10 | PSDA390b | Boc[dH-ProM-40]OtBu | 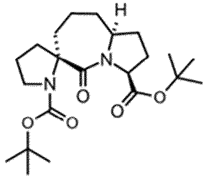 |
| 11 | PSDA403 | Boc[epi -ProM-40']OtBu | 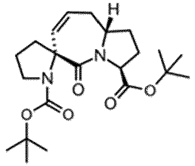 |
| 12 | PSDA410 | Boc[epi-dH-ProM-40]OtBu | 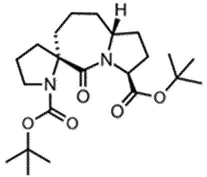 |
| 13 | PRSN-511 | Ac[2Cl-F][dH-ProM-40][dH-ProM-28]NH | 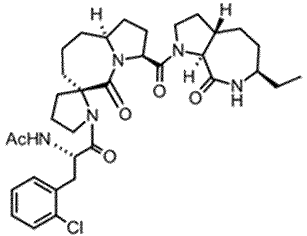 |
| 14 | PRSN-534 | Ac[2Cl-F][dH-epi -ProM-40][dH-ProM-28]NH | 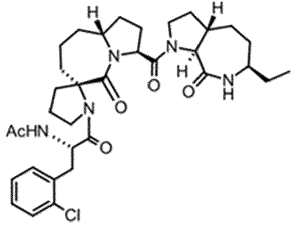 |

All features described in the present specification may be employed to define any other embodiment or aspect of the invention, for example, features used to describe the medical use of one compound may be used to describe a medical use of another compound, and vice versa. Features used to describe any one or more compounds may be combined with any one or more of the medical uses of the invention. Similarly, structural features of the compounds may be used to describe the methods of the invention, other compounds of the invention or pharmaceutical composition and vice versa, according to the understanding of a skilled person.

### DETAILED DESCRIPTION

The invention relates to chemical compounds useful as structural mimetics of peptides comprising proline-rich helix structures, preferably polyproline-type II (PPII) helix structures. The invention further relates to chemical compounds useful as inhibitors of proline-rich motif mediated protein-protein interactions. The invention further relates to the medical use of the chemical compounds in the treatment of a medical condition associated with a modified intracellular signal transduction process mediated by a proline-rich helix structure (also termed polyproline helix structure) such as an infectious disease, immune disease, neurological disease, cardiometabolic disease and/or a cancerous disease.

All patent literature cited herein is incorporated by reference. For example, diproline mimetics and molecular building blocks for such mimetics, e.g., as ligands for Ena/VASP EVH1, are disclosed in WO 2008/040332, WO 2013/030111, WO2016/092069 and WO2019/162524. The synthetic schemes and substituents disclosed therein may also be relevant to the mimetics of the present invention. The aforementioned patent literature and any US counterparts, and other relevant literature, is thereby incorporated by reference.

### General terms:

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of'.

Thus, the terms "comprising"/"including"/"having" mean that any further component (or likewise features, integers, steps and the like) can/may be present. The term "consisting of" means that no further component (or likewise features, integers, steps and the like) is present.

The term "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Thus, the term "consisting essentially of" means those specific further components (or likewise features, integers, steps and the like) can be present, namely those not materially affecting the essential characteristics of the composition, device or method. In other words, the term "consisting essentially of" (which can be interchangeably used herein with the term "comprising substantially"), allows the presence of other components in the composition, device or method in addition to the mandatory components (or likewise features, integers, steps and the like), provided that the essential characteristics of the device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts. As used herein, the "patient" or "subject" may be a vertebrate. In the context of the present invention, the term "subject" includes both humans and animals, particularly mammals, and other organisms.

### Chemical compounds

With respect to the chemical compounds described herein, the term "alkyl" refers to a branched or unbranched hydrocarbon group of preferably 1 to 5 carbon atoms, such as methyl, ethyl, n-propyl, isopropyl, n-butyl, isobutyl, sec-butyl, pentyl, and the like. Preferred alkyl groups have 1-5 carbon atoms, more preferably 1-4 or 1-3, 2 or 1 carbon atoms. In one embodiment the alkyl group may be saturated. In one embodiment the alkyl group may be unsaturated when the alkyl group comprises three or more carbon atoms. In one embodiment any one or more of the alkyl groups described herein may be "substituted alkyls", wherein one or more hydrogen atoms of the alkyl are substituted with a substituent such as halogen (preferably F, Cl and/or Br), cycloalkyl (preferably cyclopropane and/or cyclobutane), hetero cycloalkyl (preferably oxetane), alkoxy, hydroxyl, aryl, carboxyl, OR and/or SR, wherein R is preferably H, alkyl, cycloalkyl, hetero cycloalkyl, alkoxy, aryl, heteroaryl or amine.

The term "cycloalkyl" refers to a structure derived from a cycloalkane by removal of an atom of hydrogen, thereby forming preferably cyclopropyl, cyclobutyl, cyclopentyl, cyclohexyl or cycloheptyl, or the like. The term hetero cycloalkyl refers to a saturated cycloalkane structure comprising one or more heteroatoms, preferably N, S and/or O.

The term "alkoxy" refers to a straight, branched or cyclic hydrocarbon configuration and combinations thereof, including preferably 1-7 carbon atoms, more preferably 1-6, 1-5, 1-4 or 1-3 carbon atoms, that include an oxygen atom at the point of attachment (such as O-alkyl). An example of an "alkoxy group" is represented by the formula -OR, or -ROR (such as -CH₂OR), where R can be an alkyl group, optionally substituted with halogen, aryl (also referred to as aryl oxy), cycloalkyl, halogenated alkyl. Suitable alkoxy groups include methoxy, ethoxy, n-propoxy, i-propoxy, n-butoxy, i-butoxy, sec-butoxy, cyclohexyloxy, and the like.

The term "aryl" refers to any carbon-based aromatic group including, but not limited to, benzene, naphthalene, and the like. The term "aromatic" also includes "heteroaryl group," which is defined as an aromatic group that has at least one heteroatom incorporated within the ring of the aromatic group. Examples of heteroatoms include, but are not limited to, nitrogen, oxygen, sulfur. The aryl group can be substituted with one or more groups including, but not limited to, alkyl, aryl, halogen, nitro, hydroxy, carboxylic acid, or alkoxy, or the aryl group can be unsubstituted.

The term "heteroaryl" is understood to unsaturated (heteroaryl) hydrocarbon rings containing from 3 to 15 carbon atoms in a mono- or bicyclic, fused, bridged or spirocyclic ring in which 1 to 5 carbon atoms of the 3 to 15 ring carbon atoms are replaced by heteroatoms such as nitrogen, oxygen or sulfur in which further the heteroatoms can be oxidized, for example N=O, S=O, SO₂. Non-limiting examples of heterocycles are acridinyl, azaindole (1H-pyrrolopyridinyl), azabenzimidazolyl, azaspirodecanyl, azepinyl, azetidinyl, aziridinyl, benzimidazolyl, benzofuranyl, dihydrobenzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo[1,3,4]oxathiazinyl, 6H-1,5,2-dithiazinyl, dihydrofuro[2,3-b]-tetrahydrofuranyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,3-triazolyl, 1,2,4-triazolyl, 1,2,5-triazolyl, 1,3,4-triazolyl and xanthenyl.

The term "amine" refers to a group of the formula -NRR', where R and R' can be, independently, hydrogen or an alkyl, alkenyl, alkynyl, aryl, aralkyl, cycloalkyl, halogenated alkyl, or hetero cycloalkyl group described above. The term primary amine refers to a group of the formula -NH₂. The term secondary amine refers to a group of the formula -NRH, wherein R can be an alkyl, alkenyl, alkynyl, aryl, aralkyl, cycloalkyl, halogenated alkyl, or hetero cycloalkyl group described above. The term secondary amine refers to a group of the formula -NRR', wherein R and R' can be, independently an alkyl, alkenyl, alkynyl, aryl, aralkyl, cycloalkyl, halogenated alkyl, or hetero cycloalkyl group described above.

The term "amide" or "amido" is represented by the formula -C(O)NRR', where R and R' independently can be a hydrogen, alkyl, alkenyl, alkynyl, aryl, aralkyl, cycloalkyl, halogenated alkyl, or heterocycloalkyl group described above. A suitable amido group is acetamido.

"Carbonyl" refers to a group of the formula -C(O)-. Carbonyl-containing groups include any substituent containing a carbon-oxygen double bond (C=O), including acyl groups, amides, carboxy groups, carboxylic acid esters (also termed alkoxy carbonyl), ureas, carbamates, carbamate ester, carbonates, carboxamides, imides and ketones and aldehydes, such as substituents based on -COR or-RCHO where R is an aliphatic group, heteroaliphatic group, alkyl, heteroalkyl, hydroxyl, or a secondary, tertiary, or quaternary amine, phenyl, a substituted phenyl (substituted with, for example, halogen, C1-C3 alkyl, alkoxy, amine), carboxyl, alkoxycarbonyl, amine, aryl.

The term "amino alkyl" refers to alkyl groups as defined above where at least one hydrogen atom is replaced with an amino group.

The term "aminocarbonyl" refers to an amino group which is linked via a carbonyl group. Representative but non-limiting examples of aminocarbonyl are diethylaminocarbonyl, benzylaminocarbonyl, ethylaminocarbonyl.

The term "alkyl aminocarbonyl" or "aminocarbonylalkyl" refers to an aminocarbonyl group which is linked via an alkyl group. Representative but not limiting examples of aminocarbonylalkyl are 2-amino-2-oxoethyl, 2-(benzylamino)-2-oxoethyl, 2-(methylamine)-2-oxoethyl, 4-amino-4-oxobutyl, 4-(dimethylamino)- 4-oxobutyl.

The term "aminoalkylcarbonyl" refers to an amino group (-NH₂) which is linked to a carbonyl (-C=O) group via an alkyl chain.

The term "alkylcarbonyl" or "alkyl carboxyl" refers to an alkyl group which is linked via a carbonyl group. Representative but not limiting examples of alkylcarbonyl are acetyl, 1-oxopropyl, 2,2-dimethyl- 1-oxopropyl, 1-oxobutyl, 1-oxopentyl.

The term "alkoxy carbonyl" also termed "carboxylic acid ester" refers to the general structure of - C(=O)-OR, where R is an alkyl or aryl group.

The term "alkyl carboxyl ester" refers to a carboxylic acid ester which is linked via an alkyl group.

The term "halogen" preferably refers to fluorine, chlorine, bromine or iodine. If the term is used for a residue, "halogen" means, for example, a fluorine, chlorine, bromine or iodine atom.

According to the invention, "mercaptoalkyl" refers to an alkyl group having a mercaptan (thiol) substituent.

According to the invention, "sulfidoalkyl" refers to a dialkyl derivative of hydrogen sulfide (H₂S). Such alkyl sulfides have the structure R S R', where at least R or R' is alkyl. They are also known as thioethers.

According to the invention, "alkylsulfonyl" or "arylsulfonyl" means an alkyl group or aryl group containing a sulfonyl group (-SO₂-). This group is also known as the sulfone group. Sulfonyl compounds belong to the organic derivatives of oxygen acids of sulfur, wherein the two organic residues are bonded directly to the sulfur atom.

"Carboxyl" refers to a -COOH radical. Substituted carboxyl refers to -COOR where R is aliphatic, heteroaliphatic, alkyl, heteroalkyl, or a carboxylic acid or ester. The term carboxy ester refers to - COOR where R is alkyl.

The term "hydroxyl" is represented by the formula -OH.

The term "hydroxyalkyl" refers to an alkyl group that has at least one hydrogen atom substituted with a hydroxyl group. The term "alkoxyalkyl group" is defined as an alkyl group that has at least one hydrogen atom substituted with an alkoxy group described above.

The term "aralkyl", "alkyl aryl" of "aryl alkyl" refers to an aryl group having an alkyl group, as defined above, attached to the aryl group, as defined above. An example of an aralkyl group is a benzyl group.

The term "hetero aralkyl", "alkyl heteroaryl" of "heteroaryl alkyl" refers to an heteroaryl group having an alkyl group, as defined above, attached to the aryl group, as defined above.

Optionally substituted groups, such as "optionally substituted alkyl," refers to groups, such as an alkyl group or an aryl alkyl group, that when substituted, have from 1-5 substituents, typically 1, 2 or 3 substituents, preferably selected from hydroxy and alkoxy.

Optionally substituted alkyl groups include, by way of example, haloalkyl groups, such as fluoroalkyl groups, including, without limitation, trifluoromethyl groups. These potential optional substituents apply to any group of the formula disclosed herein where an optional substituent is recited. Preferable optional substituents are hydroxyl, alkyl, alkoxy, carbonyl, alkoxycarbonyl, NO₂, amine. An alkyl substituted with an amino group is referred to as "amino substituted alkyl". An alkyl substituted with an alkoxy group is referred to as "alkoxy substituted alkyl".

The term "oxy alkyl" refers to an alkyl group comprising an oxygen atom. The general structure of an "oxy alkyl" is -R-O-R', where "R" and "R‴ represent alkyl groups.

The term "amino alkyl" refers to an alkyl group that has at least one hydrogen atom substituted with an amino group such as a primary or secondary amine.

The term "aldehyde" is represented by the formula -CHO, consisting of a carbonyl center (a carbon double-bonded to oxygen) with the carbon atom also bonded to hydrogen and to an R group, preferably the backbone of the formula.

The term "carboxyester" is represented by the formula -C(O)-O-R.

The term "carboxamide" is represented by the formula -C(O)-N(R)-R.

The term "primary, secondary or tertiary amine" is represented by the formula -N(R)-R.

The term "carbamate" is represented by the formula -NR-C(O)-O-R.

The term "imide" is represented by the formula -C(O)-N(R)-C(O)-R'.

The term "sulfonyl" is represented by the formula -SO₂R.

The terms "amine sulfoxide", "sulfonamide", "sulfonamide amine" are preferably selected from the groups -N(R)-S(O)ᵤ-R, wherein u is 1 or 2, or -S(O)ᵥ-N(R)-R, wherein v is 1 or 2, preferably from the groups -NHSO₂CH₃, -SO₂NHCH₃, -NHSO₂N(CH₃)₂).

For the definitions above, preferably the terms R, R' are independently selected from the group of H, alkyl, alkylhalo, alkoxy, amine or any of the embodiments described above, and wherein X is halogen. The terms R, R' also comprise the possibility of any given group being appended to R.

The term "nitro" refers to an NO₂ group.

Optionally substituted groups, such as "optionally substituted" refers to groups, such as an alkyl group, that when substituted, have from 1-5 substituents, typically 1, 2 or 3 substituents.

The term "4-, 5- or 6-membered hetero cycloalkyl" refers to a configuration comprising a 4-, 5- or 6-membered heterocyclic ring structure, comprising preferably C and one or more of N, O and/or S. The term "5- to 8-membered cycloalkyl or aryl ring structure" refers to a configuration comprising a 5-, 6-, 7- or 8-membered cyclic optionally aromatic ring structure, optionally comprising one or more heteroatoms such as one or more of N, O and/or S.

The term "bicyclic or tricyclic ring structure" refers to a structure comprising two or three interconnected cyclic structures, respectively. These ring structures may be either fused (sharing two or more atoms) or bridged (connected by a single atom or a chain of atoms).

Where reference is made to "C1 to C7, C1 to C5, C1 to C3" alkyl, cycloalkyl, alkoxy, aryl, or the like, the number of carbon atoms C1 to C7 preferably refers to each of the substituents mentioned, although in some embodiments the shorter substituents of C1 to C5 or C1 to C3 apply to the alkyl, cycloalkyl and/or alkoxy groups, whereby aryl may remain preferably C3 to C6, such as C6 phenyl.

In one embodiment the term "alkylidene" refers to a functional group comprising a carbon-carbon double bond with the general structure - (C=C-R), wherein R is alkyl.

The term residual group of an amino acid refers to the side chain of an amino acid, also termed R residue. The side chain of an amino acid refers to the chemical group attached to the central alpha carbon atom of an amino acid. The R residues of an amino acid are preferably selected from the list set forth below. An optional substitution of the R residues is also possible, e.g. by halogen (preferably fluorine, chlorine, bromine and iodine), OH, CN, SH, amine (preferably NH₂, NHCH₃), NO₂, or-alkyl, with alkyl optionally substituted with one or more substituents. In one embodiment the residual group may be a side chain of a proteinogenic amino acid or a nonproteinogenic amino acid. In one embodiment the residual group may be a side chain of a natural amino acid or an unnatural amino acid.

| Amino acid residue | Abbreviation / code | Side chain of the amino acid |
|---|---|---|
| Alanine | Ala / A | -CH₃ |
| Arginine | Arg / R | -CH₂CH₂CH₂NH-C(NH)NH₂ |
| Asparagine | Asn/N | -CH₂CONH₂ |
| Aspartic acid | Asp / D | -CH₂COOH |
| Cysteine | Cys / C | -CH₂SH |
| Glutamic acid | Glu / E | -CH₂CH₂COOH |
| Glutamine | Gln / Q | -CH₂CH₂CONH₂H |
| Glycine | ciyic | -H |
| Histidine | His/H | -CH₂(C₃H₃N₂) |
| Isoleucine | Ile / I | -CH(CH₃)CH₂CH₃ |
| Leucine | Leu / L | -CH₂CH(CH₃)₂ |
| Lysine | Lys / K | -CH₂CH₂CH₂CH₂NH₂ |
| Methionine | Met / M | -CH₂CH₂SCH₃ |
| Phenylalanine | Phe / F | -CH₂(C₆H₅) |
| Proline | Pro/P | -CH₂CH₂CH₂- |
| Serine | Ser / S | -CH₂OH |
| Threonine | The / T | -CH(OH)CH₃ |
| Tryptophane | Trp / W | -CH₂(C₈H₆N) |
| Tyrosine | Tyr / Y | -CH₂(C₆H₄)OH |
| Valine | Val/V | -CH(CH₃)₂ |

The term "bicyclic or tricyclic ring structure" refers to a group comprising two or three 4- to 8-membered cyclic optionally aromatic ring structures, optionally comprising one or more heteroatoms such as one or more of N, O and/or S. Within the "bicyclic or tricyclic ring structure" the two or three ring structures are attached to each other by two of their adjacent atoms (condensed ring structure), by one atom (spiro ring structure) and/or one or more bridging atoms (bridge ring structures). An example for a bicyclic ring structure is indole. Examples for further bicyclic or tricyclic ring structures can for example be found in WO 2008/040332, WO 2011/003626, WO 2013/030111 , WO2016/092069 and WO2019/162524. The bicyclic or tricyclic ring structure may optionally be substituted such as by halogen (preferably fluorine, chlorine, bromine and iodine), OH, CN, SH, amine (preferably NH₂, NHCH₃), NO₂, or-alkyl, with alkyl optionally substituted with one or more substituents such as the substituents listed in this paragraph.

The term "alpha position" refers to the first carbon atom directly attached to a functional group, such as a carboxyl group, ester group, or amide group. For an alkyl carboxamide, the alpha position refers to the carbon atom directly attached to the carboxamide group. For an alkyl carboxyl ester, the alpha position refers to the carbon atom directly attached to the carboxyl ester group. For an alkyl carboxyl, the alpha position refers to the carbon atom directly attached to the carboxyl group. The term "substituted in the alpha position" refers to a substituent attached to the alpha position of a functional group such as in the alpha position of an alkyl carboxyl, alkyl carboxamide or alkyl carboxyl ester.

Protected derivatives of the disclosed compound also are contemplated, for example for use in the synthesis of the disclosed compounds. A variety of suitable protecting groups for use with the disclosed compounds are disclosed in Greene and Wuts Protective Groups in Organic Synthesis; 3rd Ed.; John Wiley & Sons, New York, 1999. In general, protecting groups are removed under conditions which will not affect the remaining portion of the molecule. These methods are well known in the art and include acid hydrolysis, hydrogenolysis and the like.

Particular examples of the presently disclosed compounds include one or more asymmetric centers (chiral centers); thus these compounds can exist in different stereoisomeric forms such as enantiomers or diastereoisomers such as epimers. Accordingly, compounds and compositions may be provided as individual pure stereoisomeric forms or as stereoisomeric mixtures, including racemic mixtures. The term "ambo" refers to a molecule with two or more chiral elements present as a mixture of diastereoisomers due to the undefined configuration of at least one chiral element of the molecule.

The compounds of the invention may also exist in various polymorphous forms, for example as amorphous and crystalline polymorphous forms. All polymorphous forms of the compounds of the invention belong within the framework of the invention and are a further aspect of the invention.

The compound of the invention may also comprise deuterium replacing hydrogen. This replacement may in some circumstances lead to improved metabolic stability (Nature Reviews Drug Discovery 15, 219-221 (2016)).

It is understood that substituents and substitution patterns of the compounds described herein can be selected by one of ordinary skill in the art to provide compounds that are chemically stable and that can be readily synthesized by techniques known in the art and further by the methods set forth in this disclosure.

### Peptide mimetics and binding domains:

The terms "functional analogs" or "mimetics" are not relative terms because these expressions have a generally recognized meaning in the art of biology. According to the invention, the terms "functional analogs" or "mimetics" can preferably be understood as equivalents. That is, mimetics compared to the natural structure are variants providing essentially the same function in essentially the same way with essentially the same result. The term polyproline mimetic or mimetic of a proline-rich peptide, for example, is an established technical term with a clear functional definition, established for those skilled in the art.

Proline takes a special place among the twenty naturally occurring amino acids, as it is the only secondary amino acid. Due to the cyclization of the a-side chain to the amide nitrogen, the torsion angle is relatively restricted as component of the five-membered ring, which means, consequently, that the peptide has fewer rotational degrees of freedom. Double alkylation of nitrogen produces the result that the otherwise common amide proton is missing (in the peptide backbone), which is why proline is not a candidate for a hydrogen bridge donor; on the other hand, the carbonyl group is particularly rich in electrons, which is why it is a better hydrogen bridge acceptor than is the case with other amino acids. Due to these geometric and electronic properties, proline is unable to stabilize an α-helix ("α-helix breaker") and also does not form any (β-pleated structure (β-pleated sheet breaker"); instead, it is preferably encountered in other typical secondary structures, so-called (p-turns and polyproline helix (PPII helix).

Proline-rich amino acid sequences (also termed polyproline amino acid sequences) are often encountered in proteins that participate in multi-protein complexes and that are formed and/or dissolved in the context of intracellular signal transduction processes. These proteins therein present sequences on the surfaces thereof where proline occurs exclusively or predominantly (often four and more proline units in succession). This induces a characteristic secondary structure, which is a "proline-rich helix", also termed "proline-rich helix structure", "polyproline helix" or "polyproline helix structure", preferably polyproline helix type II helix structure, abbreviated as PPII helix. The PPII helix is understood as a stretched, left-handed helix with torsion angles φ=-78° and ψ =+146° of the peptide backbone. As a consequence, there results a pseudo-C₃ rotational symmetry about the helical axis with exactly three proline moieties per rotation in cross-section, which is why the proline moieties in proline-rich sequences preferably repeat at least with a periodicity of three (for example, PxxPxxP or PPxPPxPPx). This way, the proline side chains and the carbonyl groups of the peptide backbone are exposed to the solvent at regular intervals. Due to the absence of intramolecular hydrogen bridges, the carbonyl groups are especially well suited to form intermolecular hydrogen bridge bonds with receptor proteins. However, the structural motif of the PPII helix can also be induced when not only proline is present. The amino acid Glu occurs particularly frequently in PPII helices, as well as in the proximity thereof; however, Gln, Arg, Ala, Leu, Ser, Asp and H are also found. The preferred binding mode between domain and ligand determines which proline positions must be strictly preserved and which can optionally be replaced with other amino acids.

The EVH1 domain consists of around 115 amino acids and occurs in a large number of signaling multidomain proteins. In addition to a few others, it also includes the family of Ena/VASP proteins, which act as molecular adapters and modulate the actin dynamics of the cytoskeleton. To date, three families of EVH1 domains are known, their classification being based on ligand preference.

The first class specifically recognizes in particular a FPPPP core motif, which is found in focal adhesion proteins such as vinculin, zyxin or RIAM, in lamellipodin, an important protein of the leading edge, in some proteins of the WAVE complex and also in the ActA protein of the intracellular bacterium Listeria monocytogenes. Ena/VASP proteins are also detected in filopodia and invadopodia, where they interact with formins with their EVH1 domain. The first class thus consists of the Ena/VASP proteins, which are significantly involved in the building up and degradation of the actin cytoskeleton. Class II EVH1 domains (Homer/Vesl family) are found in postsynaptic receptor-associated proteins, and the third class of EVH1 domains are the Wiscott-Aldrich syndrome proteins (WASP) involved in actin aggregation in a regulating manner.

The Ena/VASP EVH1 domain (class 1 EVH1) The Ena/VASP proteins (Ena, Mena, VASP, Evl) modulate the actin dynamics in the cytoskeleton and thus play a key role in the area of cell motility. As a result, they affect a number of important physiological processes, such as, e.g., the development of the nervous system or the function of the immune system. As a result, they also play a critical role in disease-related processes such as tumor metastasis, neurological diseases and cardiometabolic diseases such as atherosclerosis and restenosis and thus represent a highly interesting pharmacological "target", e.g. to develop a metastasis inhibitor.

All proteins of the Ena/VASP family have the same basic structure. The EVH1 domain is located at the N terminus of an Ena/VASP protein. Its structure was elucidated by X-ray crystal structure analysis and NMR spectroscopy and shows great similarity with the structures of the Pleckstrin homology (PH) and the phosphotyrosine binding (PTB) domains, although the respective amino acid sequences only slightly match. The EVH1 domain consists of a twisted 3-sandwich structure which is closed on one side by an a-helix. Following the EVH1 domain there is a proline-rich region which serves to bind to proteins containing SH3 and WW domains and to profilin. An EVH2 domain containing a G-actin and an F-actin binding site is located at the C-terminus. It also has a "coiled coil" motif that mediates the tetramerization of the system.

The EVH1 domains of Ena/VASP proteins specifically recognize the proline-rich sequence "FPPPP", which is found, for example, in all focal adhesion proteins (e.g. zyxin and vinculin) or in lamellipodine. These cytoskeleton-associated binding partners of the EVH1 domain serve to localize the Ena/VASP proteins at the required sites of action (e.g. focal adhesions or lamellipodia). A delocalization of the Ena/VASP proteins from these sites by the PRM or PPII helix mimicking peptide ligands thus represents a potential modulation concept.

Another binding partner of the Ena/VASP-EVH1 domain is the ActA surface protein of the intracellular pathogen Listeria monocytogenes, which also contains the "FPPPP" binding motif. As a result, the bacterium is able to localize Ena/VASP proteins on its surface and thus use their function, the formation of actin filaments, for locomotion. However, it has been shown that by inhibiting this PRM-EVH1 interaction, e.g. by removing the FPPPP motif from the ActA protein or injecting FPPPP-containing peptides into the cytoplasm, the motility of the pathogen could be drastically reduced.

Correspondingly, the invention also relates to the use of novel structural mimetics of the polyproline II helix as surprisingly good inhibitors of the protein-protein interaction with particular involvement of the SH3 domains, EVH1 domains, WW domains, GYF domains, UEV domains and profiline. Proteins containing these domains, for example VASP (EVH1 domain) or YAP (WW), play inter alia a particularly important role in the regulation of cell motility (particularly VASP) and cell proliferation (particularly YAP). As such, for example, VASP is markedly overexpressed in highly invasive breast cancer cells. The interaction of YAP with the C-terminal fragment of ERBB4, which is mediated by the WW domains, results, for example, in the nuclear localization of YAP and is the prerequisite for the function of YAP as co-activator of cell proliferation. Numerous novel and surprising possibilities for use of these novel structural mimetics therefore emerge.

The Src homology 3 (SH3) domain, is a small protein domain of approximately 60 amino acids that is found in a variety of proteins involved in intracellular signaling pathways. The SH3 domain is well-known for its role in protein-protein interactions, particularly through binding to proline-rich sequences in other proteins. Fyn is a member of the Src family kinases (SFKs), which are nonreceptor tyrosine kinases. The Fyn SH3 domain specifically mediates interactions with other signaling proteins, contributing to the regulation of pathways involved in cell growth, differentiation, and survival. Proteins comprising SH3 domains, including Fyn, regulate the activity of kinases, phosphatases, and other enzymes by facilitating the formation of multi-protein complexes that are essential for the transmission of signals from the cell membrane to the nucleus. Dysregulation of Src family kinases, including Fyn, is associated with various types of cancer. Overactivity of Fyn can lead to uncontrolled cell growth, survival, and migration, contributing to tumorigenesis and metastasis. Fyn has been implicated in breast cancer, colorectal cancer, and melanoma. Fyn is further involved in signaling pathways that regulate neuronal function such as cell growth, survival, adhesion, cytoskeletal remodeling, motility, axon guidance, synaptic function, and central myelin-forming nervous system, and its dysregulation has been linked to neurological and neurodegenerative diseases such as Alzheimer's disease. Fyn interacts with tau proteins and amyloid precursor proteins, playing a role in the pathogenesis of Alzheimer's disease (see for example Nygaard HB. Targeting Fyn Kinase in Alzheimer's Disease. Biol Psychiatry. 2018 Feb 15;83(4):369-376). Fyn is also critical in the regulation of immune responses. Altered Fyn signaling can lead to immune disorders, including autoimmune diseases and inflammatory conditions. For instance, Fyn is involved in T-cell receptor signaling, and its dysregulation can affect T-cell function and lead to immune dysfunction (Peng, S., Fu, Y. FYN: emerging biological roles and potential therapeutic targets in cancer. J Transl Med 21, 84 (2023))

The WW domain is a small protein module consisting of approximately 40 amino acids that is characterized by two conserved tryptophan (W) residues spaced 20-22 amino acids apart. The WW domain is involved in mediating protein-protein interactions, primarily through binding to proline-rich or phosphoserine/phosphothreonine-containing motifs. WW domains are present in a variety of proteins involved in signaling pathways, cell growth, transcriptional regulation, and ubiquitination. Many WW domain-containing proteins are involved in the ubiquitin-proteasome system, where they help regulate the degradation of target proteins by facilitating their ubiquitination. This function is essential for maintaining cellular homeostasis and controlling protein levels. Dysregulation of WW domain-containing proteins is implicated in several types of cancer. For example, the protein YAP (Yes-associated protein), which contains a WW domain, is a key regulator of the Hippo signaling pathway. Overactivation of YAP leads to uncontrolled cell proliferation and is associated with various cancers, including liver, breast, and lung cancers (Franklin, J.M., Wu, Z. & Guan, KL. Insights into recent findings and clinical application of YAP and TAZ in cancer. Nat Rev Cancer 23, 512-525 (2023)). Some WW domain-containing proteins are further involved in neurodegenerative diseases. For example, the WW domain protein NEDD4-1 (Neural precursor cell-expressed developmentally down-regulated protein 4-1) is an E3 ubiquitin ligase that regulates the turnover of proteins implicated in neurodegeneration, such as amyloid precursor protein (APP) in Alzheimer's disease (Huang, X., Chen, J., Cao, W. et al. The many substrates and functions of NEDD4-1. Cell Death Dis 10, 904 (2019)).

The GYF (Glycine-Tyrosine-Phenylalanine) domain is a small protein module of about 60-75 amino acids, known for mediating protein-protein interactions. It was named after the conserved glycine-tyrosine-phenylalanine sequence motif within the domain. GYF domains are often found in proteins that are involved in signaling pathways and transcriptional regulation. These domains enable the assembly of multi-protein complexes that transmit signals from the cell membrane to the nucleus, thus influencing gene expression and other cellular outcomes. The dysregulation of GYF domain-containing proteins is associated with cancer. For example, proteins with GYF domains involved in signal transduction may be overactive or improperly regulated in cancer cells, leading to unchecked cell proliferation, survival, and metastasis. Aberrant protein-protein interactions mediated by GYF domains also facilitate oncogenic signaling pathways. GYF domain-containing proteins are further associated with neurological functions, including the regulation of synaptic activity and neuronal signaling. Mutations or dysregulation of these proteins contributes to neurological disorders, including neurodevelopmental disorders or neurodegenerative diseases. GYF domain-containing proteins also play a roles in immune system regulation. Dysregulation of these proteins contribute to immune-related disorders.

The UEV (Ubiquitin E2 Variant) domain is a protein module found in a subset of proteins that are involved in the ubiquitin-proteasome system, which is a key pathway for regulating protein degradation and various cellular processes. Unlike typical E2 ubiquitin-conjugating enzymes, proteins with a UEV domain do not possess catalytic activity but instead play regulatory roles, often facilitating interactions between ubiquitin-conjugating enzymes and other components of the ubiquitination machinery. The UEV domain is structurally similar to E2 ubiquitin-conjugating enzymes but lacks the catalytic cysteine residue required for transferring ubiquitin to substrate proteins. The UEV domain typically interacts with other proteins involved in the ubiquitination process, such as ubiquitin ligases (E3s) and ubiquitin-binding proteins, to regulate the ubiquitination of specific targets. Proteins with UEV domains are involved in various steps of the ubiquitination process, including substrate recognition, assembly of multi-protein complexes, and modulation of ubiquitin chain assembly. These functions are critical for controlling the stability, localization, and activity of many cellular proteins. Dysregulation of the ubiquitin-proteasome system is associated with cancer and contributes to tumorgenesis. For example, TSG101, a protein containing a UEV domain, is involved in regulating the degradation of proteins that control cell proliferation and survival. Alterations in TSG101 function have been associated with various cancers, including breast and prostate cancer (Ferraiuolo RM, Manthey KC, Stanton MJ, Triplett AA, Wagner KU. The Multifaceted Roles of the Tumor Susceptibility Gene 101 (TSG101) in Normal Development and Disease. Cancers (Basel). 2020 Feb 14;12(2):450)). The ubiquitin-proteasome system, in which UEV domain-containing proteins play a key role, is further crucial for maintaining protein homeostasis in neurons. Dysregulation of this system can lead to the accumulation of misfolded or damaged proteins, contributing to neurodegenerative diseases such as Alzheimer's disease, Parkinson's disease, and Huntington's disease. UEV domain-containing proteins further influence the aggregation or clearance of neurotoxic proteins in these conditions. UEV domain-containing proteins also participate in the regulation of immune responses. For instance, UEV domain-containing proteins influence the stability and activity of signaling proteins involved in inflammation and immune cell activation. Dysregulation in these processes contributes to autoimmune diseases or chronic inflammatory conditions.

Profilin is a small, highly conserved actin-binding protein that plays a crucial role in the regulation of the actin cytoskeleton, a dynamic structure involved in various cellular processes, including cell shape, motility, and division. Profilin is involved in a wide range of cellular functions by modulating actin polymerization and interacting with other proteins. Profilin binds to monomeric (G-actin) and promotes the exchange of ADP for ATP, which is a necessary step for actin polymerization. However, profilin also prevents the spontaneous nucleation of actin monomers, thereby tightly regulating actin filament formation. Profilin's role in regulating the actin cytoskeleton is associated with cancer progression, particularly in processes such as cell migration, invasion, and metastasis. Overexpression or dysregulation of profilin has been observed in several types of cancers, including breast, pancreatic, and prostate cancer. Profilin is also implicated in neurodegenerative diseases such as amyotrophic lateral sclerosis (ALS). Mutations in the profilin 1 gene (PFN1) have been linked to familial forms of ALS, suggesting that defects in actin dynamics contribute to neuronal degeneration (Wu et al., Mutations in the profilin 1 gene cause familial amyotrophic lateral sclerosis. Nature. 2012 and Schmidt et al., ALS-linked PFN1 variants exhibit loss and gain of functions in the context of formin-induced actin polymerization, PNAS, 118 (23) e2024605118, 2021). Given profilin's role in cytoskeletal dynamics, it may also be involved in cardiovascular diseases, particularly those related to abnormal smooth muscle cell proliferation and migration, such as atherosclerosis (Allen A, Gau D, Roy P. The role of profilin-1 in cardiovascular diseases. J Cell Sci. 2021).

Actin is a structural protein found in all eukaryotic cells. It is part of the cytoskeleton and one of the five most common proteins in eukaryotes. Actin forms dynamic actin filaments as F-actin.

These microfilaments serve the stabilization of the outer cell shape, the formation of cellular extensions, intracellular displacements and directed cellular movements. Many eukaryotic cells have a high degree of mobility, cell motility or, as also referred to, cell migration, for example to render invaders harmless in the Body (cells of the immune system), to be able to heal wounds (e.g. skin cells) or to move cells in general (in development or unicellular organisms such as amoeba). Cell migration also plays an essential role in cancer metastases. This mobility is mainly based on two processes: the directed actin polymerization in the direction of movement (regulated by a number of regulators that react to signals from the cell periphery), and the actinmyosin interaction in fibril bundles (stress fibers), contractile traction ropes that run through the cell and brace form-giving elements with the base.

The disruption of the actin filament synthesis caused by the compounds according to the invention also represents a new and advantageous technical effect. By disrupting actin filament synthesis, the compounds according to the invention can inhibit the motility of cancer cells and thus strongly inhibit or prevent cancer metastasis.

### Medical conditions:

"Cancer", as used herein, is a disease characterized by the uncontrolled growth of abnormal cells. Cancer refers to any type of cancerous growth or carcinogenic process, metastatic tissue or malignant transformed cells, tissues or organs, regardless of histopathological type or invasive stage. Cancer cells can spread locally or to other parts of the body via the bloodstream and lymphatic system. Cancer cells spreading to other parts to the body are termed "metastatic cells" or "metastatic tumor cells". The terms "tumor" and "cancer", used herein, are utilized interchangeably, e.g., both terms include solid and liquid, e.g. general or circulating tumors, premalignant and malignant cancers and tumors.

Examples of liquid cancers include, but not limited to, acute lymphocytic leukemia (ALL), chronic lymphocytic leukemia (CLL), acute myeloid Leukemia (acute myelogenous leukemia (AML), chronic bone marrow cancer, chronic myelogenous leukemia (CML), Hodgkin lymphoma, non-Hodgkin lymphoma, and myeloma.

Examples of solid tumors are liver, lung, breast, lymphatic system, digestive organs (e.g. colon), urogenital organs (e.g. kidney, urothelial cells), prostate and throat, malignant organ systems including tumors such as sarcomas, adenocarcinomas and cancer. Examples for breast cancer that can be treated are ductal carcinoma in situ (DCIS), lobular carcinoma in situ (LCIS), invasive ductal carcinoma (IDC), invasive ductal carcinoma including tubular, medullary, mucinous, papillary, and cribriform carcinomas, invasive lobular carcinoma (ILC), inflammatory breast cancer, male breast cancer, Paget's Disease of the nipple, phyllodes tumors of the breast, recurrent and/or metastatic breast cancer. Adenocarcinoma includes most malignant tumors such as colon cancer, rectal cancer, renal cell cancer, liver cancer, non-small cell lung cancer, small intestine cancer and esophageal cancer. In certain forms the cancer is a melanoma, e.g. an advanced stage melanoma. Metastatic lesions of the cancer can also be treated or prevented with the methods and compositions of the invention.

Examples of other types of cancer that can be treated are bone cancer, pancreatic cancer, skin cancer, head and neck cancer, skin or intraocular melanoma, uterine cancer, ovarian cancer, rectal cancer, anal cancer, stomach cancer, testicular cancer, faropius duct cancer, Endometrial cancer, cervical cancer, vaginal cancer, vulvar cancer, Hodgkin's disease, non-Hodgkin's lymphoma, esophageal cancer, small intestine cancer, endocrine cancer, thyroid cancer, parathyroid cancer, adrenal cancer, soft tissue sarcoma, urethral cancer, penile cancer, acute myeloid leukemia, chronic myelocytic leukemia, acute lymphoblastic leukemia, chronic or acute leukemia including chronic lymphatic leukemia, solid tumor in childhood, lymphatic lymphoma, bladder cancer, kidney or ureter cancer, renal pelvis cancer, neoplasm of the central nervous system (CNS) primary CNS lymphoma, tumor angiogenesis, spinal axis tumor, brainstem glioma, pituitary adenoma, Kaposi's sarcoma, epidermoid carcinoma, squamous cell carcinoma, asbestos-induced T cell lymphoma Including a combination of environmental cancer and cancer including things Treatment of metastatic cancer, e.g. metastatic cancer that expresses PD-L1 (Iwai et al. (2005) Int. Immunol. 17: 133-144) can be performed with the inhibitory molecules described in this invention.

In the context of the present invention the term solid tumor refers without limitation to glioblastoma, lung carcinoma, breast carcinoma, kidney carcinoma, pancreatic carcinoma, skin carcinoma such as melanoma, intestinal carcinoma, ovarian carcinoma, prostate carcinoma, colon carcinoma, and sarcoma.

Sarcomas as defined in the context of the present invention include, but are not limited to a chondrosarcoma, fibrosarcoma, lymphosarcoma, melanosarcoma, myxosarcoma, osteosarcoma, Abernethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

Melanomas according to the present invention include, but are not limited to include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

In the context of the present invention the term solid tumor further refers without limitation to acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma exulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticurn, carcinoma telangiectodes, transitional cell carcinoma, carcinoma tuberosum, tuberous carcinoma, verrucous carcinoma, and carcinoma villosum.

The bacterial diseases are preferably such diseases that are associated with the following bacteria, particularly those that are mediated by the same: legionella, streptococci, staphylococci, klebsiella, Haemophilis influenzae, rickettsia (spotted fever), mycobacteria, mycoplasmas, ureaplasmas, neisseria (meningitis, Waterhouse-Friedrichsen syndrome, gonorrhea), pseudomonads, bordetella (pertussis), corynebacteria (diphtheria), chlamydia, campylobacter (diarrhea), Escherichia coli, proteus, salmonella, shigella, yersinia, vibriona, enterococci, clostridien, borrelia, Treponema pallidum, brucella, francisella and/or leptospira, particularly listeria. Particularly preferred diseases are those that are caused by listeria, selected from the group comprising L. monocytogenes Sv1/2a, L. monocytogenes Sv4b F2365, L. monocytogenes Sv4b H7858, 178 contigs, L. monocytogenes Sv1/2a F6854, 133 contigs, L. monocytogenes Sv4b, L. monocytogenes Sv4a, L. innocua Sv6a, L. welshimeri Sv6b, L. seeligeri Sv1/2b and/or L. ivanovii Sv5, or that essentially instrumentally related to the afore-named preferred listerias.

"Neurodegenerative diseases" also termed "neurological disease" are caused by the progressive loss of structure or function of neurons, in the process known as neurodegeneration. Such neuronal damage may ultimately involve cell death. Neurodegenerative diseases include for example amyotrophic lateral sclerosis (ALS), multiple sclerosis (MS), Parkinson's disease (PD), Alzheimer's disease (AD), Huntington's disease, multiple system atrophy, and prion diseases. The preferred neurodegenerative diseases are selected from the group comprising Alzheimer' s disease, Parkinson' s disease, Huntington's disease and/or amyotrophic lateral sclerosis (ALS). Neurodegenerative disorders in the context of the invention are also Alexander' s disease, Alpers-Huttenlocher syndrome, Huntingdon's disease, Creutzfeldt-Jakob disease, extrapyramidal syndrome, Friedreich's ataxia, corticobasal degeneration, Crabbe disease, leukodystrophy, Lewy body dementia, mask-like face, Pick' s disease, multiple sclerosis, multisystem atrophy, neurodegenerative iron deposits in the brain, progressive supranuclear palsy, Shy-Drager syndrome, spinocerebellar ataxia, synucleinopathy and/or tropic spastic paraparesis.

Viral diseases in the context of the invention are selected from the group comprising influenza, cold, cough, measles, mumps, rubella, fifth disease, three-day fever, chickenpox, Preiffer's glandular fever, SARS, zytomegalo virus, diarrhea, hepatitis, polio, labial herpes, warts, rabies, assa fever, ebola, Marburg fever, hantavirus fever, ESME, RSSE, Louping-ill encephalitis, Powassan encephalitis, Kyasanur forest fever, Omsk hemorrhagic fever, Colorado tick fever, yellow fever, Dengue fever, Japanese encephalitis, West Nile fever, Chikungunya fever, O'nyong-nyong fever, Rift Valley fever, sandfly fever, Ross River fever, Sindbis fever, Mayaro fever, Murray valley encephalitis, St. Louis encephalitis, Rocio encephalitis, California encephalitis, Bunyamwera fever, Oropouche fever, AIDS, genital herpes and/or Herpes Simplex; more preferably, the viral hepatitis diseases are selected from the group comprising hepatitis A, hepatitis B, hepatitis C, hepatitis D, hepatitis E, hepatitis F, hepatitis G and/or autoimmune hepatitis and/or asymptomatic or symptomatic HIV infections. HIV infections can lead to AIDS. In the context of the invention, AIDS is characterized or classified by a clinical picture selected from the group comprising (a) asymptomatic or symptomatic HIV infections, (b) bacillary angiomatoses, inflammations of the pelvis minor, particularly in the case of complications in a tube abscess or ovarian abscess, extended or recurrent Herpes zoster, thrombocytopenic purpura, long-lasting fever or diarrhea lasting for longer than one month, listeriosis, oral hairy leukoplakia, oropharyngeal candidiasis, chronic or difficult-to-treat vaginal candidiasis, cervical displasias, carcinoma in situ, peripheral neuropathy and/or (c) candidiasis of the respiratory pathways or of the food tubes, cytomegalievirus infections, CMV retinitis, HIV-related encephalopathy, herpes simplex with chronic ulcers (>1 month) or herpes simplex-related bronchitis, pneumonia or oesophagitis, chronic histoplasmosis, intestinal isosporiasis, Kaposi's sarkoma, disseminated or extrapulmonary coccidiomycosis, extrapulmonary cryptokoccosis, chronic intestinal kryptosporidiosis, immunoblastic, primary cerebral or Burkitt' s lymphoma, extrapulmonary mycobacteria, pneumocystis pneumonia, recurrent bacterial pneumonia, progressive multifocal leucoencephalopathy, recurrent Salmonella septicemia, tuberculosis, cerebral toxoplasmosis, wasting syndrome and/or invasive cervical carcinoma. Treatment in the context of the invention means prophylaxis, therapy, monitoring of the course and/or aftertreatment of diseases.

The term "cardiometabolic disease" refers to a spectrum of interrelated conditions that affect the cardiovascular system and metabolic processes, including those related to blood sugar regulation, lipid metabolism, and insulin sensitivity. These diseases are typically characterized by a combination of risk factors that increase the likelihood of developing cardiovascular disease (CVD), such as coronary artery disease, stroke, and heart failure, as well as metabolic disorders like obesity, type 2 diabetes, and metabolic syndrome. Cardiometabolic diseases are often driven by lifestyle factors such as poor diet, physical inactivity, and obesity, as well as genetic predispositions. Cardiometabolic diseases according to the present invention include without limitation coronary artery disease (CAD), hypertension (high blood pressure), Type 2 Diabetes Mellitus, obesity, metabolic syndrome, dyslipidemia (abnormal blood lipid levels), atherosclerosis, peripheral artery disease (PAD), heart failure, myocardial infarction, stroke, Non-Alcoholic Fatty Liver Disease (NAFLD), chronic kidney disease (CKD), hyperinsulinemia, insulin resistance, hyperglycemia, hypertriglyceridemia, hyperlipidemia, dyslipidemia, nephropathy, and cardiomyopathy.

### Compositions and modes of treatment:

The present invention relates further to pharmaceutically acceptable salts of the compounds described herein. The term "pharmaceutically acceptable salt" refers to salts or esters of the compounds described herein prepared by conventional means that include basic salts of inorganic and organic acids. "Pharmaceutically acceptable salts" are also inclusive of the free acid, base, and zwitterionic forms. Descriptions of suitable pharmaceutically acceptable salts can be found in Handbook of Pharmaceutical Salts, Properties, Selection and Use, Wiley VCH (2002). For therapeutic use, salts of the compounds are those wherein the counter-ion is pharmaceutically acceptable. However, salts of acids and bases which are non-pharmaceutically acceptable may also find use, for example, in the preparation or purification of a pharmaceutically acceptable compound.

Another aspect of the disclosure includes pharmaceutical compositions prepared for administration to a subject and which include a therapeutically effective amount of one or more of the compounds disclosed herein. In certain embodiments, the pharmaceutical compositions are useful for treating medical conditions associated with associated with a modified intracellular signal transduction process mediated by a proline-rich helix structure such as an infectious disease, immune disease, neurological disease, cardiometabolic disease and a cancerous disease.

The therapeutically effective amount of a disclosed compound will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anaesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

This preparation is preferably applied orally, subcutaneously, intravenously, intramuscularly, intraperitoneally and/or topically. The pharmaceutical agent comprising the compounds according to the invention is preferably used in total amounts from 0.05 to 500 mg per kg, preferably from 5 to 100 mg per kg of body weight, per 24 hours, such as 5, 10, 15, 20, 25, 30, 35, 40, 45, 50, 55, 60, 65, 70, 75, 80, 85, 90, 95 or 100 mg per kg of body weight, per 24 h.

The compositions of the disclosure can alternatively contain as pharmaceutically acceptable carrier substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

In one embodiment the invention comprise the topical and/or local administration of a compound as described herein and/or a composition comprising a compound as described here-in to a subject. The term "topical administration" refers to the delivery of a pharmacologically active agent to the skin or mucosa of a patient. Topical administration can provide a local rather than a systemic effect. The terms "topical administration" and "transdermal administration" are used interchangeably to mean administration of a pharmacologically active agent to the skin or mucosa of a patient to achieve a therapeutic effect in treating or preventing a medical disorder of the invention or discomfort at the site of topical or transdermal administration. Preferred administration modes relate to a topical solution, lotion, shake lotion, cream, ointment, gel, foam, trans-dermal patch, powder, solid form, sponge, tape, paste or tincture. Preferred embodiments relate to creams, foams, gels, lotions, and ointments.

Various additives, known to those skilled in the art, may be included in topical compositions of the present disclosure. For example, solvents, including relatively small amounts of alcohol, may be used to solubilize a compound of the invention. Other optional additives include antioxidants, fragrances, colorant, gelling agents, emulsifiers, thickening agents, stabilizers, surfactants, buffers, cooling agents (e.g., menthol) and the like. Other agents may also be added, such as antimicrobial agents, to prevent spoilage upon storage, i.e., to inhibit growth of microbes such as yeasts and molds. Examples of suitable antimicrobial agents include methyl and propyl esters of p-hydroxybenzoic acid (i.e., methyl and propyl paraben), sodium benzoate, sorbic acid, imidurea, and the like. When applied to skin, a topical composition of the present disclosure can be covered with an occlusive or non-occlusive dressing, which may be porous or non-porous, so as to protect the composition from mechanical removal during the period of treatment, e.g. a plastic film food wrap or other non-absorbent film. Various inert coverings may be employed. Non-woven or woven coverings may be employed, particularly elastomeric coverings, which allow for heat and vapor transport. These coverings can allow for cooling of the diseased site, which can provide for greater comfort, while protecting the composition from mechanical removal.

In accordance with the various treatment methods of the disclosure, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

"Administration of" and "administering a" compound should be understood to mean providing a compound, a prodrug of a compound, or a pharmaceutical composition as described herein. The compound or composition can be administered by another person to the subject (e.g., intravenously, gel, cream, spray) or it can be self-administered by the subject (e.g., tablets, gel, cream, spray).

Dosage can be varied by the attending clinician to maintain a desired concentration at a target site (for example, the lungs or systemic circulation). Higher or lower concentrations can be selected based on the mode of delivery, for example, trans-epidermal, rectal, oral, pulmonary, or intranasal delivery versus intravenous or subcutaneous delivery. Dosage can also be adjusted based on the release rate of the administered formulation, for example, of an intrapulmonary spray versus powder, sustained release oral versus injected particulate or transdermal delivery formulations, and so forth.

The term "subject" includes both human and veterinary subjects. The term "treatment" refers to a therapeutic intervention that ameliorates a sign or symptom of a disease or pathological condition after it has begun to develop.

As used herein, the term "ameliorating", with reference to a disease or pathological condition, refers to any given beneficial effect of the treatment. The beneficial effect can be evidenced, for example, by a delayed onset of clinical symptoms of the disease in a subject, a reduction in severity of some or all clinical symptoms of the disease, a slower progression of the disease, an improvement in the overall health or well-being of the subject, or by other parameters well known in the art that are specific to the particular disease.

The present invention encompasses both therapeutic treatment and prophylactic treatment of a subject. A "prophylactic" treatment is a treatment administered to a subject, who does not exhibit signs of the medical condition or who preferably exhibits indications of developing or developing further any given medical condition, for the purpose of decreasing the risk of developing pathology or clinical symptoms. A prophylactic administration may comprise the administration of the compounds in advance of developing symptoms, thereby avoiding or reducing the subsequent occurrence of a disease. The present invention also relates to a method of treatment of subjects suffering from the various medical conditions disclosed herein. The method of treatment com-prises preferably the administration of a therapeutically effective amount of a compound dis-closed herein to a subject in need thereof.

A "therapeutically effective amount" refers to a quantity of a specified agent sufficient to achieve a desired effect in a subject being treated with that agent. For example, this may be the amount of a compound disclosed herein useful in alleviating the symptoms of one or more of the medical conditions described herein in a subject. The therapeutically effective amount or diagnostically effective amount of an agent will be dependent on the subject being treated, the severity of illness, and the manner of administration of the therapeutic composition. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects. A non-limiting range for a therapeutically effective amount of a compound and/or other biologically active agent within the methods and formulations of the disclosure is about 0.001 mg/kg body weight to 50 mg/kg body weight, 0.01 mg/kg body weight to about 20 mg/kg body weight, such as about 0.05 mg kg to about 5 mg/kg body weight, or about 0.2 mg/kg to about 2 mg/kg body weight.

The instant disclosure also includes kits, packages and multi-container units containing the herein described pharmaceutical compositions, active ingredients, and/or means for administering the same for use in the prevention and treatment of diseases and other conditions in mammalian subjects.

### FIGURES

The invention is demonstrated by way of the example through the figures disclosed herein. The figures provided represent particular, non-limiting embodiments and are not intended to limit the scope of the invention.

### Brief description of the figures

**Fig.1:** ¹H-Spectrum of Boc[ProM-40]OtBu (Example compound 1)
**Fig. 2****:** ¹³C-Spectrum of Boc[ProM-40]OtBu (Example compound 1).
**Fig. 3****:** ¹H-Spectrum of Boc[ProM-24]OtBu (Example compound 7).
**Fig. 4****:** ¹³C-Spectrum of Boc[ProM-24]OtBu (Example compound 7).
**Fig. 5****:** ¹H-Spectrum of Boc[dH-ProM-24]OtBu (Example compound 8).
**Fig. 6****:** ¹³C-Spectrum of Boc[dH-ProM-24]OtBu (Example compound 8).
**Fig. 7****:** ¹H-Spectrum of Boc[ProM-40']OtBu (Example compound 9).
**Fig. 8****:** ¹³C-Spectrum of Boc[ProM-40']OtBu (Example compound 9).
**Fig. 9****:** ¹H-Spectrum of Boc[dH-ProM-40]OtBu [PSDA390b] (Example compound 10).
**Fig. 10****:** ¹³C-Spectrum of Boc[dH-ProM-40]OtBu [PSDA390b] (Example compound 10).
**Fig. 11****:** ¹H-Spectrum of Boc[epi -ProM-40']OtBu (Example compound 11).
**Fig. 12****:** ¹³C-Spectrum of Boc[epi -ProM-40']OtBu (Example compound 11).
**Fig. 13****:** ¹H-Spectrum of Boc[epi -dH-ProM-40]OtBu (Example compound 12).
**Fig. 14****:** ¹³C-Spectrum of Boc[epi -dH-ProM-40]OtBu (Example compound 12).
**Fig. 15****:** ¹H-Spectrum of Ac[2Cl-F][ProM-40][ProM-1]OMe (Example compound 6).
**Fig. 16****:** HPLC chromatogram of Ac[2Cl-F][ProM-40][ProM-1]OMe (Example compound 6).
**Fig. 17****:** ¹H-Spectrum of Ac[2Cl-F][dH-ProM-40][dH-ProM-28]NH (Example compound 13).
**Fig. 18****:** HPLC chromatogram of Ac[2Cl-F][dH-ProM-40][dH-ProM-28]NH (Example compound 13).
**Fig. 19****:** ¹H-Spectrum of Ac[2Cl-F][dH-epi -ProM-40][dH-ProM-28]NH (Example compound 14).
**Fig. 20****:** HPLC chromatogram of Ac[2CI-F][dH-epi -ProM-40][dH-ProM-28]NH (Example compound 14).
**Fig. 21****:** X-ray crystal structure of Boc[ProM-24]OtBu (Example compound 7).

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

### Methods

### Nuclear magnetic resonance (NMR) spectroscopy

The NMR experiments were recorded on an Avance III (500 MHz) from Bruker. All experiments were performed at 298 K. The ¹H spectra obtained were referenced to the internal standard tetramethylsilane (TMS; 0.00 ppm) contained in the solvent. The number of protons was determined by integrating the signals. The multiplicities of the obtained signals were labeled with "s" for singlet, "d" for doublet, "t" for triplet, "q" for quartet and "m" for multiplet. The ¹³C spectra were referenced to the non-deuterated portion of the solvent (77.03 ppm for CDCl₃). The signals were assigned using the measured HMQC, HMBC and H,H-COSY spectra and the underlying literature.

### Gas chromatography with coupled mass spectrometry (GC-MS)

Gas chromatography experiments were performed on an Agilent 6890 GC system in conjunction with a 7683 autosampler/injector system and a 5973 network mass detector. Ionization was performed by electron impact ionization (EI) at a potential of 70 eV. An Optima-5 Accent (30 m ▪ 0.25 mm ▪ 0.25 µm) from Macherey-Nagel was used as the capillary column. Hydrogen with a volume flow of 1.7 mL/min was used as the carrier gas. One temperature program was used: 50300M: 50 °C for 2 min; 25 °C/min to 300 °C; 300 °C for 5 min; 320 °C for 2 min.

### High-resolution mass spectrometry (HR-MS)

The high-resolution mass spectra were recorded using an LTQ Orbitrap XL-FTMS analyzer from Thermo Fischer. The analyzed ions were generated by the electrospray method.

### Biochemical competition binding

Binding studies were conducted using biolayer-interferometry (BLI) on an OCTET^{®} R2 (Sartorius) where the binding of recombinantly produced EnaH-EVH1 protein to a sensorimmobilized Biotin-Ahx-SEPSSFE(2Cl-F)PPPPTEDEL-NH₂ peptide (SEQ ID No. 1) - representing a modification of a natural EVH1 binding sequence from ActA protein of L. monocytogenes - was measured. Test compounds were dissolved in DMSO at 40 mM concentration and diluted in assay buffer (PBS, pH 7.4 with 5 mM TCEP and 0.5 % DMSO) with compound and added with a concentration range from 180µM-3nM. The compounds competed with the peptide in binding the EnaH-EVH1 protein which induced a concentration-dependent signal decrease at the sensor. From there inhibitory IC50 values were calculated.

### Parallel Artificial Membrane Permeability Assay (PAMPA)

PAMPA studies were conducted using the PAMPA Explorer kit (pION Inc.) and the double sink protocol (Avdeef et al., 2005). Stock solutions of all compounds were prepared in dimethyl sulfoxide (DMSO) to a concentration of 10 mM. Each stock solution was diluted to 50 µM in pH 7.4 Prisma HT buffer (pION) and 200 µL were added to each well of the donor plate in triplicate. The polyvinylidene fluoride (PVDF, 0.45 µm) filter membrane on the acceptor plate was coated with 5 µL of the gastrointestinal tract lipid formulation (GIT-0, pION) and to each well of the acceptor plate, 200 µL of acceptor sink buffer (ASB, pION) was added. The acceptor filter plate was carefully placed on top of the donor plate to form a "sandwich." The sandwich was incubated at 25°C for 4 h, without stirring. UV-vis spectra of the solutions in the blank, reference, acceptor, and donor plates were measured using a microplate reader (Tecan Infinite 200 PRO M Nano Plus). For each compound, apparent permeability values Papp (10-6cm/s) were calculated using the PAMPA Explorer software v. 3.6 (pION).

### Caco-2 Permeability Assay

Test compounds were dissolved in DMSO to a concentration of 10 mM and further diluted to a concentration of 10 µM in assay buffer (at 1% DMSO). Before the assay was started, TEER measurements of a representative number of wells was performed (≥500 Ω.cm2) to ensure the full polarization of the Caco2-cells (21 days of cultivation in total). The cells were washed three times with assay buffer and pre-incubated for 30 minutes at 37°C and 5% CO2. A>B and B>A incubations were performed for 2 hours at 37°C and 5% CO₂. Apical side of A>B transport and basolateral side of B>A were sampled at t=0 and quenched with 100 µl acetonitrile containing 200 nM labetalol (IS). After 2 hours incubation, 25 µl samples from the apical and basolateral side were quenched with 100 µl acetonitrile containing 200 nM labetalol (IS). The samples were centrifuged at 3700 rpm and 100 µl supernatant was analyzed using LC-MS/MS. After the samples were taken, the membrane integrity was determined using lucifer yellow. The paracellular flux should be ≤0.7% after 1 hour. All sample analysis was performed using a Waters Premier UPLC-MS/MS system.

### Microsomal Stability Study (MLM)

Test compounds were dissolved in DMSO to a concentration of 10 mM and further diluted to 100 µM using acetonitrile. Liver microsomes from selected species were incubated in duplicate with the test compound at a final concentration of 1 µM in 0.1 M potassium phosphate buffer (pH 7.4) containing 3.3 mM MgCl₂, 0.5 mg/ml microsomal protein, in the presence or absence of NADPH (1 mM). Incubations were performed at 37°C in a total volume of 500 µl. Control incubations with reference compounds were included for each experiment. At different time points (0, 5, 15, 30 and 45 min), 50 µl of the incubation mixture was transferred into a quench plate containing acetonitrile and internal standard (200 nM labetalol) cooled to 6°C. After the last time point, the quench plates were mixed and centrifuged for 15 minutes at 3700 rpm and 6°C. The supernatant was transferred to new 96-well plates and subjected to LC-MS analysis. All sample analysis was performed using a Vanquish Horizon UHPLC-system equipped with an autosampler, a binary pump, a column compartment and a diode array detector coupled to a Q Exactive focus hybrid quadrupole-Orbitrap mass spectrometer (Thermo Fisher Scientific) equipped with heated electrospray ion source. MS settings were optimized for the flow rate.

### Hepatocyte Stability Study (HepM [mouse] + HepH [human])

Test compounds are dissolved in DMSO to a concentration of 10 mM and further diluted to 100 µM using DMSO:MQ (1:1). Cryopreserved hepatocytes from selected species are thawed and incubated in duplicate with the test compound at a final concentration of 1 µM in Krebs-Henseleit buffer. Incubations are performed at 37°C, 5% CO₂ and 220 rpm in a total volume of 350 µl, with 0.5×10⁶ cells/ml. Control incubations with reference compounds are included for each experiment. At different time points (0, 10, 20, 40 and 60 min), 50 µl of the incubation mixture is transferred into a quench plate containing acetonitrile and internal standard (200 nM labetalol) cooled to 4°C. After the last time point, the quench plates are mixed and centrifuged for 15 minutes at 3700 rpm and 10°C. The supernatant is transferred to new 96-well plates and subjected to LC-MS analysis. All sample analysis is performed using a Vanquish Horizon UHPLC-system equipped with an autosampler, a binary pump, a column compartment and a diode array detector coupled to a Q Exactive focus hybrid quadrupole-Orbitrap mass spectrometer (Thermo Fisher Scientific) equipped with heated electrospray ion source. MS settings were optimized for the flow rate.

### Kinetic Solubility

Test compounds were dissolved in DMSO to a concentration of 10 mM and further diluted to 100 µM in buffer (10 mM PBS, pH 7.4) in a 96 well plate. Final DMSO concentration is 1%. Incubations were performed in triplicate. The plates were shaken for 4 h at room temperature in an Eppendorf Thermomixer. After 4 h incubation, the plates were centrifuged for 20 minutes at 3700 rpm. From the supernatant 150 µl was transferred to a new 96 well plate and 50 µl DMSO was added to ensure continued dissolution. Samples were measured on LC-UV at injection volumes of 1 and 4 µl. Peak area are determined and compared to peak area obtained using calibration curves of the test compounds in DMSO.

### Plasma Protein Binding (PPB) [mouse, human, FBS]

Test compounds were dissolved in DMSO to a concentration of 10 mM and further diluted to 100 µM using DMSO. Plasma of selected species or cell culture medium supplemented with 10% FBS was thawed and spiked with 100 µM sample to a concentration of 1 µM. The spiked plasma was incubated in triplicate for 4 hours at 37°C under continuous shaking using phosphate buffered saline (PBS) buffer as a receiver solution in a rapid equilibrium device. The recovery was determined from the pooled spiked plasma. A 1 µM standard solution was prepared at t=4h by diluting the stock solution in plasma for checking the recovery sample stability during the incubation. After the incubation all samples were matrix matched and quenched with 1% formic acid in acetonitrile including an internal standard (200 nM labetalol). The samples were mixed and centrifuged for 15 minutes at 3700 rpm and 6°C. The supernatant was transferred to new 96-well plates and subjected to LC-MS analysis. The 1 µM standard was used to check the stability of the recovery sample.

### In vitro evaluation of inventive compounds

### Biochemical competition binding

BLI was used to determine the IC50 of the displacement of a modified peptide derived from ActA from L. monocytogenes, the strongest natural occurring binding partner of EnaH-EVH1 with a K_{d} of approx. 20 µM.

### Parallel Artificial Membrane Permeability Assay (PAMPA)

The Parallel Artificial Membrane Permeability Assay (PAMPA) provides a high throughput, noncell based method for predicting passive, transcellular intestinal absorption, the process by which the majority of small molecule drugs enter circulation. The tested compounds can be categorized into low, moderate, and high permeability. Generally, compounds which have a Pe < 1.5 × 10⁻⁶ cm/sec are classified as low permeable, compounds with a Pe ≥1.5 × 10⁻⁶ cm/sec - < 10 × 10⁻⁶ cm/sec as moderate permeable and compounds with a Pe ≥ 10 × 10⁻⁶ cm/sec are classified as high permeable.

### Caco-2 Permeability Assay

The colon carcinoma (Caco-2) cell permeability assay is the industry standard for in vitro prediction of intestinal absorption of drugs. Bidirectional permeability in Caco-2 cells assesses drug absorption and efflux, crucial for predicting absorption, evaluating drug bioavailability, and understanding transporter interactions in drug development. Compounds which have a Pₐₚₚ < 1 × 10⁻⁶ cm/sec are classified as low permeable, compounds with a Pₐₚₚ ≥ 1 × 10⁻⁶ cm/sec - ≤ 7 × 10⁻⁶ cm/sec as moderate permeable and compounds with a Pₐₚₚ > 7 × 10⁻⁶ cm/sec are classified as high permeable.

### Microsomal Stability Study (MLM)/Hepatocyte Stability Study

The assays use subcellular fractions of liver, microsomes, or isolated hepatocytes to investigate the metabolic fate of compounds. The percentage of test compound remaining is defined as the ratio of test compound peak area at a specific time point and the peak area in the t = 0 min samples multiplied by 100%. For MLM, Verapamil, a compound with a low metabolic stability, was used as a control for high clearance (Clint > 300 µl/min/mg protein, t_{1/2}< 5 min) and Metoprolol, a compound with a high metabolic stability, was used as a control for low clearance (Clint < 6 µl/min/mg protein, t_{1/2} > 240 min). For hepatocytes stability assays, Propranolol, a compound with a high metabolic stability, was used as a control for low clearance (Clint < 20 µl/min/10⁶ cells, t_{1/2} > 80 min) and 7-HC, a compound with a low metabolic stability, was used as a control for high clearance (Clint > 60 µl/min/10⁶ cells, t_{1/2} < 20 min)

### Kinetic Solubility

Kinetic solubility is determined from a pre-dissolved solution of the compound in organic solvent (DMSO) which is subsequently diluted into an aqueous buffer and incubated at room temperature for 4 hours. Ketoprofen, a compound with very high solubility, was used as a positive control (< 80 µM) and Estradiol, a compound with poor solubility, as a negative control (< 10 µM).

### Plasma Protein Binding (PPB)

The binding of test compounds to plasma proteins is an important factor affecting drug efficacy, metabolism and pharmacokinetic properties. In many cases, drug efficacy is determined by the concentration of free drug (unbound), rather than the total concentration in plasma. If the drug is highly bound to plasma proteins, the amount of drug available to reach the target is reduced. Subsequently, the efficacy of that compound may be significantly reduced. Therefore, information on the free drug fraction is essential for drug development and may be helpful in correlating with in vivo efficacy.

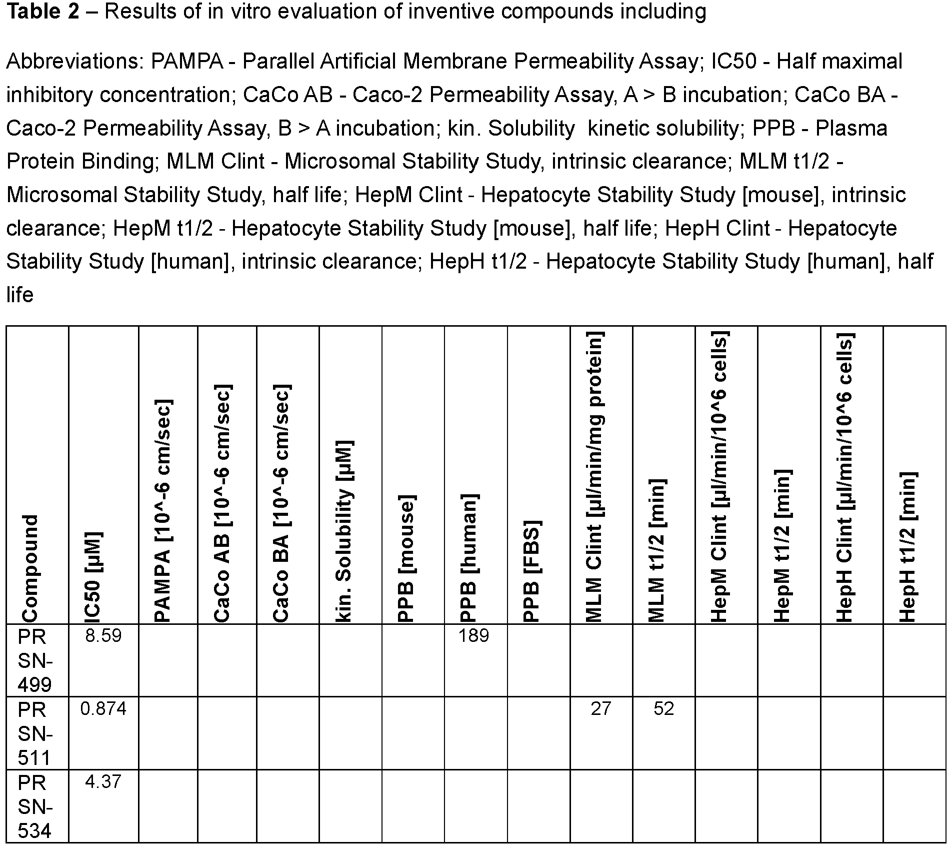

### Synthesis and analytical Chemistry:

### General strategy for the synthesis of EVH1-Inhibitors:

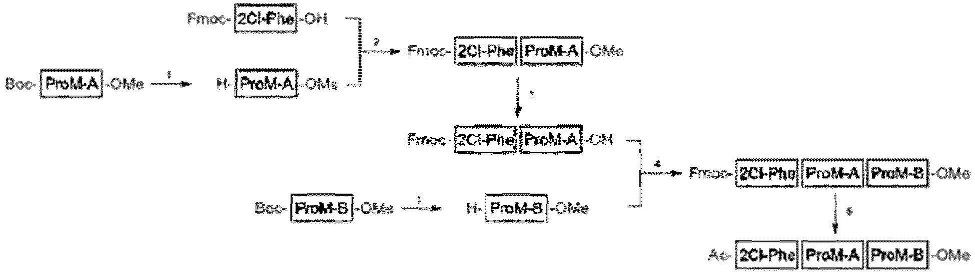

Within this general synthesis strategy ProM-A and ProM-B represent the same or different ProMs including dH-ProMs such as dH-ProM-2, dH-ProM-15, dH-ProM-27 and dH-ProM-28.

### General Procedure 1: Synthesis of HfProM-AIOMe / H[ProM-B]OMe

An inert flask was charged with Boc[ProM-A]OMe (1.00 eq.) and dry CH₂Cl₂ (5 mL/mmol of Boc[ProM-A]OMe). The reaction flask was cooled to 0 °C in order to add TMSOTf (1.3 eq.) dropwise. The mixture was stirred at 0 °C until complete consumption of the starting material was observed by TLC. Then, NaHCO₃ (aq.) was added and the resulting aqueous layer was extracted three times with CH₂Cl₂. The combined organic phases were dried over MgSO₄, filtered and the solvent was removed under reduced pressure to afford H[ProM-A]OMe.

### General Procedure 2: Synthesis of Fmoc[2Cl-F][ProM-A]OMe

An inert reaction flask was charged with Fmoc[2Cl-F]OH (1.2 eq.), HATU (1.4 eq.), DIPEA (1.2 eq.) and CH₂Cl₂ (25 mL/mmol of H[ProM-A]OMe). The reaction mixture was stirred for at least one hour at room temperature, until H[ProM-A]OMe (1.0 eq.) in CH₂Cl₂ (2.5 mL/mmol) and DIPEA (1.2 eq.) was added. The mixture was stirred at room temperature until complete consumption of the starting material was observed by TLC. Then, the solvent was removed under reduced pressure. The resulting crude material was purified by column chromatography to achieve the desired Fmoc[2Cl-F][ProM-A]OMe.

### General Procedure 3: Synthesis of Fmoc[2Cl-F1[ProM-A]OH

A round bottom flask was charged with Fmoc[2Cl-F][ProM-A]OMe (1.00 eq.) and a CaCl₂-solution (35 mL/mmol of Fmoc[2Cl-F][ProM-A]OMe; 0.8 mol/L in *i*PrOH/H₂O 7:3). To this well stirred mixture solid NaOH (1.2 eq.) was added. The mixture was stirred at room temperature until complete consumption of the starting material was observed by TLC. Then, a pH-value of 2-3 was adjusted using 1 _{N} HCl and the solvent was removed under reduced pressure. The received solid was dissolved in H₂O. The aqueous layer was extracted eight times using CH₂Cl₂. The combined organic phases were dried over MgSO₄, filtered and the solvent was removed under reduced pressure to achieve the desired Fmoc[2CI-F][ProM-A]OH.

### General Procedure 4: Synthesis of Fmoc[2CI-F][ProM-A][ProM-B]OMe

An inert reaction flask was charged with Fmoc[2Cl-F][ProM-A]OH (1.0 eq.), HATU (1.3 eq.), DIPEA (1.2 eq.) and CH₂Cl₂ (14 mL/mmol of Fmoc[2CI-F][ProM-A]OH). The reaction mixture was stirred for at least one hour at room temperature, until H[ProM-B]OMe (1.0 eq.) in CH₂Cl₂ (7 mL/mmol) and DIPEA (1.2 eq.) was added. The mixture was stirred at room temperature until complete consumption of the starting material was observed by TLC. Then, the solvent was removed under reduced pressure. The resulting crude material was purified by column chromatography to achieve the desired Fmoc[2CI-F][ProM-A][ProM-B]OMe.

### General Procedure 5: Synthesis of Ac[2Cl-F][ProM-A][ProM-B]OMe

A round bottom flask was charged with Fmoc[2CI-F][ProM-A][ProM-B]OMe (1.0 eq.), Piperidine (20 eq.) and MeCN (30 mL/mmol of Fmoc[2CI-F][ProM-A][ProM-B]OMe). The mixture was stirred at room temperature until complete consumption of the starting material was observed by TLC. Then, the solvent was removed under reduced pressure. The received solid was dissolved in dry CH₂Cl₂ (30 mL/mmol of Fmoc[2CI-F][ProM-A][ProM-B]OMe) and AczO (21 eq.) was added. The mixture was stirred for 18 hours, until the solvent was removed under reduced pressure. The resulting crude material was purified by normal phase column chromatography and reversed phase column chromatography to achieve the desired Ac[2Cl-F][ProM-A][ProM-B]OMe.

### Synthesis and characterization of example compounds

### Synthesis and characterization of BocrProM-401QtBu (Example compound 1)

### Step 1:

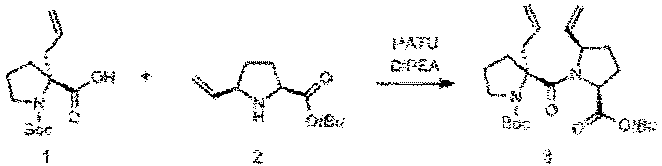

An inert *Schlenk*Flask equipped with a cooling finger was charged with 266 mg (1.04 mmol; 1.0 eq.) of compound 1,493 mg (1.3 mmol; 1.24 eq.) of HATU, 176 µL (1.01 mmol; 0.97 eq.) of DIPEA and 3 mL of MeCN. The reaction mixture was stirred for 1 h, until 240 mg (1.22 mmol; 1.17 eq.) of compound 2 in 3 mL MeCN and 196 µL (1.13 mmol; 1.08 eq.) of DIPEA was added. The orange mixture was warmed to 85 °C for 20 h and then cooled to room temperature. Then, 10% citric acid was added. The aqueous layer was extracted three times with CH₂Cl₂. The combined organic phases were washed with NaHCO₃ and brine, dried over MgSO₄, filtered and the solvent was removed under reduced pressure. The crude material was purified by column chromatography (CyHex/EtOAc 4:1) to receive 366 mg (842 µmol; 81%) of compound 3 as colorless oil.

**¹H-NMR:** (400 MHz, CDCl₃, mixture of rotameres) δ [ppm] = 6.22 - 5.91(m, 1H), 5.90 - 5.68 (m, 1H), 5.35 - 5.19 (m, 1H), 5. 19 - 5.00 (m, 3H), 4.73 - 4.62 (m, 0.7H), 4.62 - 4.46 (m, 0.7H), 4.46 - 4.36 (m, 0.3H), 4. 36 - 4.25 (m, 0.3H), 3.73 - 3.49 (m, 1.3H), 3.49 -3.29 (m, 0.7H), 3.11 - 2.96 (m, 0.5H), 2.91 - 2. 77 (m, 0.5H), 2.71 (dd, J = 13.9, 7.8 Hz, 1H), 2.22 - 1.75 (m,8), 1.47 (s, 9H 1.44 (s, 9H).

**¹³C-NMR:** (101 MHz, CDCl₃, mixture of rotameres) δ [ppm] = 171.9, 153.7, 140.2, 134.8, 118.6, 115.1, 82.1, 80. 6, 69.1, 63.4, 60.2, 47.8, 41.9, 35.6, 31.7, 28.5, 28.2, 28.1, 21.9.

**HR-MS (ESI):** Calcd. 435.28535 [M+H]⁺

Found 435.28551 [M+H]⁺.

**[α]_{λ}²⁰:** (c = 0.155 g/100 mL, CHCl₃) -44.7 (589 nm).

### Step 2:

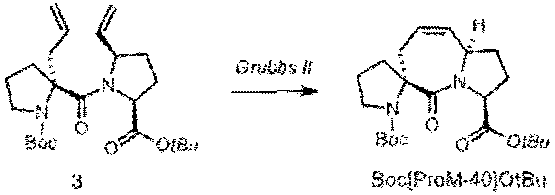

A round bottom flask was charged with 360 mg (828 µmol; 1.0 eq.) of compound 3, 10 mL of Et₂O, 29 mg (34.2 µmol; 4.1 mol%) of *Grubbs* // and 17 mg (89.3 µmol; 10.8 mol%) of Cul. The reaction mixture was warmed to 45 °C. After 3 h, another 31 mg (36.5 µmol; 4.41 mol%) of *Grubbs* // and 17 mg (89.3 µmol; 10.8 mol%) of Cul was added. After 7 h in total, the reaction monitoring by TLC indicated the complete consumption of the starting material. Therefore, Quadrasil^{™} was added and the solvent was removed under reduced pressure. The crude material was purified by column chromatography (CyHex/EtOAc 3:1) to receive 302 mg (743 µmol; 90%) of Boc[ProM-40]OtBu as slightly yellow solid.

**¹H-NMR:** (600 MHz, CDCl₃, mixture of rotameres) δ [ppm] = 6.10 (q, J = 7.4, 6.9 Hz, 1H), 6.01 - 5.95 (m, 1H), 4. 74 - 4.69 (m, 0.4H), 4.63 - 4.57 (m, 1H), 4.57 - 4.52 (m, 0.6H), 3.67 (dt, J = 10.6, 7.6 Hz, 0.6H), 3. 63 - 3.55 (m, 0.8H), 3.53 - 3.42 (m, 1H), 3.36 - 3.29 (m, 0.6H), 2.28 - 2.17 (m, 2H), 2.15 - 1. 97 (m, 4.4H), 1.97 - 1.90 (m, 1.2H), 1.90 - 1.83 (m, 0.4H), 1.83 - 1.75 (m, 1H), 1.

**¹³C-NMR:** (151 MHz, CDCl₃) δ [ppm] = 173.4, 173.3, 172.0, 171.6, 153. 7, 153.6, 135.9,135.3, 132.2, 132.0, 81.2, 80.8, 79.8, 79. 3, 67.9, 67.6, 62.2, 61.9, 55.4, 55.1, 48.9, 48.5, 38.9, 38.0, 33.6, 33.1, 32.6, 28.8, 28.7, 28.1, 27.4, 23.2, 22.6.

**HR-MS (ESI):** Calcd. 407.25405 [M+H]⁺
Found. 407.25379.

**[a]_{λ}²⁰:** (c = 0.505 g/100 mL, CHCl₃) -158.9 (589 nm).

**Melting point:** 144 - 145 °C.

### Crystal data and structure refinement:

Empirical formula C22 H34 N2 05
Moiety formula C22 H34 N2 05
Formula weight 406.51
Temperature 100(2) K
Wavelength 1.54178 Å
Crystal system Orthorhombic
Space group P212121
Unit cell dimensions a = 10.3416(8) Å a= 90°.
b = 11.6895(10) Å b= 90°.
c = 18.9680(14) Å g = 90°.
Volume 2293.0(3) Å3
Z4
Density (calculated) 1.178 Mg/m³
Absorption coefficient 0.674 mm⁻¹
F(000) 880
Crystal size 0.070 x 0.050 x 0.030 mm³
*θ* range for data collection 4.443 to 72.062°.
Index ranges -12<=h<=10, -13<=k<=14, -23<=I<=22
Reflections collected 16562
Independent reflections 4456 [R(int) = 0.1023]
Compl. to *θ* = 67.679° 99.7%
Absorption correction Semi-empirical from equivalents
Max. and min. transmission 0.7536 and 0.6129
Refinement method Full-matrix least-squares on F2
Data / restraints / param. 4456 / 0 / 269
Goodness-of-fit on F2 1.039
Final R ind. [I>2sigma(I)] R1 = 0.0692, wR2 = 0.1731
R indices (all data) R1 = 0.0778, wR2 = 0.1811
Absolute structure param. 0.0(2)
Extinction coefficient 0.0061 (12)
Largest diff. peak and hole 0.713 and -0.202 e.Å-3

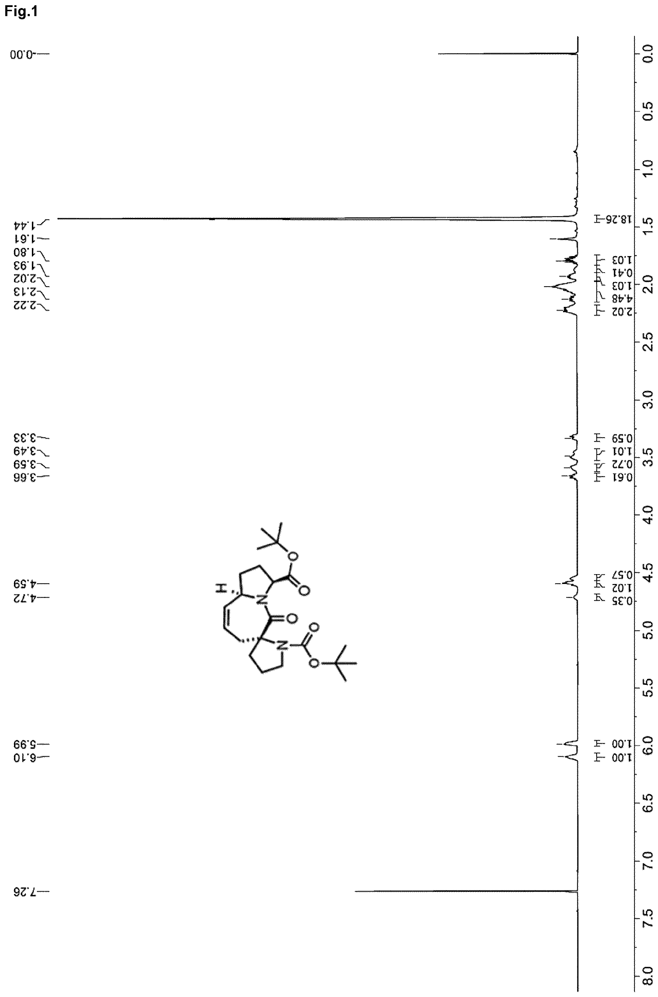

The ¹H-Spectrum of Boc[ProM-40]OtBu (Example compound 1) is shown in Figure 1.

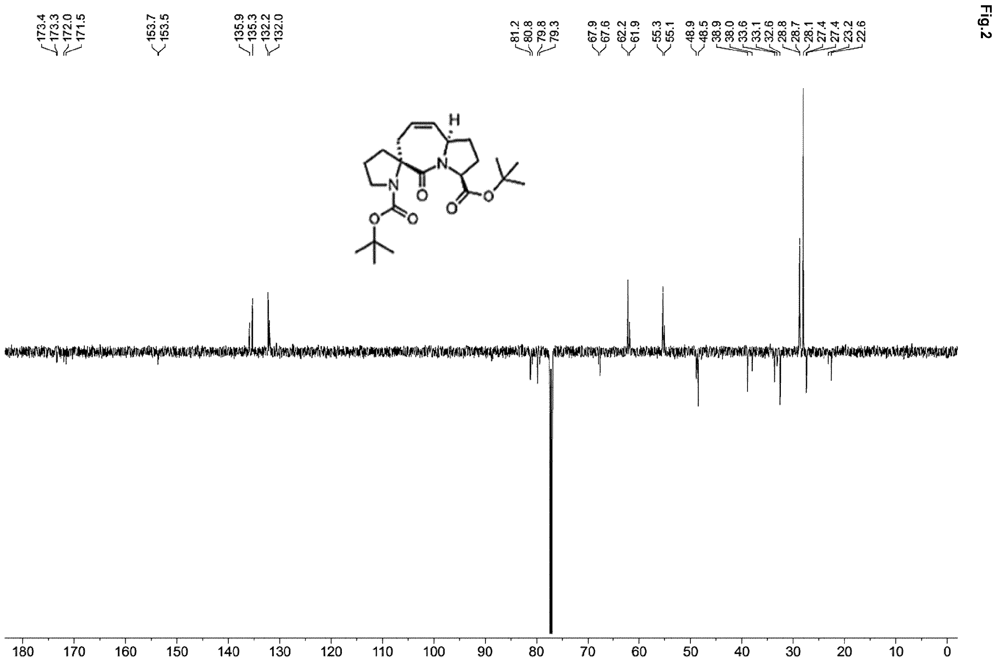

The ¹³C-Spectrum of Boc[ProM-40]OtBu (Example compound 1) is shown in Fig. 2

### Synthesis and characterization of H[ProM-40]OMe (Example compound 2)

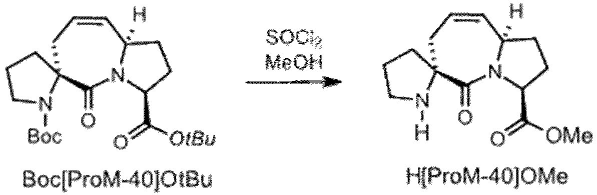

A round bottom flask was charged with 297 mg (731 µmol; 1.0 eq.) of Boc[ProM-40]OtBu and 8 mL of MeOH. The reaction mixture was cooled to 0°C. At this temperature, 0.28 mL (3.86 mmol; 5.28 eq.) of SOCl₂ was added slowly. The reaction mixture was allowed to warm up to room temperature overnight. Then, the solvent was removed under reduced pressure. The crude material was dissolved in saturated NaHCO₃. The aqueous layer was extracted six times using CH₂Cl₂. The combined organic phases were dried over MgSO₄, filtered and the solvent was removed under reduced pressure to receive 157 mg (594 µmol; 81%) of H[ProM-40]OMe as yellowish solid.

**¹H-NMR:** (600 MHz, CDCl₃) δ [ppm] = 5.70 - 5.66 (m, 1H), 5.66 - 5.62 (m, 1H), 4.98 - 4.93 (m, 1H), 4.61 (dd, J = 8.9, 1.5 Hz, 1H), 3.71 (s, 3H), 3.05 (ddd, J = 10.8, 7.2, 5.7 Hz, 1H), 2.87 (dt, J = 10.7, 6.8 Hz, 1H), 2.78 (ddd, J = 12.8, 7.8, 6.6 Hz, 1H), 2.70 (ddt, J = 17.9, 3.6, 1.7 Hz, 1H), 2.30 - 2.19 (m, 2H), 2.09 - 2.04 (m, 1H), 1.99 - 1.94 (m, 2H), 1.85 - 1.77 (m, 1H), 1.74 - 1.65 (m, 1H), 1.45 - 1.40 (m, 1H).

**¹³C-NMR:** (150 MHz, CDCl₃) δ [ppm] = 174.1, 173.1, 129.8, 126.2, 68.1, 61.5, 56.8, 52.2, 47.7, 38.0, 36.7, 33.5, 26.8, 26.2.

**HR-MS (ESI):** Calcd. 265.15467 [M+H]⁺
Found. 265.15467 [M+H]⁺

**[α]_{λ}²⁰**: (c = 0.515 g/100 mL, CHCl₃) -78.1 (589 nm).

### Melting point: 110 - 111°C.

### Synthesis and characterization of Fmoc[2Cl-F][ProM-401OMe (Example compound 3)

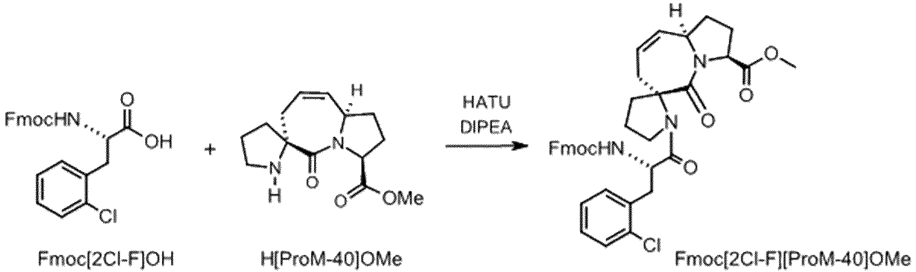

An inerted *Schlenk*flask was charged with 270 mg (0.64 mmol; 1.10 eq.) of FmoC[2CI-F]OH, 291 mg (0.765 mmol; 1.31 eq.) of HATU, 107 µL (0.614 mmol; 1.05 eq.) of DIPEA and 8 mL of CH₂Cl₂. The reaction mixture was stirred at RT for 2 h, until 154 mg (0.583 mmol; 1.0 eq.) of H[ProM-40]OMe in 8 mL MeCN and 117 µL (0.672 mmol; 1.15 eq.) of DIPEA were added. The reaction conversion was monitored by TLC and a complete consumption of the starting material was observed after 20 h. Then, the solvent was removed under reduced pressure. The received crude material was purified by column Chromatography (CyHex/EtOAc/MeOH (40:40:1) to achieve 353 mg (528 µmol; 91%) of Fmoc[2Cl-F][ProM-40]OMe as colorless solid.

**¹H-NMR:** (499 MHz, CDCl₃) δ [ppm] = 7.74 (d, J = 7.6 Hz, 2H), 7.50 (dd, J = 7.6, 4.4 Hz, 2H), 7.38 (td, J = 7.6, 2.7 Hz, 2H), 7.33 (dd, J = 7.7, 1.6 Hz, 1H), 7.31 - 7.25 (m, 2H), 7.24 (dd, J = 7.3, 2.0 Hz, 1H), 7.19 - 7.05 (m, 2H), 6.14 - 6.07 (m, 1H), 6.06 - 6.00 (m, 1H), 5.61 (d, J = 9.1 Hz, 1H), 4.86 (td, J = 9.8, 3.9 Hz, 1H), 4.80 - 4.70 (m, 2H), 4.26 - 4.18 (m, 1H), 4.08 - 4.00 (m, 2H), 3.96 - 3.83 (m, 2H), 3.71 (s, 3H), 3.62 (dd, J = 14.3, 6.0 Hz, 1H), 3.37 (dd, J = 14.1, 4.0 Hz, 1H), 2.78 (dd, J = 14.1, 10.3 Hz, 1H), 2.34 - 1.90 (m, 9H).

**¹³C-NMR:** (126 MHz, CDCl₃) δ [ppm] = 172.9, 172.3, 169.7, 155.6, 144.0, 143.8, 141.3, 141.2, 136.1, 134.5, 134.2, 132.5, 131.5, 129.4, 128.5, 127.6, 127.6, 127.1, 127.0, 126.7, 125.3, 125.2, 119.9, 69.4, 66.9, 60.8, 55.1, 52.5, 52.2, 48.8, 47.0, 36.8, 36.0, 32.3, 31.5, 27.3, 24.0.

**HR-MS (ESI):** Calcd. 668.25219 [M+H]⁺
Found 668.25306 [M+H]⁺

**[α]_{λ}²⁰:** (c = 0.155 g/100 mL, CHCl₃): -125.0 (589 nm).

### Synthesis and characterization of Fmoc[2Cl-F][ProM-40]OH (Example compound 4)

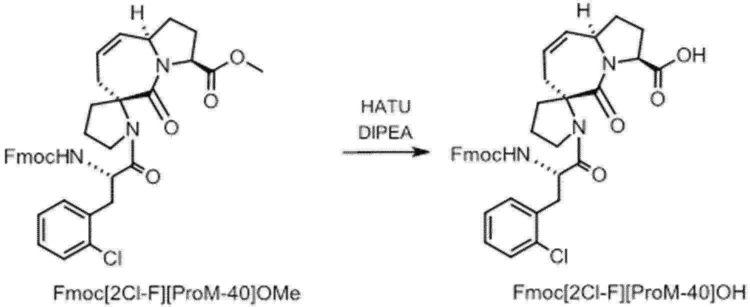

A round bottom flask was charged with 200 mg (0.30 mmol, 1.0 eq.) Fmoc[2CI-F][ProM-40]OMe (example compound 3), 13 mg (0.32 mmol, 1.1 eq.) NaOH and 10.3 mL (8.2 mmol, 27 eq.) CaCl₂ solution (0.8 M, iPrOH/H₂O 7:3). The reaction was monitored by TLC, and complete conversion of the starting material was observed after 3 days. The solution was acidified with 1 N HOAc to pH = 4 and then extracted with EtOAc. After drying the organic layers over MgSO₄, the solvent was removed under reduced pressure. The crude product was taken up in 1 mL MeOH, and 15 mL water was added to precipitate the product. After filtration and drying, the precipitate was obtained as a yellowish solid. Further product was obtained from the mother liquor by extraction with EtOAc, drying over MgSO₄ and removing the solvent. In total, the Fmoc[2Cl-F][ProM-40]OHwas obtained as a yellowish solid (159 mg, 0.24 mmol, 81%).

**¹H-NMR:** (499 MHz, CDCl₃) δ [ppm] = 7.87 (q, J = 6.9 Hz, 2H), 7.77 (d, J = 9.1 Hz, 1H), 7.65 (d, J = 7.6 Hz, 1H), 7.61 (d, J = 7.5 Hz, 1H), 7.51 - 7.45 (m, 1H), 7.45 - 7.37 (m, 3H), 7.37 - 7.25 (m, 2H), 7.22 (td, J = 7.7, 1.7 Hz, 1H), 7.15 (t, J = 7.4 Hz, 1H), 6.18 - 6.07 (m, 1H), 6.05 - 5.95 (m, 1H), 4.67 - 4.58 (m, 1H), 4.56 - 4.47 (m, 1H), 4.44 - 4.34 (m, 1H), 4.16 - 4.02 (m, 3H), 3.77 - 3.61 (m, 2H), 3.47 - 3.40 (m, 1H), 3.05 (dt, J = 14.3, 4.2 Hz, 1H), 2.75 (ddd, J = 14.7, 11.0, 4.4 Hz, 1H), 2.32 - 2.18 (m, 1H), 2.14 - 1.79 (m, 8H).

**¹³C-NMR:** (126 MHz, CDCl₃) δ [ppm] = 173.3, 172.3, 172.0, 155.7, 143.6, 140.7, 137.4, 135.8, 135.0, 132.6, 131.7, 131.5, 129.1, 129.0, 128.4, 127.6, 127.3, 127.0, 126.8, 125.4, 125.3, 121.4, 120.1, 68.7, 65.6, 60.5, 54.6, 52.0, 48.0, 46.6, 36.1, 34.0, 31.8, 31.0, 26.8, 23.5.

**HR-MS (ESI):** Calcd. 654.23654 [M+H]⁺
Found. 654.23759 [M+H]⁺

### Melting point: 200 - 210 °C (decomposed.)

### Synthesis and characterization of Fmoc[2Cl-F][ProM-40][ProM-1]OMe (Example compound 5)

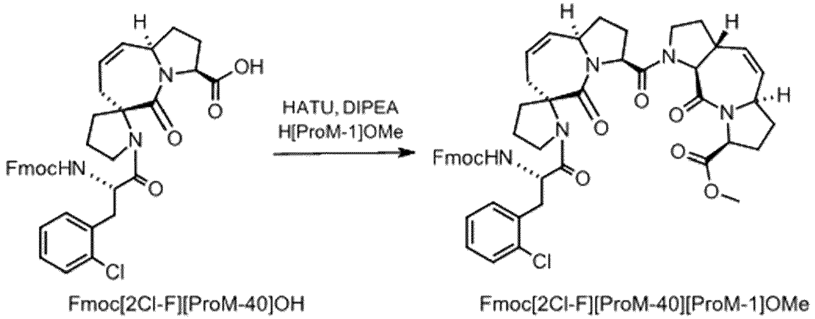

An inerted *Schlenk*flask was charged with 75 mg (0.11 mmol, 1.0 eq.) of Fmoc[2Cl-F][ProM-40]OH, 96 mg (0.25 mmol, 2.3 eq.) of HATU, 20 µL (0.13 mmol; 1.2 eq.) of DIPEA and 2 mL of CH₂Cl₂ at 0°C. The reaction mixture was stirred for 1 h, until 33 mg (0.13 mmol, 1.2 eq.) of H[ProM-1]OMe in 2 mL of CH₂Cl₂ and 20 µL (0.13 mmol; 1.2 eq.) of DIPEA were added. The reaction mixture was warmed to room temperature. The reaction conversion was monitored by TLC and a complete consumption of the starting material was observed after 17 h. Then, the solvent was removed under reduced pressure. The received crude material was purified by column Chromatography (CyHex/EtOAc/MeOH (5:5:1) to achieve (89 mg, 0.10 mmol, 91 %) of Fmoc[2CI-F][ProM-40][ProM-1]OMe as colorless solid.

**¹H-NMR:** (499 MHz, CDCl₃) δ [ppm] = 7.76 - 7.72 (m, 2H), 7.50 (t, J = 8.3 Hz, 2H), 7.42 - 7.35 (m, 3H), 7.34 - 7.27 (m, 3H), 7.23 - 7.17 (m, 2H), 6.13 - 6.03 (m, 2H), 5.86 (d, J = 11.2 Hz, 1H), 5.59 (d, J = 11.2 Hz, 1H), 5.03 (d, J = 8.3 Hz, 1H), 4.86 - 4.71 (m, 4H), 4.65 (d, J = 11.1 Hz, 1H), 4.23 (dd, J = 10.2, 7.2 Hz, 1H), 4.17 - 4.07 (m, 3H), 4.06 - 4.00 (m, 1H), 3.96 - 3.89 (m, 1H), 3.71 (s, 3H), 3.63 - 3.57 (m, 1H), 3.48 - 3.43 (m, 1H), 3.37 - 3.32 (m, 1H), 3.06 - 3.01 (m, 1H), 2.95 - 2.88 (m, 1H), 2.40 - 2.06 (m, 13H), 1.88 (dt, J = 20.6, 11.0 Hz, 2H).

¹³C-NMR: (126 MHz, CDCl₃) δ [ppm] = 173.6, 172.0, 171.6, 169.4, 156.0, 143.9, 143.7, 141.4, 135.8, 132.2, 131.1, 129.9, 129.6, 129.0, 128.0, 127.8, 127.2, 127.2, 125.4, 125.3, 120.1, 69.8, 67.4, 62.8, 61.7, 59.9, 58.0, 56.8, 52.9, 52.6, 48.6, 48.3, 47.1, 40.5, 35.6, 34.8, 32.9, 32.0, 31.3, 30.4, 29.9, 27.2, 23.8.

**HR-MS (ESI):** Calcd. 886.35772 [M+H]⁺
Found. 886.35895 [M+H]⁺

### Synthesis and characterization of Ac[2Cl-F][ProM-40][ProM-1]OMe (Example compound 6)

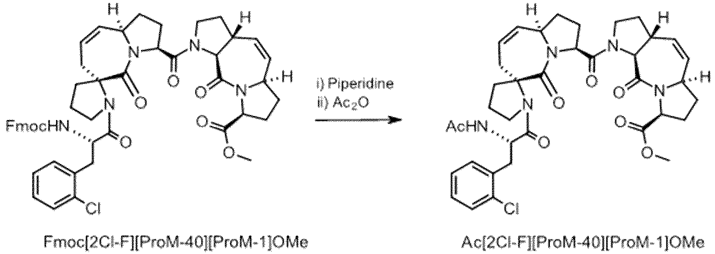

A round bottom flask was charged with 60 mg (67 µmol, 1.0 eq.) of Fmoc[2CI-F][ProM-40][ProM-1]OMe, 1.0 mL (10 mmol, 150 eq.) piperidine and 3.6 mL of MeCN. The reaction mixture was stirred at room temperature for 3 h, until the solvent was removed under reduced pressure. The residue was dissolved in 3.6 mL MeCN and treated again with 1.0 mL (10 mmol, 150 eq.) of piperidine. The reaction mixture was stirred at room temperature for 3 h, until the solvent was removed under reduced pressure. The residue was dissolved in 3.0 mL of CH₂Cl₂ and 0.5 mL (5.4 mmol, 80 eq.) of Ac₂O were added. The reaction mixture was stirred for 18 h. Then, the solvent was removed under reduced pressure. The crude material was purified by column chromatography (CyHex/EtOAc/MeOH 3:3:1) to receive 15 mg (21 µmol, 31%) Ac[2Cl-F][ProM-40][ProM-1]OMe.

**¹H-NMR:** (500 MHz, CDCl₃) δ [ppm] = 7.33 (dd, J = 7.3, 2.0 Hz, 1H), 7.24 (dd, J = 6.9, 2.4 Hz, 1H), 7.19 - 7.13 (m, 2H), 6.30 (d, J = 8.8 Hz, 1H), 6.09 - 5.99 (m, 2H), 5.83 (dt, J = 11.1, 2.2 Hz, 1H), 5.55 (ddd, J = 11.2, 2.9, 1.6 Hz, 1H), 5.09 (td, J = 9.4, 4.3 Hz, 1H), 4.97 (d, J = 8.7 Hz, 1H), 4.78 - 4.71 (m, 2H), 4.70 - 4.64 (m, 1H), 4.55 (d, J = 10.9 Hz, 1H), 4.19 - 4.13 (m, 1H), 3.97 - 3.91 (m, 1H), 3.82 - 3.76 (m, 1H), 3.69 (s, 3H), 3.58 - 3.50 (m, 2H), 3.34 - 3.27 (m, 1H), 3.02 - 2.93 (m, 1H), 2.81 (dd, J = 14.2, 9.8 Hz, 1H, H-3), 2.39 - 2.26 (m, 3H, H-21/H-33), 2.26 - 2.19 (m, 3H), 2.19 - 2.09 (m, 2H), 2.09 - 1.94 (m, 6H), 1.90 - 1.78 (m, 5H).

**¹³C-NMR:** (126 MHz, CDCl3) δ [ppm] = 172.5, 171.8, 171.3, 169.7, 169.6, 169.2, 137.0, 134.7, 134.4, 132.3, 130.8, 129.7, 129.6, 128.7, 128.5, 126.7, 69.6, 62.2, 60.1, 59.4, 57.6, 55.3, 52.4, 50.8, 48.8, 47.2, 40.5, 36.7, 35.4, 33.1, 32.4, 31.8, 31.3, 27.6, 27.3, 24.0, 23.2.c

**HR-MS (ESI):** Calcd. 706.30020 [M+H]⁺
Found. 706.30109 [M+H]⁺

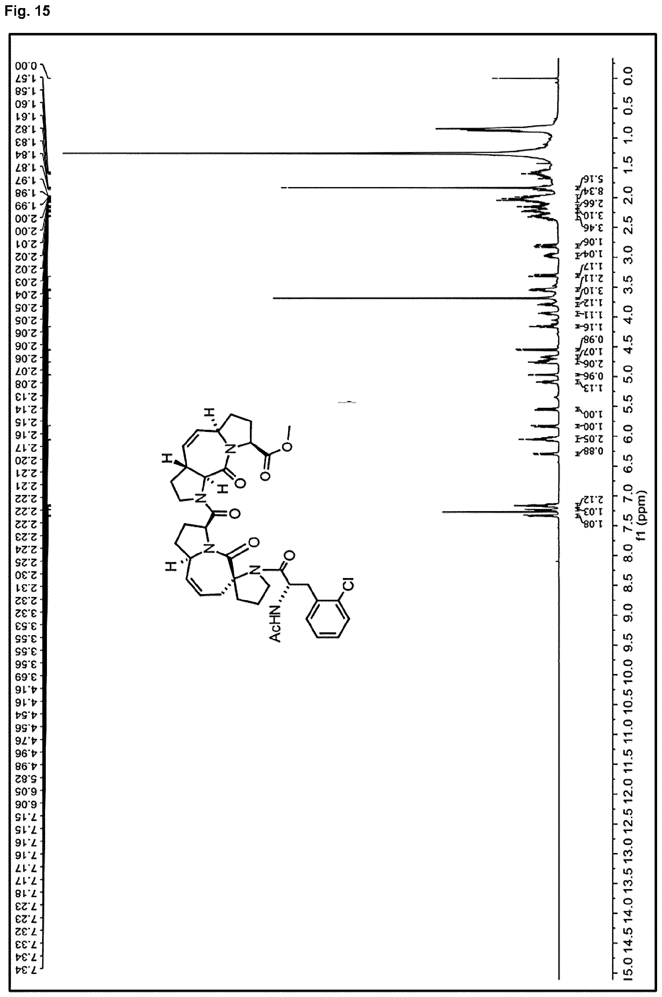

The ¹H-Spectrum of Ac[2Cl-F][ProM-40][ProM-1]OMe (Example compound 6) is shown in Figure 15.

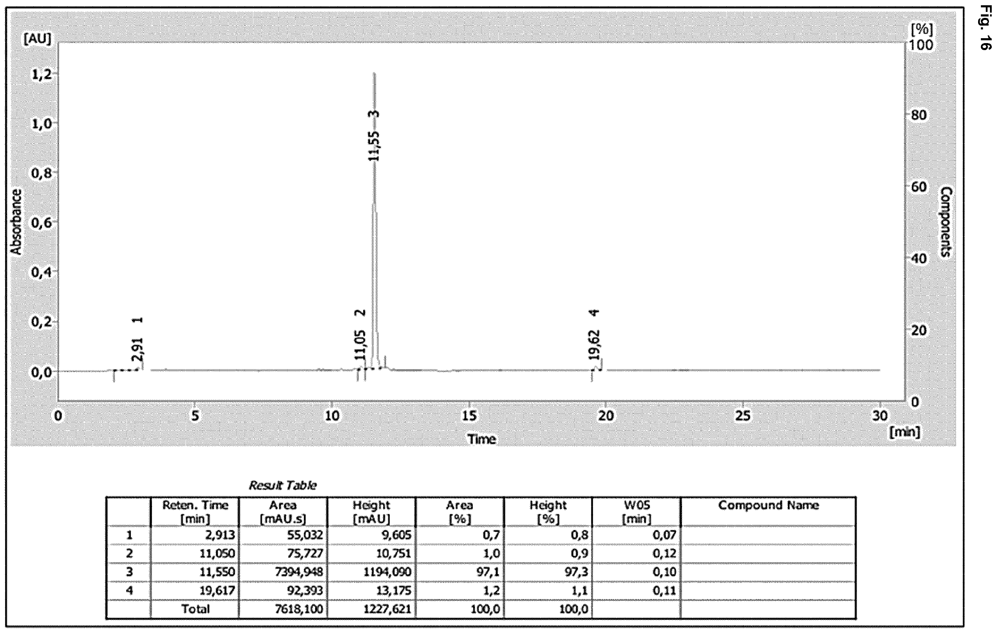

The HPLC-Chromatogram of Ac[2Cl-F][ProM-40][ProM-1]OMe (Example compound 6) is shown in Fig. 16.

The following conditions were used for HPLC: Column: XSelect CSH C18 from Waters. Temperature: 30 °C. Gradient: Water/MeCN (95:5) to water/MeCN (10:90) over 15 minutes. Hold the mixture for 10 minutes. Initial gradient is resumed over 5 minutes.

### Synthesis and characterization of Boc[ProM-24]OtBu (Example compound 7)

### Step1:

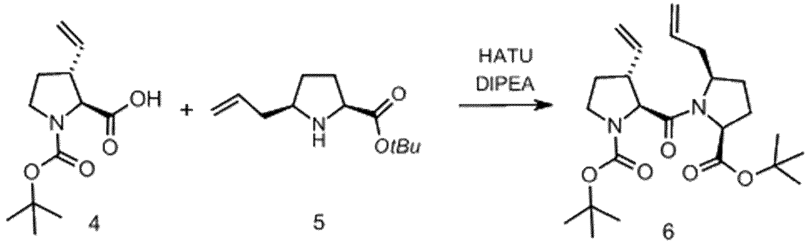

An inert *Schlenk*flask was charged with 1.04 g (4.31 mmol; 1.0 eq.) of compound **4,** 2.04 g (4.54 mmol; 1.05 eq.) of HATU, 758 µL (4.35 mmol; 1.03 eq.) of DIPEA and 6 mL of MeCN. The reaction mixture was stirred at room temperature for 1 h, until 960 mg (4.54 mmol; 1.0 eq.) of compound 5 in 6 mL of MeCN and 758 µL (4.35 mmol; 1.03 eq.) of DIPEA was added. The reaction was monitored by TLC and the complete consumption of the starting material was observed after 20 h. Then, 10% citric acid was added. The aqueous layer was extracted three times using CH₂Cl₂. The combined organic phases were washed with NaHCO₃, brine, dried over MgSO₄, filtered and the solvent was removed under reduced pressure. The crude material was purified by column chromatography (CyHex/EtOAc 5:1 to 3:1) to receive 1.5 g (3.46 mmol; 80%) of compound 6 as yellowish oil.

**¹H-NMR:** (300 MHz, CDCl₃) δ [ppm] = 5.96 - 5.69 (ddt, J = 36.1; 17.8; 9.6 Hz, 2H), 5.18 - 5.14 (m, 2H), 5.12 - 5.06 (m, 2H), 4.46 - 4.38 (dd, J = 15.2; 7.9 Hz, 1H), 4.35 - 4.33 (m, 1H), 3.78 - 3.41 (m, 2H), 3.00 - 2.91 (m, 1H), 2.51 - 2.37 (m, 2H), 2.34 - 2.10 (m, 2H), 2.03-1.97 (m, 1H), 1.95-1.85 (m, 2H), 1.77 - 1.61 (m, 2H), 1.53 - 1.47 (m, 9H), 1.44 - 1.41 (d, J = 9.1 Hz, 9H).

**¹³C-NMR:** (75 MHz, CDCl₃) δ [ppm] = 171.6, 171.2, 154.4, 138.5, 134.3, 117.0, 111.3, 81.1, 79.9, 61.7, 60.4, 58.2, 47.8, 46.9, 39.7, 30.2, 29.2, 28.4, 28.3, 28.3, 28.3, 28.2, 28.0, 27.9. GC-MS: m/z (%) = 434 ([M]+).

**[α]_{λ}²⁰:** (c = 0.550 g/100 mL, CHCl3) -12.2 (589 nm).

### Step 2:

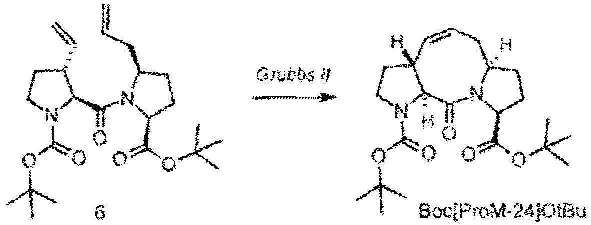

A round bottom flask was charged with 1.01 g (2.32 mmol; 1.0 eq.) of compound 6, 17 mL of Toluene, 69 mg (110 µmol; 4.7 mol%) of *Grubbs Hoveyda II* and 32 mg (296 µmol; 12.7 mol%) of 1,4-Benzoquinone. The reaction mixture was warmed to 80 °C. After 3 h, another batch of 30 mg (47.9 µmol; 2.1 mol%) *Grubbs Hoveyda II* was added. After 4 h in total, the reaction monitoring by TLC indicated the complete consumption of the starting material. Therefore, Quadrasil^{™} was added and the solvent was removed under reduced pressure. The crude material was purified by column chromatography (CyHex/EtOAc 3:1 to 2:1) to receive 680 mg (1.67 mmol; 72%) of Boc[ProM-24]OtBu as colorless solid.

**¹H-NMR:** (300 MHz, CDCl₃) δ [ppm] = 5.93 - 5.84 (m, 1H), 5.80 - 5.76 (dd, J = 11.0; 2.2 Hz, 1H), 4.54 - 4.45 (m, 1H), 4.40 - 4.26 (dd, J = 33.2; 7.3 Hz, 1H), 4.09 - 4.03 (t, J = 7.7 Hz, 1H), 3.82 - 3.73 (m, 1H), 3.36 - 3.24 (tt, J = 11.8; 5.9 Hz, 1H), 3.16 - 3.05 (m, 1H), 3.00 - 2.87 (m, 1H), 2.30 - 2.16 (m, 2H), 2.14 - 2.04 (m, 2H, 1.93 - 1.84 (dd, J = 19.2; 7.3 Hz, 1H, 1.78 - 1.72 (m, 2H), 1.46 - 1.45 (s, 9H), 1.36 (d, J = 26.6 Hz, 9H).

**¹³C-NMR:** (75 MHz, CDCl3) δ [ppm] = 174.0, 170.5, 152.6, 136.4, 131.1, 81.2, 81.0, 60.6, 45.8, 31.7, 29.8, 29.5, 28.7, 28.5, 28.2, 28.0, 27.9, 27.6, 27.3. GC-MS: m/z (%) = 406 ([M]+).

**[α]_{λ}²⁰:** (c = 0.650 g/100 mL, CHCl₃) - 198.8 (589 nm).

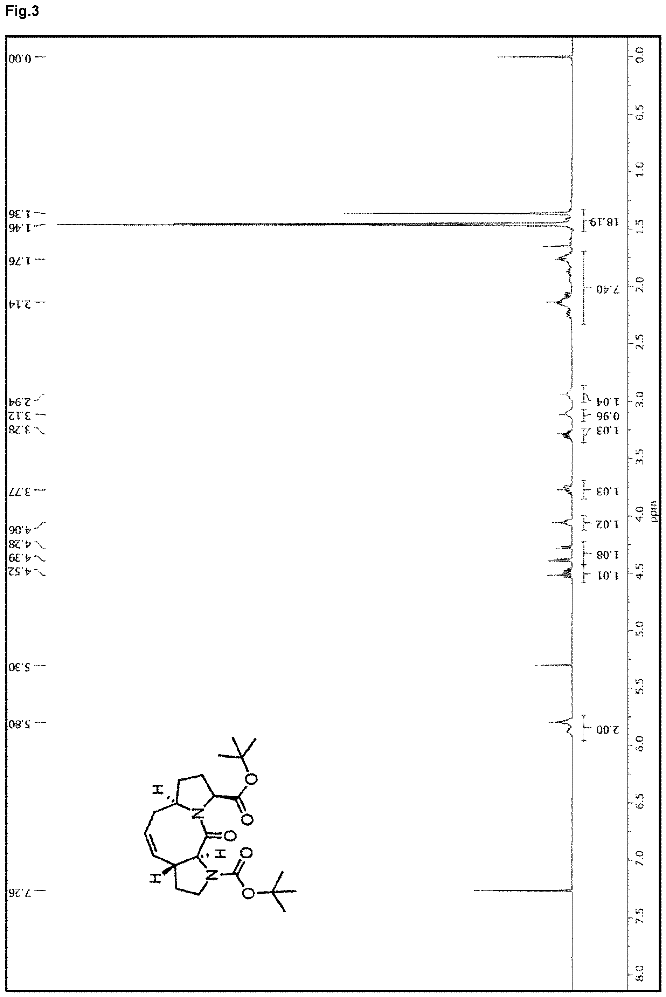

The ¹H-Spectrum of Boc[ProM-24]OtBu (Example compound 7) is shown in Fig. 3.

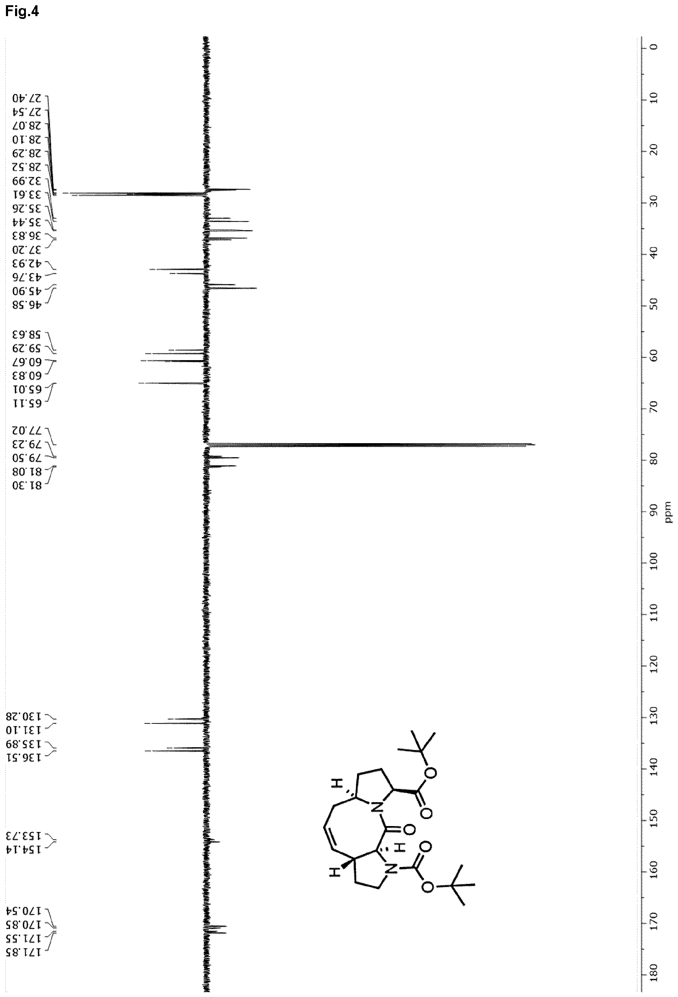

The ¹³C-Spectrum of Boc[ProM-24]OtBu (Example compound 7) is shown in Fig. 4

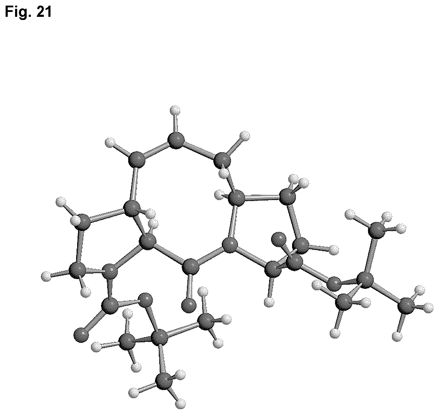

The X-ray crystal structure of Boc[ProM-24]OtBu is shown in Fig. 21.

### Characterization of Boc[dH-ProM-24]OtBu (Example compound 8)

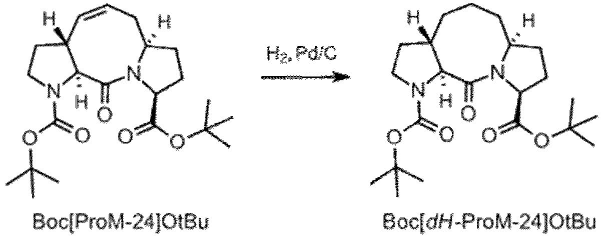

A Schlenkflask was charged with 275 mg (676 µmol; 1.0 eq.) of Boc[ProM-24]OtBu, 141 mg (132 µmol; 0.20 eq.; 10 w%) of Pd/C and 6 mL of MeOH. An argon atmosphere was applied and the reaction mixture was vigorously stirred. After 18 h, the mixture was filtered through a pad of Celite and the solvent was removed under reduced pressure to receive 277 mg (676 µmol; quant.) of Boc[dH-ProM-24]OtBu as colorless oil.

**¹H-NMR:** (500 MHz, CDCl₃; mixture of rotamers) δ = 4.53 - 4.26 (m, 2H), 4.11 (m, 1H), 3.66 (m, 1H), 3.44 - 3.31 (m, 1H), 2.37 - 2.22 (m, 1H), 2.21 - 2.09 (m, 1H), 2.09 - 1.79 (m, 5H), 1.77 - 1.50 (m, 5H), 1.50 - 1.35 (m, 18H), 1.33 - 1.20 (m, 1H).

¹³C-NMR: (125 MHz, CDCl₃; mixture of rotamers) δ = 171.95, 171.66, 170.21, 169.66, 154.41, 153.68, 81.19, 80.84, 79.30, 79.22, 64.35, 63.84, 60.56, 60.22, 55.97, 55.89, 47.82, 47.66, 46.88, 46.16, 36.26, 36.18, 33.53, 32.95, 32.44, 32.33, 31.35, 28.57, 28.30, 28.12, 26.95, 26.78, 24.12, 23.91.

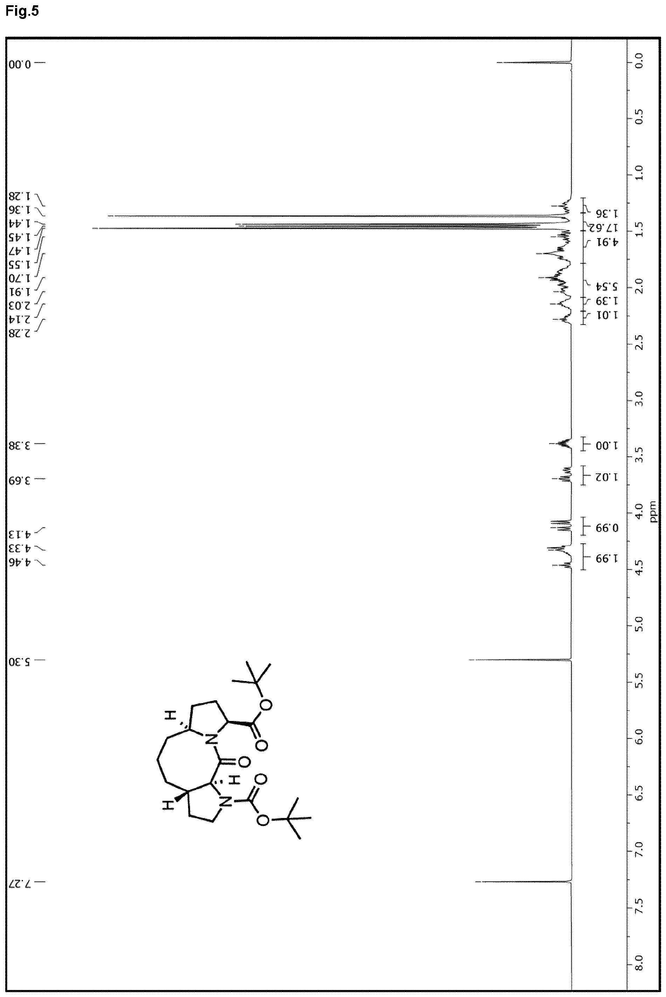

The ¹H-Spectrum of Boc[dH-ProM-24]OtBu (Example compound 8) is shown in Figure 5.

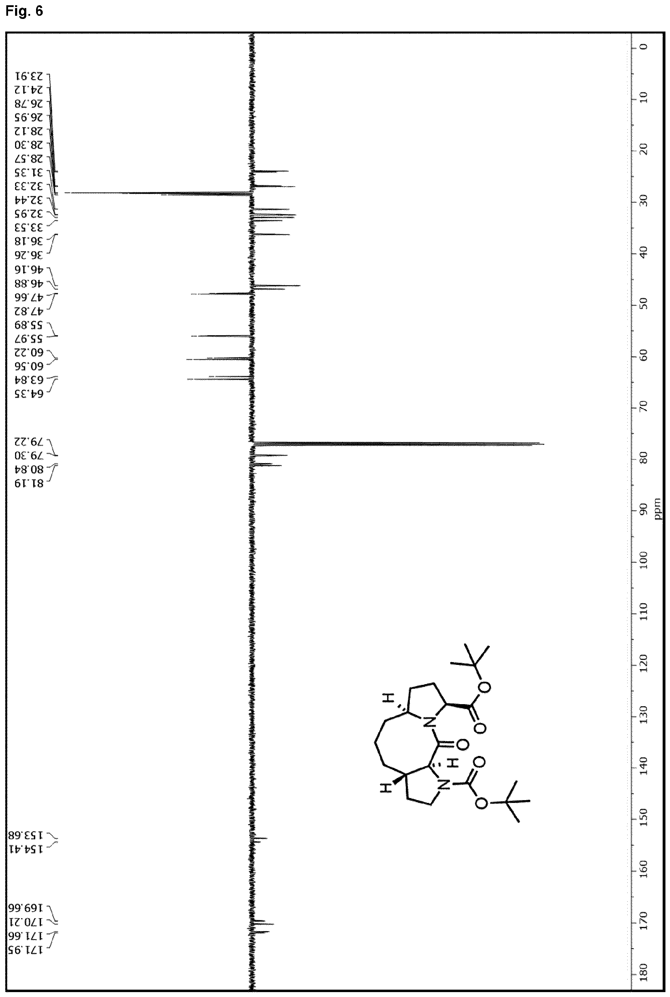

The ¹³C-Spectrum of Boc[dH-ProM-24]OtBu (Example compound 8)is shown in Fig. 6.

### Characterization of Boc[ProM-40']OtBu (Example compound 9)

### Step 1:

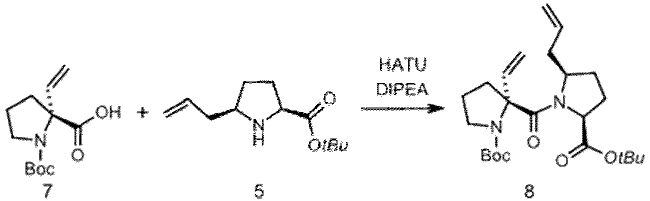

An inert *Schlenk*flask equipped with a reflux condenser was charged with 2.01 g (8.33 mmol; 1.0 eq.) of compound 7, 4.12 g (10.8 mmol; 1.3 eq.) of HATU, 1.5 mL (8.61 mmol; 1.03 eq.) of DIPEA and 15 mL of MeCN. The reaction mixture was stirred for 1 h, until 1.96 g (9.28 mmol; 1.1 eq.) of compound 5 in 15 mL of MeCN and 1.5 mL (8.61 mmol; 1.03 eq.) of DIPEA were added. The reaction mixture was heated to 80 °C, until complete consumption of the starting material was observed by TLC (18 h). Then, 10% citric acid was added. The aqueous layer was extracted three times using CH₂Cl₂. The combined organic phases were washed with sat. NaHCO₃, brine, dried over MgSO₄, filtered and the solvent was removed under reduced pressure. The crude product was purified by column chromatography in CyHex/EtOAc (4:1) to receive 3.36 g (7.72 mmol; 93%) of compound **8** as yellowish oil.

### Step 2:

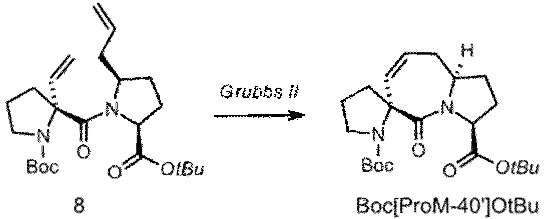

A round bottom flask was charged with 3.34 g (7.69 mmol; 1.0 eq.) of compound **8,** 70 mL of Et₂O, 265 mg (312 µmol; 4.1 mol%) of *Grubbs II* and 90 mg (473 µmol; 6.15 mol%) of CuI. The reaction mixture was warmed to 45 °C. After 1 h, another batch of 263 mg (310 µmol; 4.0 mol%) *Grubbs II* and 91 mg (478 µmol; 6.2 mol%) of Cul were added. After 6 h in total, the reaction monitoring by TLC indicated the complete consumption of the starting material. Therefore, Quadrasil^{™} was added and the solvent was removed under reduced pressure. The crude material was purified by column chromatography (CyHex/EtOAc 4:1) to receive 2.99 g (7.38 mmol; 96%) of Boc[ProM-40']OtBu as orange oil.

**¹H NMR:** (500 MHz, CDCl₃; mixture of rotamers) δ = 5.89 - 5.75 (m, 2H), 4.58 (d, J = 9.0 Hz, 1H), 3.83 (s, 1H), 3.65 (dt, J = 10.4, 6.8 Hz, 1H), 3.53 (dt, J = 10.3, 6.7 Hz, 1H), 2.74 - 2.59 (m, 1H), 2.53 (ddd, J = 15.5, 10.7, 2.2 Hz, 1H), 2.29 (dddd, J = 15.3, 5.9, 3.8, 1.3 Hz, 1H), 2.14 - 1.94 (m, 4H), 1.93 - 1.73 (m, 3H), 1.51 - 1.23 (m, 18H).

**¹³C NMR:** (125 MHz, CDCl₃; mixture of rotamers) δ = 171.87, 170.77, 154.00, 125.49, 80.82, 79.39, 69.81, 63.21, 60.91, 53.44, 48.07, 33.19, 31.66, 28.54, 27.96, 26.47, 23.57.

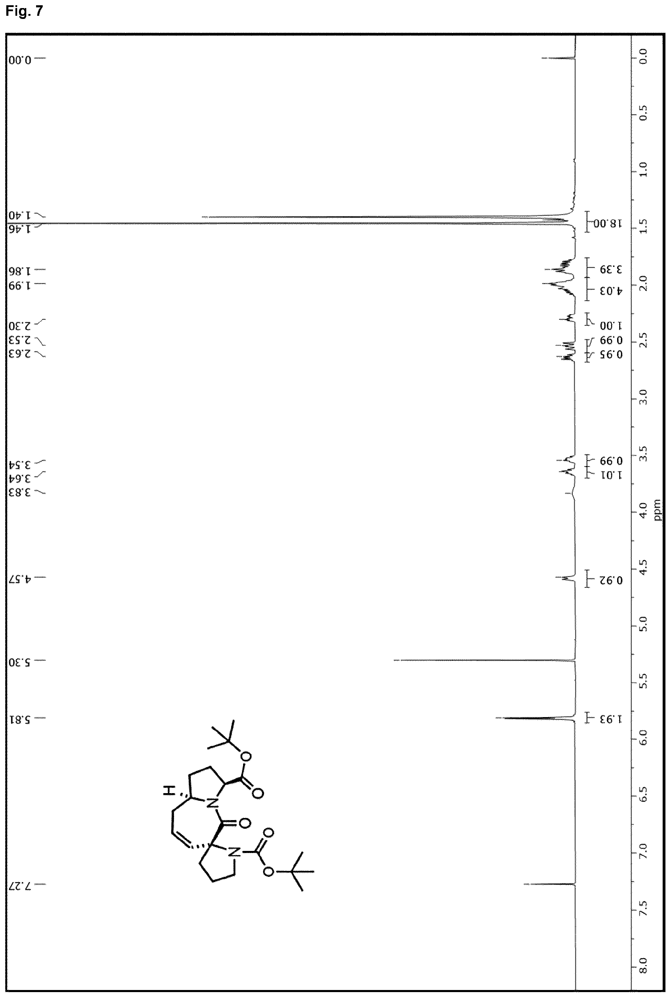

The ¹H-Spectrum of Boc[ProM-40']OtBu (Example compound 9) is shown in Figure 7.

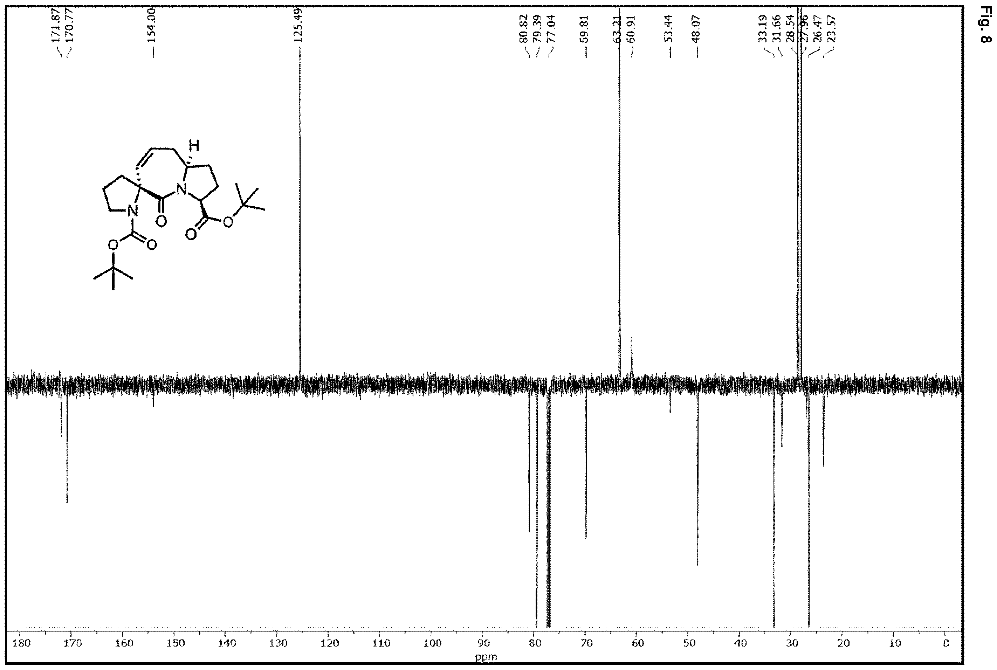

The ¹³C-Spectrum of Boc[ProM-40']OtBu (Example compound 9) is shown in Fig. 8.

### Characterization of Boc[dH-ProM-40]OtBu (Example compound 10)

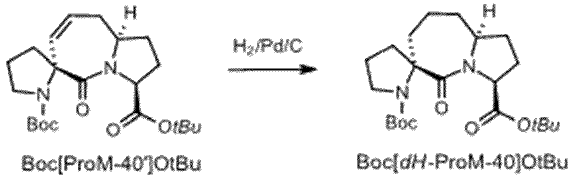

A *Schlenk*flask was charged with 3.0 g (7.38 mmol; 1.0 eq.) Boc[ProM-40']OtBu, 160 mg (705 µmol; 0.10 eq.) of Pt₂O and 70 mL of MeOH. An argon atmosphere was applied and the mixture was vigorously stirred for 24 h. Then, the reaction mixture was filtrated over a pad of celite and the solvent was removed under reduced pressure. The crude material was purified by column chromatography in CyHex/EtOAc (3:1) to receive 2.83 g (6.93 mmol; 94%) of Boc[dH-ProM-40]OtBu as yellowish oil.

**¹H NMR:** (500 MHz, CDCl₃; mixture of rotamers) δ = 4.44 (dd, J = 23.3, 8.6 Hz, 1H), 4.22 - 4.10 (m, 0.5H), 3.86 (q, J = 8.7 Hz, 0.5H), 3.77 - 3.56 (m, 1H), 3.45 - 3.32 (m, 1H), 2.98 - 2.72 (m, 1H), 2.34-2.14 (m, 1H), 2.12 - 1.66 (m, 11H), 1.63 - 1.53 (m, 1H), 1.52 - 1.33 (m, 18H).

**¹³C NMR:** (125 MHz, CDCl₃; mixture of rotamers) δ = 173.83, 173.60, 172.08, 171.70, 153.71, 80.88, 80.51, 79.25, 79.01, 68.60, 68.10, 63.23, 62.97, 57.14, 56.62, 53.44, 48.36, 47.76, 36.44, 33.04, 32.85, 32.10, 31.92, 30.98, 30.83, 28.54, 27.96, 27.18, 27.03, 23.06, 22.77, 20.69, 20.47.

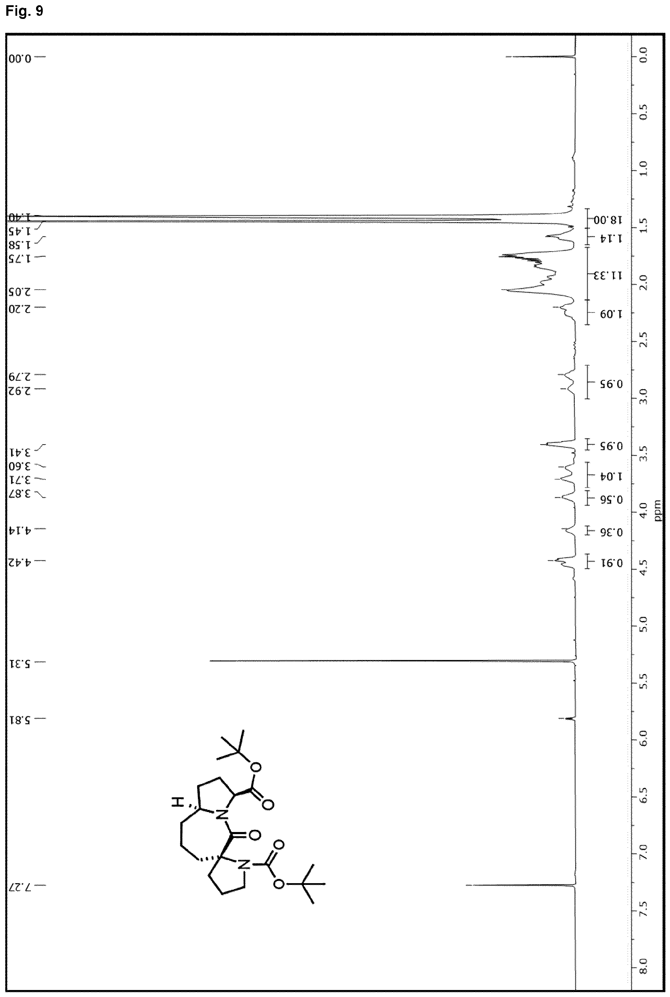

The ¹H-Spectrum of Boc[dH-ProM-40]OtBu (Example compound 10) is shown in Figure 9.

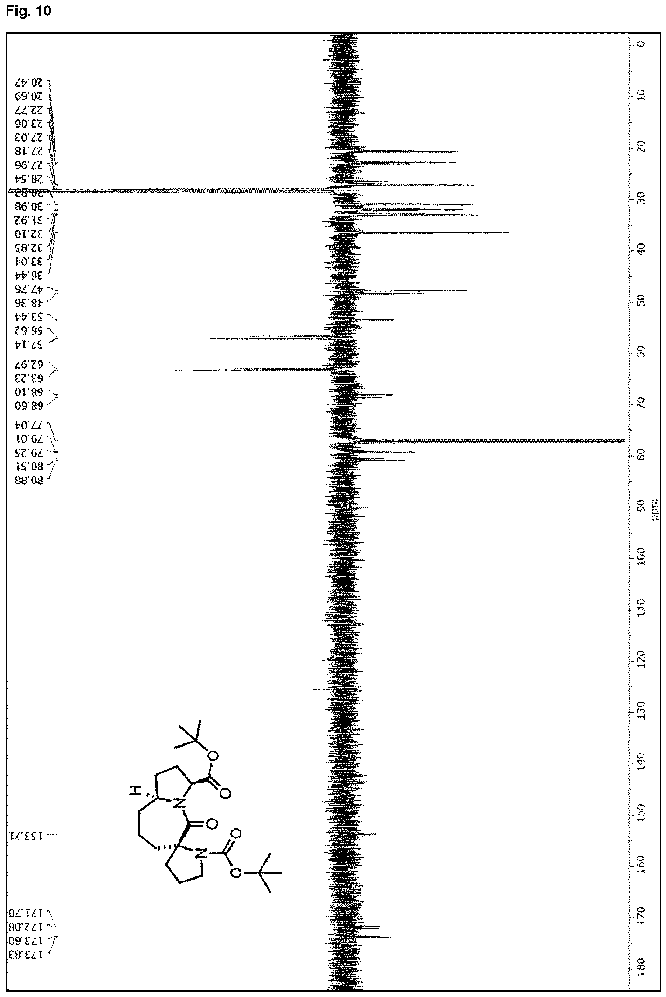

The ¹³C-Spectrum of Boc[dH-ProM-40]OtBu (Example compound 10) is shown in Fig. 10.

### Characterization of Boc[epi-ProM-40']OtBu (Example compound 11)

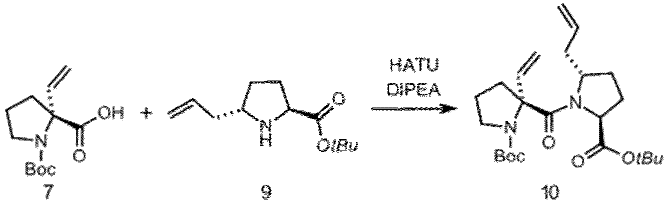

### Step 1:

An inert *Schlenk*flask equipped with a reflux condenser was charged with 920 mg (3.81 mmol; 1.1 eq.) of compound 7, 1.71 g (4.5 mmol; 1.3 eq.) of HATU, 0.62 mL (3.58 mmol; 1.03 eq.) of DIPEA and 6 mL of MeCN. The reaction mixture was stirred for 1 h, until 732 mg (3.46 mmol; 1.0 eq.) of compound **9** in 6 mL of MeCN and 0.62 mL (3.58 mmol; 1.03 eq.) of DIPEA were added. The reaction mixture was heated to 80 °C, until complete consumption of the starting material was observed by TLC (5 h). Then, 10% citric acid was added. The aqueous layer was extracted three times using CH₂Cl₂. The combined organic phases were washed with sat. NaHCO₃, brine, dried over MgSO₄, filtered and the solvent was removed under reduced pressure. The crude product was purified by column chromatography in CyHex/EtOAc (4:1) to receive 688 mg (1.58 mmol; 46%) of compound **10** as yellowish oil.

### Step 2:

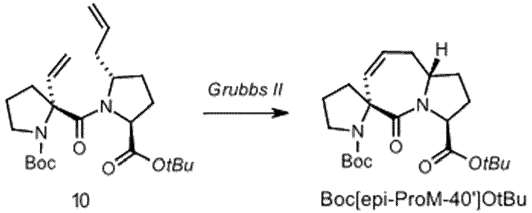

A round bottom flask was charged with 915 mg (2.11 mmol; 1.0 eq.) of compound **10,** 20 mL of Et₂O, 75 mg (88.3 µmol; 4.2 mol%) of *Grubbs II* and 27 mg (142 µmol; 6.73 mol%) of CuI. The reaction mixture was warmed to 45 °C. After 3 h, another batch of 78 mg (91.9 µmol; 4.36 mol%) *Grubbs II* and 23 mg (121 µmol; 5.74 mol%) of Cul were added. After 5 h in total, the reaction monitoring by TLC indicated the complete consumption of the starting material. Therefore, Quadrasil^{™} was added and the solvent was removed under reduced pressure. The crude material was purified by column chromatography (CyHex/EtOAc 3:1) to receive 529 mg (1.30 mmol; 62%) of Boc[epi-ProM-40']OtBu as colorless oil.

**¹H NMR:** (500 MHz, CDCl₃; mixture of rotamers) δ = 5.69 - 5.49 (m, 2H), 4.73 - 4.56 (m, 1H), 4.33 - 4.18 (m, 1H), 3.75 - 3.57 (m, 1H), 3.49 - 3.41 (m, 1H), 2.54 - 2.02 (m, 6H), 1.99 - 1.76 (m, 3H), 1.75 - 1.66 (m, 1H), 1.50 - 1.32 (m, 18H).

¹³C **NMR:** (125 MHz, CDCl₃; mixture of rotamers) δ = 171.52, 171.42, 171.19, 170.61, 154.76, 154.52, 135.17, 133.86, 124.56, 123.58, 81.20, 81.10, 79.25, 79.21, 70.14, 69.97, 62.29, 56.02, 55.78, 47.98, 47.72, 40.48, 39.91, 35.38, 35.17, 31.41, 31.24, 28.59, 28.25, 28.04, 28.00, 26.64, 22.74, 22.38.

**GC-MS:** m/z (%) = 408 (20), 352 (<5), 335 (12), 308 (23), 279 (46), 251 (100), 234 (6), 210 (22), 179 (85), 162 (20), 136 (9), 111 (35), 96 (46), 80 (25), 57 (71).

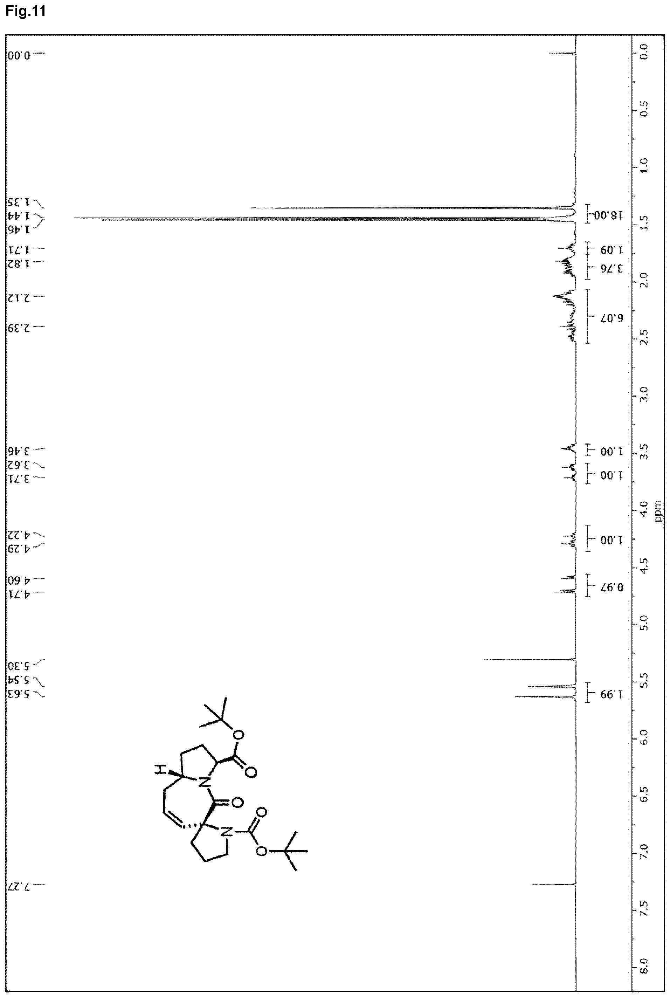

The ¹H-Spectrum of Boc[epi -ProM-40']OtBu (Example compound 11) is shown in Figure 11.

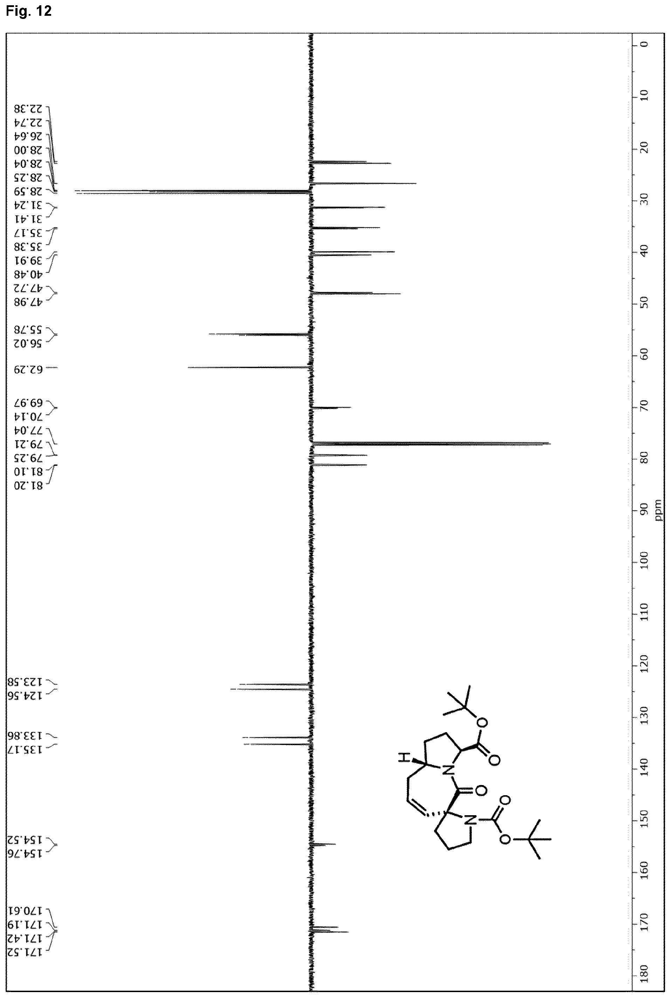

The ¹³C-Spectrum of Boc[epi -ProM-40']OtBu (Example compound 11) is shown in Fig. 12.

### Characterization of Boc[epi-dH-ProM-40]OtBu (Example compound 12)

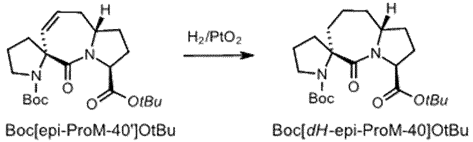

A *Schlenk*flask was charged with 511 mg (1.26 mmol; 1.0 eq.) Boc[epi-ProM-40']OtBu, 29 mg (128 µmol; 0.10 eq.) of Pt₂O and 12 mL of MeOH. An argon atmosphere was applied and the mixture was vigorously stirred for 16 h. Then, the reaction mixture was filtrated over a pad of celite and the solvent was removed under reduced pressure to receive 512 mg (1.25 mmol; quant.) of Boc[dH-epi-ProM-40]OtBu as colorless oil.

**¹H NMR:** (500 MHz, CDCl₃) δ = 4.66 - 4.48 (m, 1H), 3.86 (tddd, J = 15.4, 12.7, 4.4, 1.9 Hz, 1H), 3.74 (tt, J = 8.3, 3.5 Hz, 1H), 3.68 - 3.59 (m, 1H), 3.35 (ddt, J = 10.4, 8.9, 7.3 Hz, 1H), 3.00 - 2.57 (m, 1H), 2.46 - 2.34 (m, 1H), 2.33 - 2.17 (m, 1H), 2.16 -2.02 (m, 1H), 2.01 - 1.63 (m, 5H), 1.57 - 1.48 (m, 1H), 1.47 - 1.39 (m, 18H).

**¹³C NMR:** (125 MHz, CDCl₃) δ = 172.33, 172.10, 172.01, 171.79, 155.16, 154.89, 80.93, 80.81, 79.39, 78.85, 71.03, 70.65, 63.40, 63.17, 58.80, 58.71, 48.43, 48.23, 36.86, 36.05, 35.72, 35.59, 33.29, 33.23, 28.67, 28.52, 28.02, 26.33, 26.21, 24.92, 24.79, 21.84, 21.72.

**GC-MS:** m/z (%) = 408 (20), 352 (<5), 335 (12), 308 (23), 279 (46), 251 (100), 234 (6), 210 (22), 179 (85), 162 (20), 136 (9), 111 (35), 96 (46), 80 (25), 57 (71).

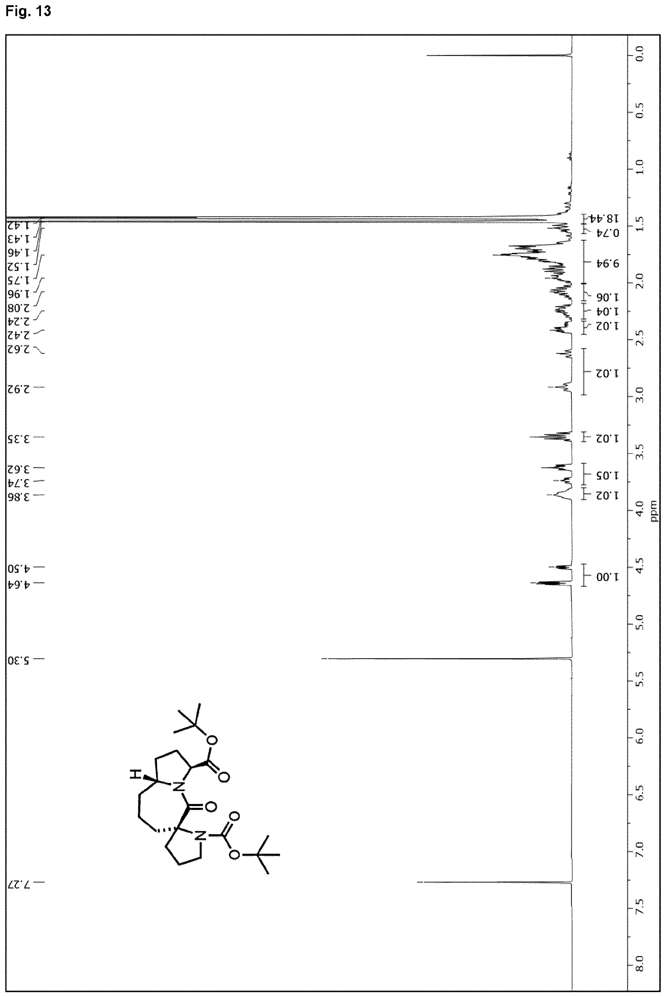

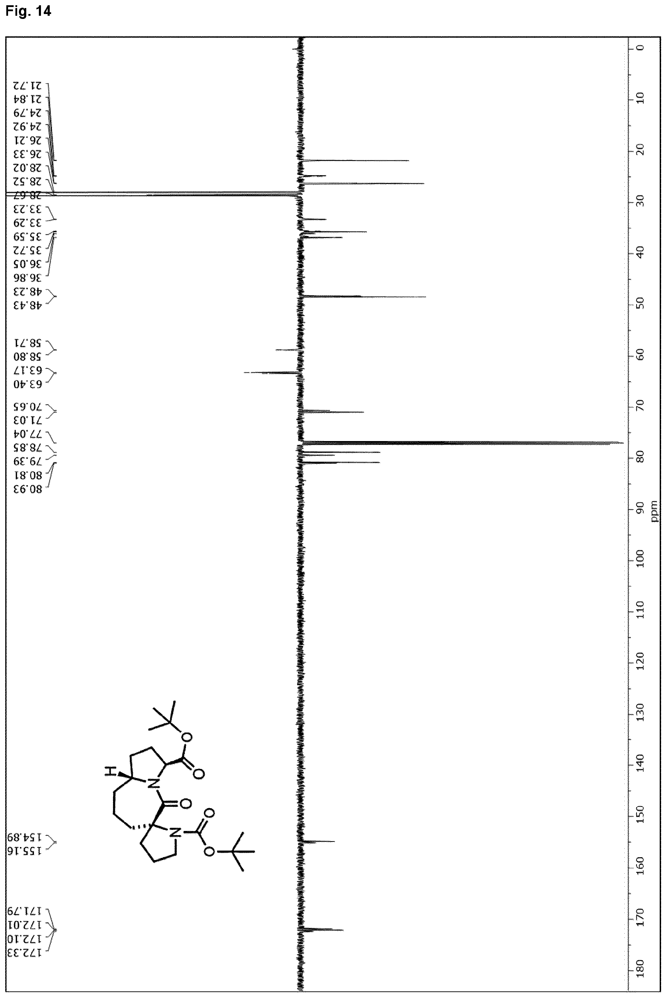

The ¹H-Spectrum of Boc[epi -dH-ProM-40]OtBu (Example compound 12) is shown in Figure 13. The ¹³C-Spectrum of Boc[epi -dH-ProM-40]OtBu (Example compound 12) is shown in Fig. 14.

### Characterization of Ac[2Cl-F][dH-ProM-40][dH-ProM-28]NH (Example compound 13)

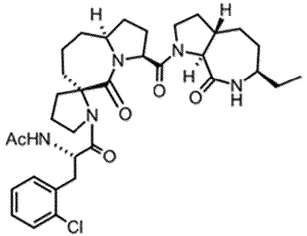

The compound Ac[2Cl-F][*dH*-ProM-40][*dH*-ProM-28]NH (example compound 14) was synthesized according to the general strategy for the synthesis of EVH1-Inhibitors.

**¹H NMR:** (500 MHz, CDCl₃; mixture of rotamers) δ = 7.36 - 7.30 (m, 1H), 7.26 - 7.20 (m, 1H), 7.19 - 7.11 (m, 2H), 6.25 (d, J = 8.8 Hz, 1H), 5.34 (d, J = 5.5 Hz, 1H), 5.08 (td, J = 9.4, 4.2 Hz, 1H), 4.93 - 4.84 (m, 1H), 4.39 (d, J = 9.7 Hz, 1H), 4.26 - 4.16 (m, 1H), 4.12 (dd, J = 9.7, 7.9 Hz, 1H), 3.93 - 3.78 (m, 2H), 3.53 - 3.38 (m, 2H), 3.32 (dd, J = 14.1, 4.2 Hz, 1H), 2.91 (ddd, J = 14.9, 10.2, 7.5 Hz, 1H), 2.82 (dd, J = 14.2, 9.9 Hz, 1H), 2.37 - 2.25 (m, 1H), 2.23 - 1.83 (m, 16H), 1.82 (s, 3H), 1.81 - 1.65 (m, 3H), 1.60 - 1.43 (m, 4H), 1.39 - 1.27 (m, 1H), 0.95 (t, J = 7.4 Hz, 3H).

**HR-MS (ESI):** Calcd. ([M+H]⁺ ): 640.3260; Found: 640.3272.
Calcd. ([M+Na]⁺ ): 662.3079; Found: 662.3080.

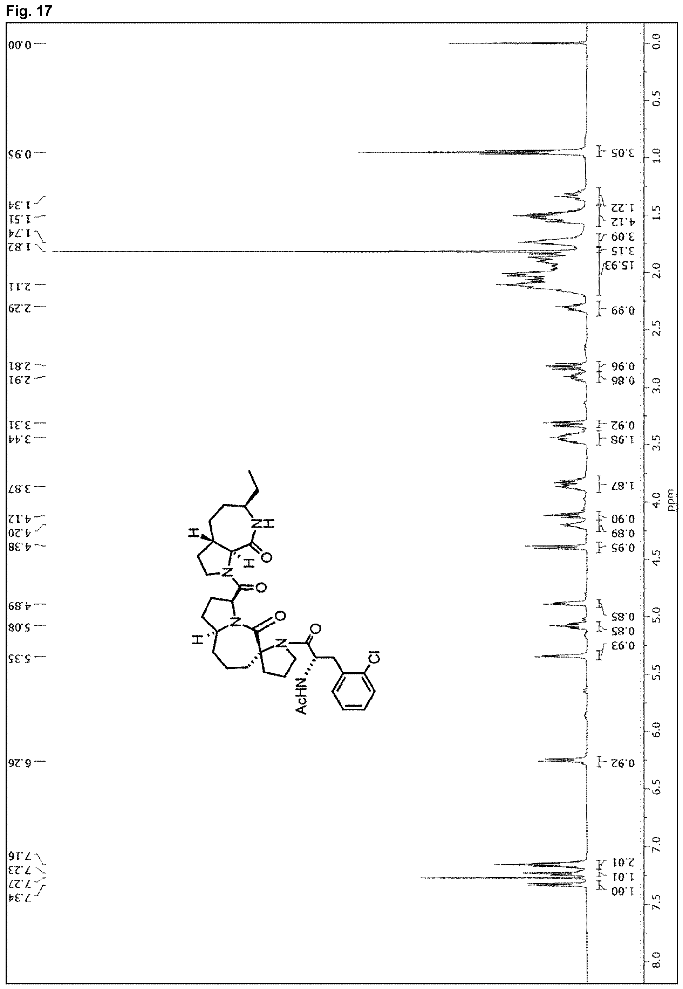

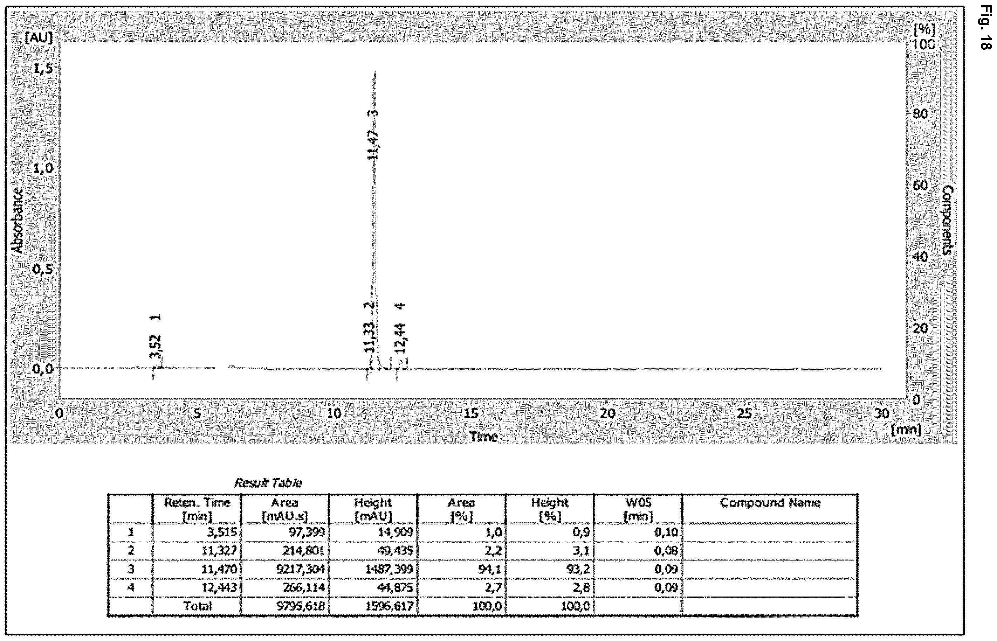

The ¹H-Spectrum of Ac[2Cl-F][dH-ProM-40][dH-ProM-28]NH (Example compound 13) is shown in Figure 17. The HPLC chromatogram of Ac[2Cl-F][dH-ProM-40][dH-ProM-28]NH (Example compound 13) is shown in Fig. 18.

The following conditions were used for HPLC: Column: XSelect CSH C18 from Waters. Temperature: 30 °C. Gradient: Water/MeCN (95:5) to water/MeCN (10:90) over 15 minutes. Hold the mixture for 10 minutes. Initial gradient is resumed over 5 minutes.

### Characterization of Ac[2Cl-F][dH-epi-ProM-40][dH-ProM-28]NH (Example compound 14)

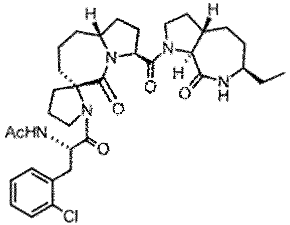

The compound Ac[2Cl-F][*dH*-*epi*-ProM-40][*dH*-ProM-28]NH (example compound 15) was synthesized according to the general strategy for the synthesis of EVH1-Inhibitors.

**¹H NMR:** (500 MHz, CDCl₃; mixture of rotamers) δ 7.36 - 7.12 (m, 4H), 6.32 (d, J = 9.0 Hz, 1H), 5.28 (d, J = 5.5 Hz, 1H), 5.13 (ddd, J = 11.0, 9.0, 3.6 Hz, 1H), 5.00 - 4.94 (m, 1H), 4.39 (d, J = 9.6 Hz, 1H), 3.99 (qd, J = 7.7, 2.8 Hz, 2H), 3.94 - 3.79 (m, 2H), 3.42 (tdd, J = 14.4, 10.6, 4.3 Hz, 3H), 2.93 (dd, J = 14.4, 11.0 Hz, 1H), 2.86 (t, J = 13.0 Hz, 1H), 2.55 (dt, J = 12.2, 5.9 Hz, 1H), 2.45 (dq, J = 12.5, 8.9 Hz, 1H), 2.15 - 1.82 (m, 8H), 1.80 - 1.74 (m, 7H), 1.73 - 1.64 (m, 3H), 1.59 - 1.44 (m, 4H), 1.32 (tdd, J = 13.9, 10.3, 3.6 Hz, 1H), 0.96 (t, J = 7.5 Hz, 3H).

**HR-MS (ESI):** Calcd. ([M+H]⁺ ): 640.3260; Found: 640.3275.
Calcd. ([M+H]⁺): 662.3079; Found: 662.2082.

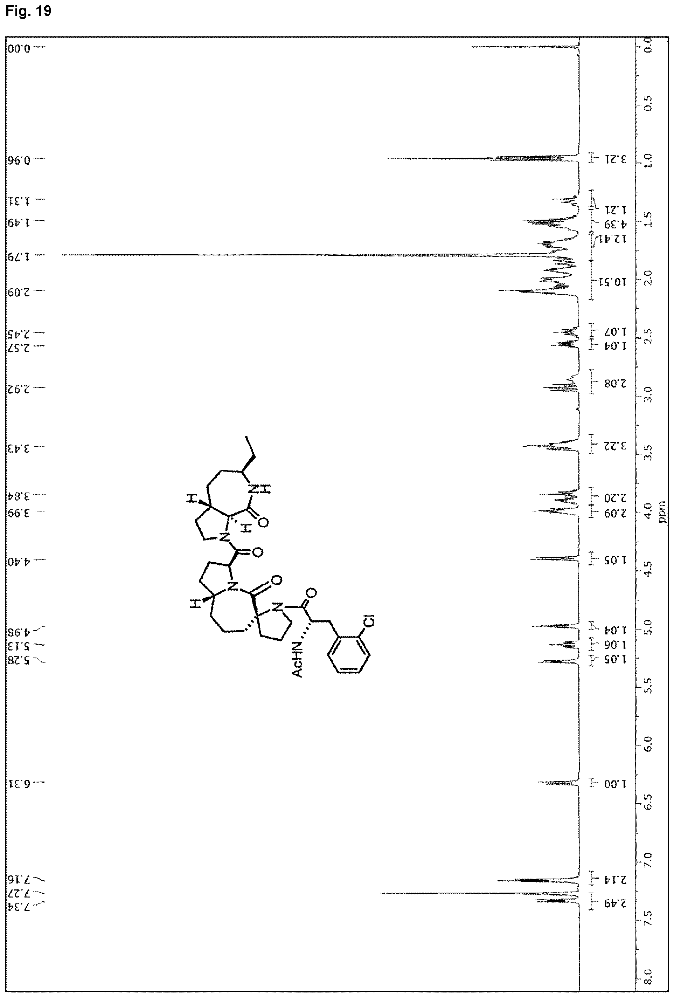

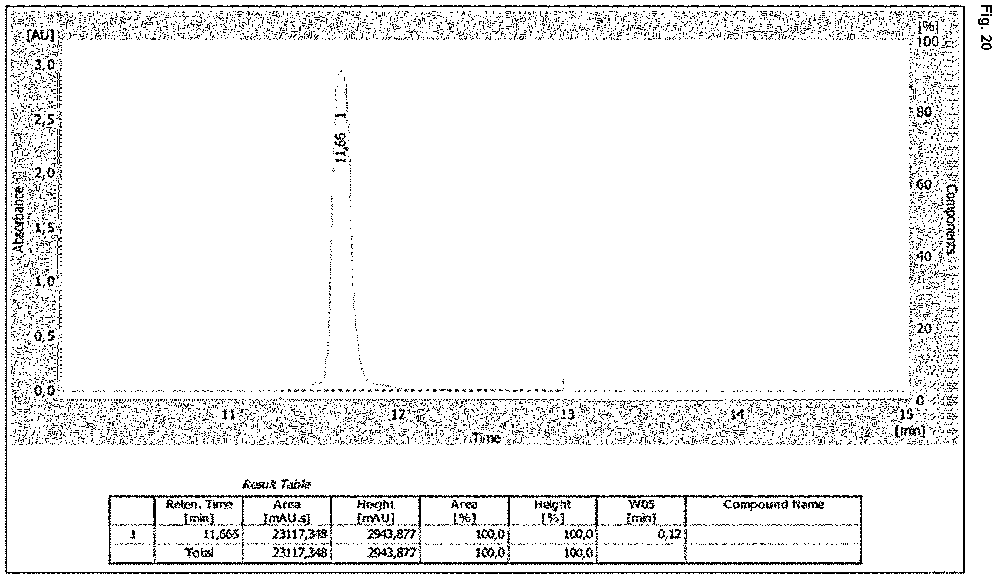

The ¹H-Spectrum of Ac[2Cl-F][dH-epi-ProM-40][dH-ProM-28]NH (Example compound 14) is shown in Figure 19. The HPLC chromatogram of Ac[2Cl-F][dH-epi-ProM-40][dH-ProM-28]NH (Example compound 14) is shown in Fig. 20.

The following conditions were used for HPLC: Column: XSelect CSH C18 from Waters. Temperature: 30 °C. Gradient: Water/MeCN (95:5) to water/MeCN (10:90) over 15 minutes. Hold the mixture for 10 minutes. Initial gradient is resumed over 5 minutes.

### REFERENCES

Review: N.T. Ross, W. P. Katt, A. D. Hamilton, Phil. Trans. R. Soc. A 2010, 368, 989-1008.
B. K. Kay, M. P. Williamson, M. Sudol, FASEB J. 2000, 14, 231-241. L. J. Ball, R. Kühne, J. Schneider-Mergener, H. Oschkinat, Angew. Chem. Int. Ed. 2005, 44, 2852-2869.
R. Opitz, M. Müller, C. Reuter, M. Barone, A. Soicke, Y. Roske, K. Piotukh, P. Huy, M. Beerbaum, B. Wiesner, M. Beyermann, P. Schmieder, C. Freund, R. Volkmer, H. Oschkinat, H.-G. Schmalz, R. Kühne, Proc. Natl. Acad. Sci. USA 2015, 112, 5011-5016.
M. Barone, M. Müller, S. Chiha, J. Ren, D. Albat, A. Soicke, S. Dohmen, M. Klein, J. Bruns, M. van Dinther, R. Opitz, P. Lindemann, M. Beerbaum, K. Motznya, Y. Roske, P. Schmieder, R. Volkmer, M. Nazaré, U. Heinemann, H. Oschkinat, P. ten Dijke, H.-G. Schmalz, R. Kühne, Proc. Natl. Acad. Sci. USA 2020, 117, 29684-29690.
Dohmen et al. Chem. Eur. J. 2020, 26, 3049 - 3053;
Albat et al. Eur. J. Org. Chem. 2021, 4237-4242;
Albat et al. Eur. J. Org. Chem. 2022, e202200188),
D. Albat, S. Chiha, S. Dohmen, P.M. Engelhardt, H. Sebode, A. Soicke, M. Barone, M. Müller, R. Kühne, H.-G. Schmalz. Eur. J. Org. Chem. 2023, 2023e2023007.
A. A. Morgan, E. Rubenstein, PLOS One 2013, 8, e53785.
B. K. Kay, M. P. Williamson, M. Sudol, FASEB J. 2000, 14, 231-241.
L. J. Ball, R. Kühne, J. Schneider-Mergener, H. Oschkinat, Angew. Chem. Int. Ed. 2005, 44, 2852-2869.
E. R. Humphreys, J.-B. Hong, K. L. Green, Synth. Commun. 2011, 41, 2256 - 2264.
M. J. Genin, P. W. Baures, R. L. Johnson, Tetrahedron Lett. 1994, 35, 4967' - 4968.
E. M. Khalil, N. L. Subasinghe, R. L. Johnson, Tetrahedron Lett. 1996, 37, 3441- 3444.
C. Reuter, P. Huy, J. M. Neudörfl, R. Kühne, H.-G. Schmalz, Chem. Eur. J. 2011, 17, 12037-12044.
C. Reuter, R. Opitz, A. Soicke, S. Dohmen, M. Barone, S. Chiha, M. T. Klein, J.-M. Neud 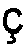 rfl, R. Kghne, H.-G. Schmalz, *Chem. Eur. J.* **2015,** 21, 8464-8470.
J. Zaminer, C. Brockmann, P. Huy, R. Opitz, C. Reuter, M. Beyermann, C. Freund, M. Müller, H. Oschkinat, H.-G. Schmalz, R. Kühne, Angew. Chem. Int. Ed. 2010, 49, 7111-7115.
P. Huy, J.-M. Neudörfl, H.-G. Schmalz, Org. Lett. 2011, 13, 216-219.

## Claims

1. A compound according to **Formula 1** 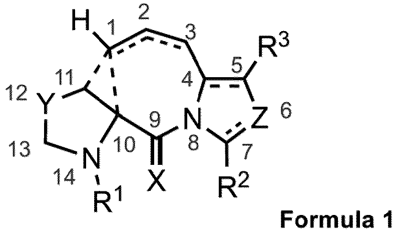
wherein between carbon **C-1** and **C-10,** or **C-1-** and **C-11** a covalent bond is present, carbon C-**1** and C-**2**, or C-**2** and C-**3** are covalently linked by a single bond or a double bond,
up to three hydrogen atoms are replaced by F,
carbon C-**4** and C-**5**, and **Z** and C-**7** are covalently linked by a single bond or a double bond,
**X** is O, S or absent (two H covalently bound to **C-9),**
**Y** is C₁ to C₂ alkylidene, O, S, OCH₂ or SCH₂,
**Z** is C₁ to C₂ alkylidene, CH, -CH₂-CH₂-, O, S, NH, OCH₂, SCH₂ or NR, wherein **R** is C₁ to C₈ branched or linear alkyl (preferably C₁ to C₅ branched or linear alkyl), acyl, alkoxy carbonyl, amino carbonyl or sulfonyl, when **C-4** and **C-5,** and **Z** and **C-7** are covalently linked by a single bond, or
**Z** is N, CH or C**R**, wherein **R** is C₁ to C₈ branched or linear alkyl (preferably C₁ to C₅ branched or linear alkyl), acyl, hydroxy alkyl, alkoxy carbonyl, amino carbonyl, sulfonyl or halogen, when **C-4** and **C-5,** and **Z** and **C-7** are covalently linked by a double bond,
**R¹** is H, alkoxy carbonyl, C₁ to C₅ alkyl aminocarbonyl (preferably C₁ to C₃ alkyl aminocarbonyl), sulfonyl, C₁ to C₈ branched or linear alkyl (preferably C₁ to C₅ branched or linear alkyl), aryl, heteroaryl (preferably comprising N, S and/or O), or acyl -(C=O)-**R**, wherein **R** is C₁ to C₅ branched or linear alkyl (preferably C₁ to C₃ branched or linear alkyl), aryl, heteroaryl (preferably comprising N, S and/or O), a bicyclic or tricyclic ring structure (preferably comprising N, S and/or O), a residual group of an amino acid, an amino acid (preferably an alpha-, beta- or gamma-amino acid), or a peptide,
**R²** is H, C₁ to C₈ branched or linear alkyl (preferably C₁ to C₅ branched or linear alkyl), alkoxy carbonyl, N-substituted or N-unsubstituted amino carbonyl, aryl aminocarbonyl, heteroaryl aminocarbonyl, (preferably comprising N, S and/or O), sulfonyl or acyl -(C=O)-**R**, herein **R** is C₁ to C₅ branched or linear alkyl (preferably C₁ to C₃ branched or linear alkyl), aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide, and
**R³** is H or C₁ to C₈ branched or linear alkyl (preferably C₁ to C₅ branched or linear alkyl).

2. The compound according to claim 1, according to **Formula 1,** wherein
**R¹** is H, alkoxy carbonyl, C₁ to C₃ alkyl aminocarbonyl, sulfonyl, C₁ to C₅ branched or linear alkyl, aryl, heteroaryl (preferably comprising N, S and/or O), or acyl -(C=O)-**R**, wherein **R** is C₁ to C₃ branched or linear alkyl, aryl, heteroaryl (preferably comprising N, S and/or O), a bicyclic or tricyclic ring structure (preferably comprising N, S and/or O) a residual group of an amino acid, an amino acid (preferably an alpha-, beta- or gamma-amino acid), or a peptide,
**R²** is H, C₁ to C₅ branched or linear alkyl, alkoxy carbonyl, N-substituted amino carbonyl, aryl aminocarbonyl, heteroaryl aminocarbonyl, (preferably comprising N, S and/or O), sulfonyl or acyl -(C=O)-**R**, herein **R** is C₁ to C₃ branched or linear alkyl, aryl, heteroaryl (preferably comprising N, S and/or O) or a bicyclic or tricyclic ring structure (preferably comprising N, S and/or O), a residual group of an amino acid, an amino acid (preferably an alpha-, beta- or gamma-amino acid), or a peptide,
**R³** is H or C₁ to C₅ branched or linear alkyl, and
**X, Y** and **Z** are according to claim 1.

3. The compound according to any one of the preceding claims, according to **Formula 2,** 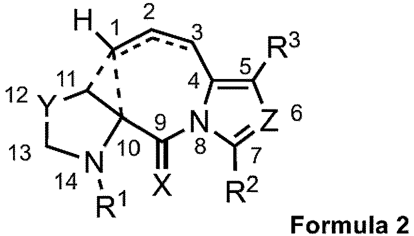 wherein
between carbon **C-1** and **C-10,** or **C-1-** and **C-11** a covalent bond is present,
carbon **C-1** and **C-2,** or **C-2** and **C-3** are covalently linked by a single bond or a double bond,
**Z** is N, CH or C**R**, wherein **R** is C₁ to C₈ branched or linear alkyl (preferably C₁ to C₅ branched or linear alkyl), acyl, hydroxy alkyl, alkoxy carbonyl, amino carbonyl, sulfonyl or halogen, and
**X, Y, R¹, R²** and **R³** are according to claim 1.

4. The compound according to any one of the preceding claims, according to **Formula 3,** 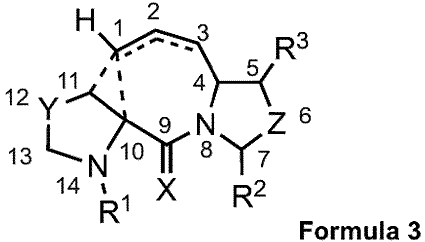 wherein
between carbon C-1 and C-10, or C-1- and C-11 a covalent bond is present,
carbon C-1 and C-2, or C-2 and C-3 are covalently linked by a single bond or a double bond,
Z is C₁ to C₂ alkylidene, CH, -CH₂-CH₂-, O, OCH₂ or SCH₂, and
**X, Y, R¹, R²** and **R³** are according to claim 1.

5. The compound according to any one of the preceding claims, according to **Formula 4a** or **4b** 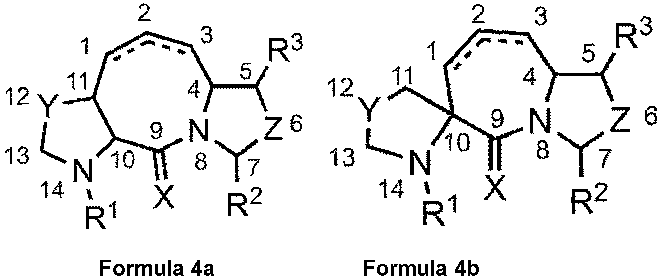 wherein
carbon **C-1** and **C-2,** or **C-2** and **C-3** are covalently linked by a single bond or a double bond,
**Z** is C₁ to C₂ alkylidene, CH, -CH₂-CH₂-, O, OCH₂ or SCH₂, and
**X, Y, R¹, R²** and **R³** are according to claim 1.

6. The compound according to any one of the preceding claims, according to **Formula 5a** or **5b** 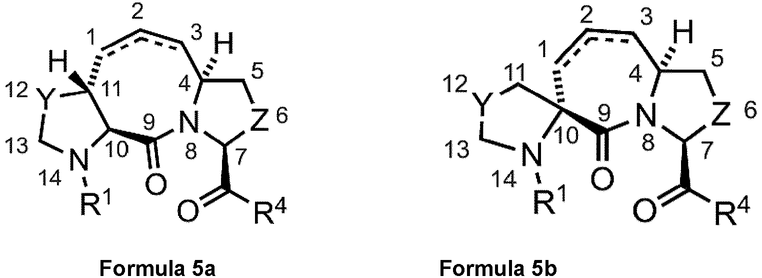 wherein
carbon **C-1** and **C-2,** or **C-2** and **C-3** are covalently linked by a single bond or a double bond,
**R¹** is according to claim 1, and
**R⁴** is OH, oxy alkyl, alkoxy, amine (preferably NH₂, NH-alkyl or N(alkyl)₂), a residue attached by an N, wherein the residue is an amino acid, a peptide, a bicyclic or tricyclic ring structure (preferably comprising N, S and/or O), a residual group of an amino acid, an amino acid (preferably an alpha-, beta- or gamma-amino acid), a peptide or an amino alcohol.

7. The compound according to any one of the preceding claims, wherein the compound is one of 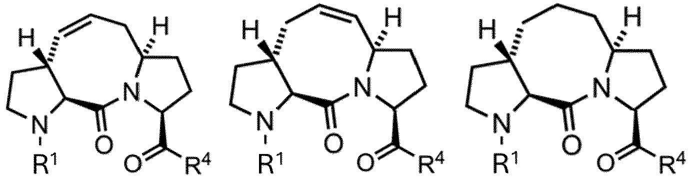 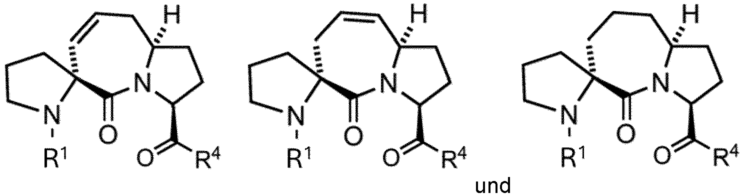 wherein
**R¹** is according to claim 1, and R⁴ is according to claim 6.

8. The compound according to claim 6 or 7, wherein
**R¹** is (S)-2-chloro-phenylalanine or N-acetylated (S)-2-chloro-phenylalanine, optionally substituted with an amino acid or a peptide, and
R⁴ is a residue attached via an N atom, wherein the residue is an amino acid, a peptide, a bicyclic or tricyclic ring group (preferably comprising N and optionally S and/or O).

9. The compound according to any one of the preceding claims, wherein the compound is one of 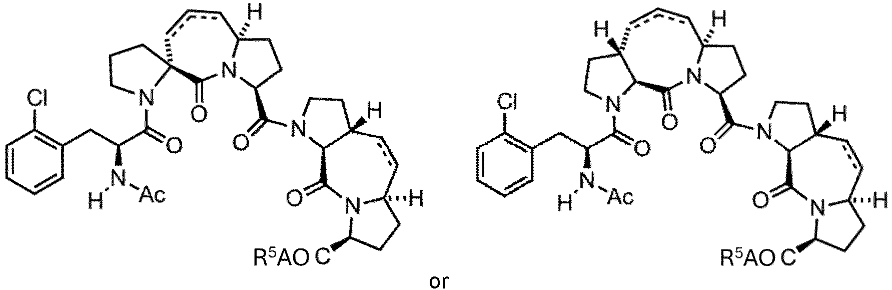 wherein
R⁵ is H, C₁ to C₈ branched or linear alkyl (preferably C₁ to C₅ branched or linear alkyl), a bicyclic or tricyclic ring structure (preferably comprising N, S and/or O), a residual group of an amino acid, an amino acid (preferably an alpha-, beta- or gamma-amino acid), or a peptide, and
**A** is O or NH.

10. A pharmaceutical composition, comprising one or more compounds according to any one of the preceding claims or a pharmaceutically acceptable salt thereof and a pharmaceutically acceptable carrier.

11. A compound according to any one of claims 1-9 or the composition according to claim 10 for use in the treatment of a medical condition.

12. The compound or composition for use according to the preceding claim, wherein the medical condition is associated with and/or caused by a modified intracellular signal transduction process mediated by a poly-proline helix structure, preferably wherein the proline-rich helix structure is a polyproline-type II (PPT II) helix structure.

13. The compound or composition for use according to any one of claims 11 or 12, wherein the proline-rich helix structure binds to a protein having a Src homology 3(SH3) domain, WW domain, Ena/VASP homology 1 domain (EVH1), GYF domain, UEV domain and/or profilin domain.

14. The compound or composition for use according to any one of claims 11 to 13, wherein the disease is selected from the group consisting of an infectious disease, immune disease, neurological disease, cardiometabolic disease and a cancerous disease.

15. An in vitro method for modifying an intracellular signal transduction process mediated by a proline-rich helix structure and/or modulating (preferably inhibiting) proline-rich helix structure mediated protein-protein interactions, comprising contacting a compound according to any one of claims 1-9 with a biological cell, preferably wherein the proline-rich helix structure is a polyproline-type II (PPT II) helix structure, more preferably wherein the proline-rich helix structure binds to a protein having a Src homology 3(SH3) domain, WW domain, Ena/VASP homology 1 domain (EVH1), GYF domain, UEV domain and/or profilin domain.
